# EUROPEAN PATENT APPLICATION

(11) **EP 3 072 968 A1**
(43) Date of publication of application: **28.09.2016**
(21) Application number: 16156020.6
(22) Date of filing: 22.02.2011
(51) Int. Cl.: C12N 15/10, C40B 50/08, C40B 40/06, C07H 21/00, C12Q 1/68

(54) **METHOD OF MAKING NUCLEIC ACID LIBRARIES**

(30) Priority: 25.02.2010 US 307950 P; 20.04.2010 US 326000 P; 26.07.2010 US 367513 P; 05.11.2010 US 410646 P
(62) Divisional of application: 11747926.1
(71) Applicant: Advanced Liquid Logic, Inc., San Diego, CA 92122 (US)
(72) Inventor: POLLACK, Michael G., Durham, NC 27701 (US); ECKHARDT, Allen E., Durham, NC 27705 (US); SUDARSAN, Arjun, Cary, NC 27518 (US); SRINIVASAN, Vijay, Cary, NC 27519 (US); ROUSE, Jeremy, Raleigh, NC 27612 (US); YI, Uichong, Cary, NC 27519 (US); TROTTA, Nicholas, Durham, NC 27713 (US); DHOPESHWARKAR, Rahul, Durham, NC 27713 (US); THWAR, Prasanna, Los Altos, CA 94024 (US); SMITH, Gregory F., Cary, NC 27519 (US); NORTON, Scott, Cary, NC 27519-7426 (US); LINNARTZ, Peter, Durham, NC 27705 (US)
(74) Representative: Thom, Russell

(57) **Abstract**

A method of preparing a nucleic acid library in droplets in contact with oil, including: (a) blunt-ending nucleic acid fragments in a droplet in the oil to yield blunt-ended nucleic acid fragments; (b) phosphorylating the blunt-ended nucleic acid fragments in a droplet in the oil to yield phosphorylated nucleic acid fragments; coupling A-tails to the phosphorylated nucleic acid fragments in a droplet in the oil to yield A-tailed nucleic acid fragments; and (d) coupling nucleic acid adapters to the A-tailed nucleic acid fragments in a droplet in the oil to yield the nucleic acid library comprising adapter-ligated nucleic acid fragments.

## Description

### 1 Related Applications

This application relates to and claims priority to the filing dates of the following U.S. Provisional Patent Application Nos.: 61/307,950, entitled "Automated Library Construction for Next-Generation Sequencing" filed on February 25, 2010; 61/326,000, entitled "Automated Library Construction for Next-Generation Sequencing" filed on April 20, 2010; 61/367,513, entitled "Automated Library Construction for Next-Generation Sequencing" filed on July 26, 2010; and 61/410,646, entitled "Automated Library Construction for Next-Generation Sequencing" filed on November 5, 2010. The disclosures of the aforementioned applications, along with all other documents cited herein, are specifically incorporated herein by reference in their entireties.

### 2 Field of the Invention

The invention generally relates to new droplet-based methods of conducting nucleic acid library construction chemistry in oil.

### 3 Background of the Invention

A droplet actuator typically includes one or more substrates configured to form a surface or gap for conducting droplet operations. The substrates may also include electrodes arranged to conduct the droplet operations. The substrate or the gap between the substrates may be coated and/or filled with a liquid that is immiscible with the liquid that forms the droplets. Droplet actuators have been used to conduct a variety of molecular protocols such as amplification of nucleic acids (e.g., quantitative polymerase chain reaction (qPCR)) and nucleic acid sequencing. Recently, there have been improvements in all aspects of nucleic acid sequencing technology (e.g., cost, speed, throughput, workflow, accuracy and data assembly). So-called "next-generation" sequencing platforms based on these improved technologies are often used in large-scale sequencing projects such as the 1000 Genomes Project, and the Human Microbiome Project. Other applications of these technologies include transcriptome sequencing (RNA-Seq), protein-chromatin interaction analysis (ChIP-Seq), whole exome sequencing, metagenomics and copy number variation analysis. Sequencing methodology of next-generation sequencing platforms makes use of nucleic acid fragment libraries. The full potential of the next-generation sequencing platforms may be realized by optimizing (e.g., reducing cost, increasing yield, and increasing throughput) the process of fragment library construction. Library construction typically involves multiple labor and time intensive steps, including physical processing, enzymatic reactions and purification. The quality of the sequence data (e.g., depth of sequence coverage and sequencing bias) depends on the quality of the fragment library construction.

In a typical library construction protocol, the nucleic acid sample to be sequenced is first randomly fragmented either by hydrodynamic shear or mechanical forces or fragmented by enzymatic or chemical digestion. The resulting nucleic acid fragments are then subjected to additional modification resulting in the attachment of so-called "adapter" sequences to one or both ends of the fragments. This process requires that the fragments are first end-repaired or blunt ended, which is optionally followed by an A-tailing step prior to ligation of the adapter sequences to the sample fragments. The prepared nucleic acid libraries are quantitated and made ready for subsequent sequencing processes. In more advanced protocols, there may be a long circularization step and a second round of end repair and adapter ligation. Short sequences of multiplex identifiers or barcodes may also be ligated to the fragments, or included within the ligated adapter sequences to assist in sequence assembly.

The multiple purification steps required in a typical library construction protocol, including nucleic acid capture, washing, and elution between the various enzymatic reactions typically result in significant nucleic acid loss. Automation involving commercial robotic liquid handlers is even more susceptible to material losses and hence results in very poor yields. Certain applications, such as cancer genomics, metagenomics and paleogenomics, often start with trace amounts of nucleic acid to be processed for library construction and cannot tolerate significant loss of the nucleic acid sample prior to sequencing. There is a need for a flexible, automated platform for library construction that provides high yield, reduced reagent consumption, and allows the user to operate over a range of multiplexed operations.

### 4 Brief Description of the Invention

The invention provides a method of preparing a nucleic acid library in droplets in contact with oil, including: blunt-ending nucleic acid fragments in a droplet in the oil to yield blunt-ended nucleic acid fragments; phosphorylating the blunt-ended nucleic acid fragments in a droplet in the oil to yield phosphorylated nucleic acid fragments; coupling A-tails to the phosphorylated nucleic acid fragments in a droplet in the oil to yield A-tailed nucleic acid fragments; and coupling nucleic acid adapters to the A-tailed nucleic acid fragments in a droplet in the oil to yield a nucleic acid library of adapter-ligated nucleic acid fragments.

In some cases, recovery of adapter-ligated nucleic acid fragments is at least 5% on a molar basis of nucleic acid fragments. In some cases, recovery of adapter-ligated nucleic acid fragments is at least 10% on a molar basis of nucleic acid fragments. In some cases, recovery of adapter-ligated nucleic acid fragments is at least 15% on a molar basis of nucleic acid fragments. In some cases, recovery of adapter-ligated nucleic acid fragments is at least 20% on a molar basis of nucleic acid fragments. In some cases, recovery of adapter-ligated nucleic acid fragments is at least 50% on a molar basis of nucleic acid fragments. In some cases, recovery of adapter-ligated nucleic acid fragments is at least 75% on a molar basis of nucleic acid fragments. In some cases, recovery of adapter-ligated nucleic acid fragments is at least 90% on a molar basis of nucleic acid fragments. In some cases, recovery of adapter-ligated nucleic acid fragments is at least 95% on a molar basis of nucleic acid fragments. In some cases, recovery of adapter-ligated nucleic acid fragments is at least 99% on a molar basis of nucleic acid fragments.

The method may include purifying the blunt-ended nucleic acid fragments before or after one or more of the steps. The method may include purifying the blunt-ended nucleic acid fragments prior to initiating step 1(b). The method may include purifying the phosphorylated nucleic acid fragments prior to initiating step 1(c). The method may include purifying the A-tailed nucleic acid fragments prior to initiating step 1(d). The purifying may, for example, include capturing the nucleic acid fragments on beads in a droplet in the oil and washing the beads in the oil. The beads may, for example, include charge switch beads or solid phase reversible immobilization beads. In some embodiments, the purifying includes: merging wash droplets with a droplet including the beads to yield a merged droplet; and splitting the merged droplet while restraining the beads yielding a daughter droplet with the beads and a daughter droplet which is substantially lacking in the beads. Ideally, any bead loss during the splitting process is not sufficient to render the nucleic acid library unsuitable for its intended purpose.

Nucleic acid fragments used to construct the library may sometimes include 5'- and/or 3'-overhangs. Blunt-ending of such fragments may include combining in the oil a droplet including the nucleic acid fragments with a droplet including blunt-ending reagents. Phosphorylating may include combining in the oil a droplet including the nucleic acid fragments with a droplet including phosphorylating reagents. Coupling A-tails may include combining in the oil a droplet including the nucleic acid fragments with a droplet including A-tailing reagents. Coupling nucleic acid adapters may include combining in the oil a droplet including the nucleic acid fragments with a droplet including adapter-ligation reagents.

The invention provides a method of performing nucleic acid library construction in droplets in contact with oil, including: blunt-ending and phosphorylating nucleic acid fragments in a droplet in the oil to yield blunt-ended/phosphorylated nucleic acid fragments; capturing the blunt-ended/phosphorylated nucleic acid fragments in a droplet in the oil using solid phase reversible immobilization beads in a binding buffer; washing the solid phase reversible immobilization beads in a droplet in the oil using an aqueous buffer; eluting the blunt-ended/phosphorylated nucleic acid fragments from the solid phase reversible immobilization beads in a droplet in the oil; coupling A-tails on both ends of the phosphorylated nucleic acid fragments in a droplet in the oil to yield A-tailed nucleic acid fragments; capturing the A-tailed nucleic acid fragments in a droplet in the oil using solid phase reversible immobilization beads in a binding buffer; washing the solid phase reversible immobilization beads in a droplet in the oil using an aqueous buffer; eluting the A-tailed nucleic acid fragments from the solid phase reversible immobilization beads in a droplet in the oil; coupling nucleic acid adapters to the A-tailed nucleic acid fragments in a droplet in the oil to yield adapter-ligated nucleic acid fragments; capturing the adapter-ligated nucleic acid fragments in a droplet in the oil using solid phase reversible immobilization beads in a binding buffer; washing the solid phase reversible immobilization beads in a droplet in the oil using an aqueous buffer; eluting the adapter-ligated nucleic acid fragments from the solid phase reversible immobilization beads in a droplet in the oil; and separating the adapter-ligated nucleic acid fragments from the solid phase reversible immobilization beads in a droplet in the oil. In some cases, recovery of adapter-ligated nucleic acid fragments is at least 5% on a molar basis of nucleic acid fragments. In some cases, recovery of adapter-ligated nucleic acid fragments is at least 10% on a molar basis of nucleic acid fragments. In some cases, recovery of adapter-ligated nucleic acid fragments is at least 15% on a molar basis of nucleic acid fragments. In some cases, recovery of adapter-ligated nucleic acid fragments is at least 20% on a molar basis of nucleic acid fragments. In some cases, recovery of adapter-ligated nucleic acid fragments is at least 50% on a molar basis of nucleic acid fragments. In some cases, recovery of adapter-ligated nucleic acid fragments is at least 75% on a molar basis of nucleic acid fragments. In some cases, recovery of adapter-ligated nucleic acid fragments is at least 90% on a molar basis of nucleic acid fragments.

In some cases, recovery of adapter-ligated nucleic acid fragments is at least 95% on a molar basis of nucleic acid fragments. In some cases, recovery of adapter-ligated nucleic acid fragments is at least 99% on a molar basis of nucleic acid fragments.

The method may further include amplifying the nucleic acid library. The method may also include amplifying the nucleic acid library on a droplet actuator. The method may also include sequencing the nucleic acid library on an automated sequencer. The method may also include sequencing the nucleic acid library on an automated sequencer without an intervening nucleic acid amplification step. The method may also include sequencing the nucleic acid library on an automated sequencer without conducting a nucleic acid amplification step.

The invention provides a method of making blunt-ended/phosphorylated nucleic acid fragments in a droplet in contact with oil, including merging a sample droplet including nucleic acid fragments with one or more reagent droplets including blunt-ending and phosphorylating reagents to yield a product droplet including blunt-ended/phosphorylated nucleic acid fragments. The method may include merging the product droplet with a bead droplet including solid phase reversible immobilization beads to capture the blunt-ended/phosphorylated nucleic acid fragments in a capture droplet. The method may include washing the solid phase reversible immobilization beads using a droplet-based merge-and-split wash protocol using wash buffer droplets to yield a droplet including washed beads including the blunt-ended/phosphorylated nucleic acid fragments, wherein the wash buffer droplets consist essentially of an aqueous buffer. The method may include merging a droplet including washed beads with an elution buffer droplet to yield an elution droplet including eluted blunt-ended/phosphorylated nucleic acid fragments. The method may include separating the blunt-ended/phosphorylated nucleic acid fragments from the solid phase reversible immobilization beads to yield a droplet including the blunt-ended/phosphorylated nucleic acid fragments in the oil.

The invention provides a method of ligating a nucleic acid to a blunt-ended nucleic acid fragment in a droplet in contact with oil, including merging a sample droplet including bunt-ended nucleic acid fragments with one or more reagent droplets including the nucleic acid and ligation reagents to yield a product droplet including ligated nucleic acid fragments. The method may include merging the product droplet with a bead droplet including solid phase reversible immobilization beads to capture the ligated nucleic acid fragments. The method may include washing the solid phase reversible immobilization beads using a droplet-based merge-and-split wash protocol using wash buffer droplets to yield a droplet including washed beads including the ligated nucleic acid fragments, wherein the wash buffer droplets consist essentially of an aqueous buffer. The method may include merging a droplet including washed beads with an elution buffer droplet to yield an elution droplet including eluted ligated nucleic acid fragments. The method may include separating the A-tailed nucleic acid fragments from the solid phase reversible immobilization beads to yield a droplet including the ligated nucleic acid fragments in the oil.

The invention provides a method of making A-tailed nucleic acid fragments in a droplet in contact with oil, including merging in the oil a sample droplet including nucleic acid fragments with one or more reagent droplets including A-tailing reagents to yield a product droplet including A-tailed nucleic acid fragments. The method may include merging the product droplet with a bead droplet including solid phase reversible immobilization beads to capture the A-tailed nucleic acid fragments. The method may include washing the solid phase reversible immobilization beads using a droplet-based merge-and-split wash protocol using wash buffer droplets to yield a droplet including washed beads including the A-tailed nucleic acid fragments, wherein the wash buffer droplets consist essentially of an aqueous buffer.

The method may include merging a droplet including washed beads with an elution buffer droplet to yield an elution droplet including eluted A-tailed nucleic acid fragments. The method may include separating the A-tailed nucleic acid fragments from the solid phase reversible immobilization beads to yield a droplet including the A-tailed nucleic acid fragments in the oil.

The invention provides a method of making adapter ligated nucleic acid fragments in a droplet in contact with oil, including merging in oil a sample droplet including nucleic acid fragments with one or more reagent droplets including A-tailing reagents to yield a product droplet including adapter ligated nucleic acid fragments. The method may include merging the product droplet with a bead droplet including solid phase reversible immobilization beads to capture the adapter ligated nucleic acid fragments. The method may include washing the solid phase reversible immobilization beads using a droplet-based merge-and-split wash protocol using wash buffer droplets to yield a droplet including washed beads including the adapter ligated nucleic acid fragments, wherein the wash buffer droplets consist essentially of an aqueous buffer. The method may include merging a droplet including washed beads with an elution buffer droplet to yield an elution droplet including eluted adapter ligated nucleic acid fragments. The method may include separating the adapter ligated nucleic acid fragments from the solid phase reversible immobilization beads to yield a droplet including the adapter ligated nucleic acid fragments in the oil.

The invention provides a method of purifying nucleic acid fragments in a droplet in contact with oil, including conducting the following, steps in contact with oil: merging a droplet including the nucleic acid fragments with a bead droplet including solid phase reversible immobilization beads to capture the nucleic acid fragments; washing the solid phase reversible immobilization beads using a droplet-based merge-and-split wash protocol using wash buffer droplets to yield a droplet including washed beads including the nucleic acid fragments; merging a droplet including washed beads with an elution buffer droplet to yield an elution droplet including eluted blunt-ended/phosphorylated nucleic acid fragments; and separating the nucleic acid fragments from the solid phase reversible immobilization beads to yield a droplet including the purified nucleic acid fragments in the oil.

In any of the methods described herein, the wash buffer droplets may include droplets that consist essentially of an aqueous buffer. In some embodiments, the aqueous buffer consists essentially of a binding buffer. In some embodiments, the aqueous buffer is substantially lacking in organic solvents. In some embodiments, the aqueous buffer includes no more than about 10% organic solvent. In some embodiments, the aqueous buffer is substantially lacking in ethanol. In some embodiments, the aqueous buffer includes no more than about 10% ethanol. In some embodiments, the wash buffer droplets include droplets including at least about 25% organic solvent. In some embodiments, the wash buffer droplets include droplets including at least about 50% organic solvent. In some embodiments, the wash buffer droplets include droplets including at least about 50% organic solvent. In some embodiments, the organic solvent includes an alcohol. In some embodiments, the organic solvent includes ethanol. In some embodiments, the organic solvent consists essentially of ethanol. In some embodiments, the wash buffer droplets are spiked with a salt. In some embodiments, the wash buffer droplets are spiked with NaCl.

In some embodiments, the wash buffer droplets include a salt in an amount ranging from about 0.01 to about 100 mM. In some embodiments, the wash buffer droplets include a salt in an amount ranging from about 0.1 to about 10 mM. In some embodiments, the wash buffer droplets are spiked in an amount ranging from about 0.01 to about 100 mM with a normal salt that is soluble in the wash buffer.

In some embodiments, the wash buffer droplets are spiked in an amount ranging from about 0.1 to about 10 mM with a normal salt that is soluble in the wash buffer. In some embodiments, the wash buffer droplets are spiked in an amount ranging from about 0.01 to about 100 mM with a simple salt that is soluble in the wash buffer. In some embodiments, the wash buffer droplets are spiked in an amount ranging from about 0.1 to about 10 mM with a simple salt that is soluble in the wash buffer. In some embodiments, the wash buffer droplets are spiked with NaCl in an amount ranging from about 0.01 to about 100 mM. In some embodiments, the wash buffer droplets are spiked with NaCl in an amount ranging from about 0.1 to about 10 mM. In some embodiments, the salt improves the repeatability of one or more electrowetting droplet operations relative to the wash buffer droplet including the organic solvent in the absence of the salt. In some embodiments, the one or more electrowetting droplet operations are selected from the group consisting of: droplet transport, droplet splitting, and droplet dispensing.

In various embodiments, some or all steps of the library construction protocols of the invention are executed in oil. For example, in some embodiments, the oil includes a silicone oil or a fluorosilicone oil. Other oils and surfactants for doping oils are described herein.

In some embodiments, the method further includes sequencing the nucleic acid library on an automated sequencer. In some embodiments, the method further includes sequencing the nucleic acid library on an automated sequencer without an intervening nucleic acid amplification step. In some embodiments, the method further includes sequencing the nucleic acid library on an automated sequencer without conducting a nucleic acid amplification step.

In some embodiments, the droplets in the oil are controlled by a droplet actuator in order to execute the protocols of the invention. The droplet actuator may control the steps using droplet operations. For example, the droplet operations may include electrode-mediated droplet operations, e.g., electrowetting-mediated droplet operations, optoelectrowetting-mediated droplet operations, dielectrophoresis-mediated droplet operations. Other techniques for conducting droplet operations are described herein. Ideally, the steps are performed in an integrated manner on a single droplet actuator without operator interference.

Some or all of the droplets used in the processes of the invention may be in contact with oil. For example, the droplet may be substantially surrounded by the oil. The droplet may be floating in the oil. The droplet may be submersed in the oil. The droplet and the oil may sandwiched between two substrates separated to form a droplet operations gap which contains the oil. The droplet in contact with oil may be sandwiched between two substrates separated to form a droplet operations gap which is substantially filled with the oil.

The invention provides a method of blunt-ending nucleic acid fragments including merging in oil a droplet including nucleic acid fragments with a droplet including blunt-ending reagents to conduct a blunt-ending reaction yielding blunt-ended nucleic acid fragments. The invention provides a method of phosphorylating nucleic acid fragments including merging in oil a droplet including blunt-ended nucleic acid fragments with a droplet including phosphorylation reagents to conduct a phosphorylation reaction yielding phosphorylated nucleic acid fragments. The invention provides a method of modifying nucleic acid fragments including merging in oil a droplet including blunt-ended nucleic acid fragments with a droplet including blunt-ending and phosphorylation reagents to conduct blunt-ending and phosphorylation reactions yielding blunt-ended, phosphorylated nucleic acid fragments. The invention provides a method of A-tailing nucleic acid fragments including merging in oil a droplet including phosphorylated nucleic acid fragments with a droplet including A-tailing reagents to conduct an A-tailing reaction yielding A-tailed nucleic acid fragments. The invention provides a method of ligating nucleic acid fragments including merging in oil a droplet including a first nucleic acid with one or more droplets including a second nucleic acid and ligation reagents. The first nucleic acid include an A-tailed nucleic acid, and the second nucleic acid include an adapter.

The invention provides systems which control the steps in the library construction process. The invention includes, for example, a system including a droplet actuator and a controller programmed to conduct any of the methods or any individual steps of the methods. The invention includes a storage medium including encoded software programmed to conduct any of the methods.

The invention also provides a droplet actuator, including: a top substrate and a bottom substrate, the two substrates configured to form a droplet operations gap having a height hi; an electrode path including electrodes associated with one or both of the bottom substrate and the top substrate, and configured for conducting droplet operations in the gap having a height hi; and a recessed area formed in one of the top or bottom substrates configured to form a cavity between the two substrates having a height h2, wherein h2 is greater than hi. In some embodiments the cavity is adjacent to the electrodes configured for conducting droplet operations such that deactivation of electrodes in the presence of a droplet permits a droplet to flow from the electrodes into the recessed area. In some embodiments the droplet enters the cavity as a result of displacement caused by deformation of the droplet to a more energetically stable conformation. In some embodiments the cavity has dimensions selected to prevent the droplet from re-entering the region of the gap having a height hi upon reactivation of electrodes of the electrode path.

In some embodiments the shape of the recessed area includes a stair step shape from hi to h2. In some embodiments the shape of the recessed area includes a slope from hi to h2. In some embodiments the recessed area is formed in the top substrate, the bottom substrate, or both substrates. The recessed area may be open at its top or bottom.

The invention provides a method of displacing a droplet from atop an electrode in a droplet actuator, the method including deactivating an electrode to permit the droplet to be displaced into an adjacent region of the droplet actuator in which the droplet takes on a more energetically stable conformation relative to its conformation atop the electrode. The invention provides a method of displacing a droplet in an initial position in a droplet actuator, the method including deactivating an electrode to permit the droplet to be displaced into an adjacent region of the droplet actuator in which the droplet takes on a more energetically stable conformation relative to its conformation in its initial position. In some cases, the displacement is permanent such that reactivation of the electrode cannot return the droplet to its former position atop the electrode. In some cases, the displacement is temporary such that reactivation of the electrode returns the droplet to its former position atop the electrode. In some cases, the displaced droplet is positioned adjacent to a third electrode, such that activation of the third electrode displaces the droplet to a position atop the third electrode. In some cases, the shape of the recessed area includes a stair step shape from hi to h2. In some cases, the shape of the recessed area includes a slope from hi to h2. In some cases, the recessed area is formed in the top substrate, the bottom substrate, or both substrates. In some cases, the recessed area is open at its top.

The invention provides a method of dispensing a droplet including: collecting a source droplet at an end of a segmented path of reservoir electrodes; elongating the source droplet along a set of path electrodes and path flanking electrodes; deactivating the path flanking electrodes; deactivating one or more of the path electrodes to yield a dispensed droplet and a remaining portion of the source droplet. In some cases, the source droplet includes magnetically responsive beads. In some cases, an initial path electrode may be inset into an adjacent flanking electrode.

A magnetic field may be situated at a position which attracts the magnetically responsive beads into a region of the droplet atop the path electrodes. In some cases, the deactivating step yields the dispensed droplet with at least 50% of magnetically responsive beads from the source droplet. In some cases, the deactivating step yields the dispensed droplet with at least 25% of magnetically responsive beads from the source droplet. In some cases, the deactivating step yields the dispensed droplet with at least 50% of magnetically responsive beads from the source droplet. In some cases, the deactivating step yields the dispensed droplet with at least 75% of magnetically responsive beads from the source droplet. In some cases, the deactivating step yields the dispensed droplet with at least 90% of magnetically responsive beads from the source droplet. In some cases, the deactivating step yields the dispensed droplet with at least 95% of magnetically responsive beads from the source droplet. In some cases, the deactivating step yields the dispensed droplet with at least 99% of magnetically responsive beads from the source droplet. In some cases, the deactivating step yields the dispensed droplet with substantially all magnetically responsive beads from the source droplet. As with all bead-containing droplets described in this specification, in some cases, the source droplet includes tens of beads, hundreds of beads, thousands of beads; millions of beads; or more. As with all bead-containing droplets described in this specification, in some cases, the source droplet includes tens of magnetically responsive beads, hundreds of magnetically responsive beads, thousands of magnetically responsive beads; millions of magnetically responsive beads; or more.

The invention provides, a droplet actuator assembly including: one or more substrates; a series of reaction lanes on the one or more substrates, each reaction lane including a path of electrodes; a first set of droplet dispensing electrode assemblies on the one or more substrates, each assembly of the first set arranged to dispense sample droplets onto one of the reaction lanes without traversing any other of the reaction lanes; a second set of droplet dispensing electrode assemblies on the one or more substrates, each assembly of the second set arranged to dispense reagent droplets onto one of the reaction lanes. The one or more substrates may be arranged to form a droplet operations gap. The reaction lanes may be situated in the droplet operations gap. The droplet actuator assembly may include a fluid path extending from an exterior of the droplet operations gap into the droplet operations gap and arranged to deliver liquid into proximity one or more of the first set of droplet dispensing electrodes. The droplet actuator assembly may include a fluid path extending from an exterior of the droplet operations gap into the droplet operations gap and arranged to deliver liquid into proximity one or more of the second set of droplet dispensing electrodes. Each of the first set of droplet dispensing electrode assemblies may be associated with a reservoir including a sample fluid. The droplet actuator assembly may include at least 2 reaction lanes. The droplet actuator assembly may include at least 8 reaction lanes. The droplet actuator assembly may include at least 16 reaction lanes. The droplet actuator assembly may include at least 24 reaction lanes. The droplet actuator assembly may include at least 48 reaction lanes. The droplet actuator assembly may include at least 96 reaction lanes. In some cases, each of the second set of droplet dispensing electrode assemblies is situated in a reservoir including a library construction reagent. In some cases, the second set of droplet dispensing electrode assemblies is divided into subsets, each subset including two or more droplet dispensing electrode assemblies arranged to dispense sample droplets onto the same one of the reaction lanes without traversing any other of the reaction lanes. In some cases, each subset of droplet dispensing electrode assemblies, each assembly within such subset is associated with a reservoir including a different library construction reagent. In some cases, the reagents are selected from blunt-ending reagents, phosphorylation reagents, A-tailing reagents, and adapter ligation reagents. The droplet actuator assembly may also include a magnet array situated relative to the reaction lanes such that the magnetic fields in the vicinity of the reaction lanes have strength sufficient to immobilize magnetically responsive beads in droplets in one or more regions of the reaction lanes. The droplet actuator assembly may also include a magnet array situated relative to the reaction lanes such that the magnetic fields in the vicinity of the reaction lanes have strength sufficient to restrain magnetically responsive beads in droplets during a droplet splitting reaction controlled by the electrodes of the reaction lane. The magnet array may include magnets arranged to produce reinforced regions of the magnetic field and the reinforced regions are aligned with the reaction lanes to immobilize magnetically responsive beads in the reaction lanes.

The invention provides a method of conducting a droplet based assay using electrode-mediated droplet operations, the method including: dispensing two or more sample droplets and transporting each sample droplet onto an independent reaction lane without causing any sample droplet to traverse a reaction lane of another droplet; and dispensing a first set of reagent droplets and transporting each droplet of the first set of reagent droplets onto a reaction lane without causing any droplet of the first set of reagent droplets to traverse any region of any other reaction lane that has been previously traversed by a sample droplet. The method may also include merging each sample droplet with one of the first set of reagent droplets. The method may also include advancing each sample droplet along its independent reaction lane. The method may also include dispensing a second set of reagent droplets and transporting each droplet of the second set of reagent droplets onto a reaction lane without causing any droplet of the second set of reagent droplets to traverse any region of any other reaction lane that has been previously traversed by a sample droplet. The method may also include merging each sample droplet with one of the second set of reagent droplets. The method may also include advancing each sample droplet along its independent reaction lane.

### 5 Definitions

As used herein, the following terms have the meanings indicated.

"Activate," with reference to one or more electrodes, means affecting a change in the electrical state of the one or more electrodes which, in the presence of a droplet, results in a droplet operation. Activation of an electrode can be accomplished using alternating or direct current. Any suitable voltage may be used. For example, an electrode may be activated using a voltage which is greater than about 50 V, or greater than about 100 V, or greater than about 150 V, or greater than about 200 V, or greater than about 250 V, or from about 275 V to about 375 V, or about 300 V. Where alternating current is used, any suitable frequency may be employed. For example, an electrode may be activated using alternating current having a frequency from about 1 Hz to about 1000 Hz, from about 1 Hz to about 100 Hz, or from about 10 Hz to about 60 Hz, or from about 20 Hz to about 40 Hz, or about 30 Hz.

"Bead," with respect to beads on a droplet actuator, means any bead or particle that is capable of interacting with a droplet on or in proximity with a droplet actuator. Beads may be any of a wide variety of shapes, such as spherical, generally spherical, egg shaped, disc shaped, cubical, amorphous and other three dimensional shapes. The bead may, for example, be capable of being subjected to a droplet operation in a droplet on a droplet actuator or otherwise configured with respect to a droplet actuator in a manner which permits a droplet on the droplet actuator to be brought into contact with the bead on the droplet actuator and/or off the droplet actuator. Beads may be provided in a droplet, in a droplet operations gap, or on a droplet operations surface. Beads may be provided in a reservoir that is external to a droplet operations gap or situated apart from a droplet operations surface, and the reservoir may be associated with a fluid path and/or electrode path that permits a droplet including the beads to be brought into a droplet operations gap or into contact with a droplet operations surface. Beads may be manufactured using a wide variety of materials, including for example, resins, and polymers. The beads may be any suitable size, including for example, microbeads, microparticles, nanobeads and nanoparticles. In some cases, beads are magnetically responsive; in other cases beads are not significantly magnetically responsive or are not magnetically responsive. For magnetically responsive beads, the magnetically responsive material may constitute substantially all of a bead, a portion of a bead, or only one component of a bead. The remainder of the bead may include, among other things, polymeric material, coatings, and moieties which permit attachment of an assay reagent. Examples of suitable beads include flow cytometry microbeads, polystyrene microparticles and nanoparticles, functionalized polystyrene microparticles and nanoparticles, coated polystyrene microparticles and nanoparticles, silica microbeads, fluorescent microspheres and nanospheres, functionalized fluorescent microspheres and nanospheres, coated fluorescent microspheres and nanospheres, color dyed microparticles and nanoparticles, magnetic microparticles and nanoparticles, superparamagnetic microparticles and nanoparticles (e.g., DYNABEADS® particles, available from Invitrogen Group, Carlsbad, CA), fluorescent microparticles and nanoparticles, coated magnetic microparticles and nanoparticles, ferromagnetic microparticles and nanoparticles, coated ferromagnetic microparticles and nanoparticles, and those described in U.S. Patent Publication Nos. 20050260686, entitled "Multiplex flow assays preferably with magnetic particles as solid phase," published on November 24, 2005; 20030132538, entitled "Encapsulation of discrete quanta of fluorescent particles," published on July 17, 2003; 20050118574, entitled "Multiplexed Analysis of Clinical Specimens Apparatus and Method," published on June 2, 2005; 20050277197. Entitled "Microparticles with Multiple Fluorescent Signals and Methods of Using Same," published on December 15, 2005; 20060159962, entitled "Magnetic Microspheres for use in Fluorescence-based Applications," published on July 20, 2006; the entire disclosures of which are incorporated herein by reference for their teaching concerning beads and magnetically responsive materials and beads. Beads may be pre-coupled with a biomolecule or other substance that is able to bind to and form a complex with a biomolecule. Beads may be pre-coupled with an antibody, protein or antigen, DNA/RNA probe or any other molecule with an affinity for a desired target. Examples of droplet actuator techniques for immobilizing magnetically responsive beads and/or non-magnetically responsive beads and/or conducting droplet operations protocols using beads are described in U.S. Patent Application No. 11/639,566, entitled "Droplet-Based Particle Sorting," filed on December 15, 2006; U.S. Patent Application No. 61/039,183, entitled "Multiplexing Bead Detection in a Single Droplet," filed on March 25, 2008; U.S. Patent Application No. 61/047,789, entitled "Droplet Actuator Devices and Droplet Operations Using Beads," filed on April 25, 2008; U.S. Patent Application No. 61/086,183, entitled "Droplet Actuator Devices and Methods for Manipulating Beads," filed on August 5, 2008; International Patent Application No. PCT/US2008/053545, entitled "Droplet Actuator Devices and Methods Employing Magnetically responsive beads," filed on February 11, 2008; International Patent Application No. PCT/US2008/058018, entitled "Bead-based Multiplexed Analytical Methods and Instrumentation," filed on March 24, 2008; International Patent Application No. PCT/US2008/058047, "Bead Sorting on a Droplet Actuator," filed on March 23, 2008; and International Patent Application No. PCT/US2006/047486, entitled "Droplet-based Biochemistry," filed on December 11, 2006; the entire disclosures of which are incorporated herein by reference. Bead characteristics may be employed in the multiplexing aspects of the invention. Examples of beads having characteristics suitable for multiplexing, as well as methods of detecting and analyzing signals emitted from such beads, may be found in U.S. Patent Publication No. 20080305481, entitled "Systems and Methods for Multiplex Analysis of PCR in Real Time," published on December 11, 2008; U.S. Patent Publication No. 20080151240, "Methods and Systems for Dynamic Range Expansion," published on June 26, 2008; U.S. Patent Publication No. 20070207513, entitled "Methods, Products, and Kits for Identifying an Analyte in a Sample," published on September 6, 2007; U.S. Patent Publication No. 20070064990, entitled "Methods and Systems for Image Data Processing," published on March 22, 2007; U.S. Patent Publication No. 20060159962, entitled "Magnetic Microspheres for use in Fluorescence-based Applications," published on July 20, 2006; U.S. Patent Publication No. 20050277197, entitled "Microparticles with Multiple Fluorescent Signals and Methods of Using Same," published on December 15, 2005; and U.S. Patent Publication No. 20050118574, entitled "Multiplexed Analysis of Clinical Specimens Apparatus and Method," published on June 2, 2005; U.S. Patent 6,914,137, entitled "Isolation of nucleic acids," issued on July 5, 2005; each of which is incorporated by reference for its teaching concerning the composition of such beads and conditions for capturing and eluting substances, such as DNA, using such beads.

"Droplet" means a volume of liquid. Typically, a droplet is at least partially bounded by a filler fluid. For example, a droplet may be completely surrounded by a filler fluid or may be bounded by filler fluid and one or more surfaces of the droplet actuator. As another example, a droplet may be bounded by filler fluid, one or more surfaces of the droplet actuator, and/or the atmosphere. As another example, a droplet may be bounded by filler fluid and the atmosphere. Droplets may, for example, be aqueous or non-aqueous or may be mixtures or emulsions including aqueous and non-aqueous components. Droplets may take a wide variety of shapes; nonlimiting examples include generally disc shaped, slug shaped, truncated sphere, ellipsoid, spherical, partially compressed sphere, hemispherical, ovoid, cylindrical, combinations of such shapes, and various shapes formed during droplet operations, such as merging or splitting or formed as a result of contact of such shapes with one or more surfaces of a droplet actuator. For examples of droplet fluids that may be subjected to droplet operations using the approach of the invention, see International Patent Application No. PCT/US 06/47486, entitled, "Droplet-Based Biochemistry," filed on December 11, 2006. In various embodiments, a droplet may include a biological sample, such as whole blood, lymphatic fluid, serum, plasma, sweat, tear, saliva, sputum, cerebrospinal fluid, amniotic fluid, seminal fluid, vaginal excretion, serous fluid, synovial fluid, pericardial fluid, peritoneal fluid, pleural fluid, transudates, exudates, cystic fluid, bile, urine, gastric fluid, intestinal fluid, fecal samples, liquids containing single or multiple cells, liquids containing organelles, fluidized tissues, fluidized organisms, liquids containing multi-celled organisms, biological swabs, biological washes, and combinations of the foregoing. Moreover, a droplet may include a reagent, such as water, deionized water, saline solutions, acidic solutions, basic solutions, detergent solutions and/or buffers. Other examples of droplet contents include reagents, such as a reagent for a biochemical protocol, such as a nucleic acid amplification protocol, an affinity-based assay protocol, an enzymatic assay protocol, a sequencing protocol, and/or a protocol for analyses of biological fluids. Reagent droplets of the invention, such as blunt-ending reagents, A-tailing reagents, ligation reagents, wash buffers, elution buffers, binding buffers, and bead solutions (e.g., SPRI® beads) typically include a surfactant. Reagents may, for example, include from about 0.001 to about 0.5% v/v of an aqueous soluble surfactant, or from about 0.01 to about 0.25% v/v of an aqueous soluble surfactant, or from about 0.01 to about 0.15% v/v of an aqueous soluble surfactant. Reagents may, for example, include from about 0.001 to about 0.5% v/v of an aqueous soluble polysorbate surfactant, or from about 0.01 to about 0.25% v/v of an aqueous soluble polysorbate surfactant, or from about 0.01 to about 0.15% v/v of an aqueous soluble polysorbate surfactant. Reagents may, for example, include from about 0.001 to about 0.5% v/v of an aqueous soluble polyoxyethylene sorbitan monolaurate surfactant, or from about 0.01 to about 0.25% v/v of an aqueous soluble polyoxyethylene sorbitan monolaurate surfactant, or from about 0.01 to about 0.15% v/v of an aqueous soluble polyoxyethylene sorbitan monolaurate surfactant. An example of a suitable polyoxyethylene sorbitan monolaurate is polyoxyethylene (20) sorbitan monolaurate, which is commercially available as TWEEN® 20 from Promega Corp. In certain embodiments, kits of the invention may include one or more droplet actuator cartridges of the invention together with one or more reagents of the invention stored on the cartridges in wet or dry form and/or stored in separate containers for loading on the cartridges.

"Droplet Actuator" means a device for manipulating droplets. For examples of droplet actuators, see Pamula et al., U.S. Patent 6,911,132, entitled "Apparatus for Manipulating Droplets by Electrowetting-Based Techniques," issued on June 28, 2005; Pamula et al., U.S. Patent Application No. 11/343,284, entitled "Apparatuses and Methods for Manipulating Droplets on a Printed Circuit Board," filed on filed on January 30, 2006; Pollack et al., International Patent Application No. PCT/US2006/047486, entitled "Droplet-Based Biochemistry," filed on December 11, 2006; Shenderov, U.S. Patents 6,773,566, entitled "Electrostatic Actuators for Microfluidics and Methods for Using Same," issued on August 10, 2004 and 6,565,727, entitled "Actuators for Microfluidics Without Moving Parts," issued on January 24, 2000; Kim and/or Shah et al., U.S. Patent Application Nos. 10/343,261, entitled "Electrowetting-driven Micropumping," filed on January 27, 2003, 11/275,668, entitled "Method and Apparatus for Promoting the Complete Transfer of Liquid Drops from a Nozzle," filed on January 23, 2006, 11/460,188, entitled "Small Object Moving on Printed Circuit Board," filed on January 23, 2006, 12/465,935, entitled "Method for Using Magnetic Particles in Droplet Microfluidics," filed on May 14, 2009, and 12/513,157, entitled "Method and Apparatus for Real-time Feedback Control of Electrical Manipulation of Droplets on Chip," filed on April 30, 2009; Velev, U.S. Patent 7,547,380, entitled "Droplet Transportation Devices and Methods Having a Fluid Surface," issued on June 16, 2009; Sterling et al., U.S. Patent 7,163,612, entitled "Method, Apparatus and Article for Microfluidic Control via Electrowetting, for Chemical, Biochemical and Biological Assays and the Like," issued on January 16, 2007; Becker and Gascoyne et al., U.S. Patent Nos. 7,641,779, entitled "Method and Apparatus for Programmable fluidic Processing," issued on January 5, 2010, and 6,977,033, entitled "Method and Apparatus for Programmable fluidic Processing," issued on December 20, 2005; Decre et al., U.S. Patent 7,328,979, entitled "System for Manipulation of a Body of Fluid," issued on February 12, 2008; Yamakawa et al., U.S. Patent Pub. No. 20060039823, entitled "Chemical Analysis Apparatus," published on February 23, 2006; Wu, International Patent Pub. No. WO/2009/003184, entitled "Digital Microfluidics Based Apparatus for Heat-exchanging Chemical Processes," published on December 31, 2008; Fouillet et al., U.S. Patent Pub. No. 20090192044, entitled "Electrode Addressing Method," published on July 30, 2009; Fouillet et al., U.S. Patent 7,052,244, entitled "Device for Displacement of Small Liquid Volumes Along a Micro-catenary Line by Electrostatic Forces," issued on May 30, 2006; Marchand et al., U.S. Patent Pub. No. 20080124252, entitled "Droplet Microreactor," published on May 29, 2008; Adachi et al., U.S. Patent Pub. No. 20090321262, entitled "Liquid Transfer Device," published on December 31, 2009; Roux et al., U.S. Patent Pub. No. 20050179746, entitled "Device for Controlling the Displacement of a Drop Between two or Several Solid Substrates," published on August 18, 2005; Dhindsa et al., "Virtual Electrowetting Channels: Electronic Liquid Transport with Continuous Channel Functionality," Lab Chip, 10:832-836 (2010); the entire disclosures of which are incorporated herein by reference, along with their priority documents. Certain droplet actuators will include one or more substrates arranged with a gap therebetween and electrodes associated with (e.g., layered on, attached to, and/or embedded in) the one or more substrates and arranged to conduct one or more droplet operations. For example, certain droplet actuators will include a base (or bottom) substrate, droplet operations electrodes associated with the substrate, one or more dielectric layers atop the substrate and/or electrodes, and optionally one or more hydrophobic layers atop the substrate, dielectric layers and/or the electrodes forming a droplet operations surface. A top substrate may also be provided, which is separated from the droplet operations surface by a gap, commonly referred to as a droplet operations gap. Various electrode arrangements on the top and/or bottom substrates are discussed in the above-referenced patents and applications and certain novel electrode arrangements are discussed in the description of the invention. During droplet operations it is preferred that droplets remain in continuous contact or frequent contact with a ground or reference electrode. A ground or reference electrode may be associated with the top substrate facing the gap, the bottom substrate facing the gap, in the gap. Where electrodes are provided on both substrates, electrical contacts for coupling the electrodes to a droplet actuator instrument for controlling or monitoring the electrodes may be associated with one or both plates. In some cases, electrodes on one substrate are electrically coupled to the other substrate so that only one substrate is in contact with the droplet actuator. In one embodiment, a conductive material (e.g., an epoxy, such as MASTER BOND™ Polymer System EP79, available from Master Bond, Inc., Hackensack, NJ) provides the electrical connection between electrodes on one substrate and electrical paths on the other substrates, e.g., a ground electrode on a top substrate may be coupled to an electrical path on a bottom substrate by such a conductive material. Where multiple substrates are used, a spacer may be provided between the substrates to determine the height of the gap therebetween and define dispensing reservoirs. The spacer height or gap height may, for example, be from about 5 µm to about 5 mm, or from about 5 µm to about 1 µm, or from about 5 µm to about 600 µm, or about 100 µm to about 400 µm, or about 200 µm to about 350 µm, or about 250 µm to about 300 µm, or about 275 µm. The spacer may, for example, be formed of a layer of projections form the top or bottom substrates, and/or a material inserted between the top and bottom substrates. One or more openings may be provided in the one or more substrates for forming a fluid path through which liquid may be delivered into the droplet operations gap. The one or more openings may in some cases be aligned for interaction with one or more electrodes, e.g., aligned such that liquid flowed through the opening will come into sufficient proximity with one or more droplet operations electrodes to permit a droplet operation to be effected by the droplet operations electrodes using the liquid. The base (or bottom) and top substrates may in some cases be formed as one integral component. One or more reference electrodes may be provided on the base (or bottom) and/or top substrates and/or in the gap. Examples of reference electrode arrangements are provided in the above referenced patents and patent applications. In various embodiments, the manipulation of droplets by a droplet actuator may be electrode mediated, e.g., electrowetting mediated or dielectrophoresis mediated or Coulombic force mediated. Examples of other techniques for controlling droplet operations that may be used in the droplet actuators of the invention include using devices that induce hydrodynamic fluidic pressure, such as those that operate on the basis of mechanical principles (e.g. external syringe pumps, pneumatic membrane pumps, vibrating membrane pumps, vacuum devices, centrifugal forces, piezoelectric/ultrasonic pumps and acoustic forces); electrical or magnetic principles (e.g. electroosmotic flow, electrokinetic pumps, ferrofluidic plugs, electrohydrodynamic pumps, attraction or repulsion using magnetic forces and magnetohydrodynamic pumps); thermodynamic principles (e.g. gas bubble generation/phase-change-induced volume expansion); other kinds of surface-wetting principles (e.g. electrowetting, and optoelectrowetting, as well as chemically, thermally, structurally and radioactively induced surface-tension gradients); gravity; surface tension (e.g., capillary action); electrostatic forces (e.g., electroosmotic flow); centrifugal flow (substrate disposed on a compact disc and rotated); magnetic forces (e.g., oscillating ions causes flow); magnetohydrodynamic forces; and vacuum or pressure differential. In certain embodiments, combinations of two or more of the foregoing techniques may be employed to conduct a droplet operation in a droplet actuator of the invention. Similarly, one or more of the foregoing may be used to deliver liquid into a droplet operations gap, e.g., from a reservoir in another device or from an external reservoir of the droplet actuator (e.g., a reservoir associated with a droplet actuator substrate and a fluid path from the reservoir into the droplet operations gap). Droplet operations surfaces of certain droplet actuators of the invention may be made from hydrophobic materials or may be coated or treated to make them hydrophobic. For example, in some cases some portion or all of the droplet operations surfaces may be derivatized with low surface-energy materials or chemistries, e.g., by deposition or using in situ synthesis using compounds such as poly- or per-fluorinated compounds in solution or polymerizable monomers. Examples include TEFLON® AF (available from DuPont, Wilmington, DE), members of the cytop family of materials, coatings in the FLUOROPEL® family of hydrophobic and superhydrophobic coatings (available from Cytonix Corporation, Beltsville, MD), silane coatings, fluorosilane coatings, hydrophobic phosphonate derivatives (e.g.., those sold by Aculon, Inc), and NOVEC™ electronic coatings (available from 3M Company, St. Paul, MN), and other fluorinated monomers for plasma-enhanced chemical vapor deposition (PECVD). In some cases, the droplet operations surface may include a hydrophobic coating having a thickness ranging from about 10 nm to about 1,000 nm. Moreover, in some embodiments, the top substrate of the droplet actuator includes an electrically conducting organic polymer, which is then coated with a hydrophobic coating or otherwise treated to make the droplet operations surface hydrophobic. For example, the electrically conducting organic polymer that is deposited onto a plastic substrate may be poly(3,4-ethylenedioxythiophene) poly(styrenesulfonate) (PEDOT:PSS). Other examples of electrically conducting organic polymers and alternative conductive layers are described in Pollack et al., International Patent Application No. PCT/US2010/040705, entitled "Droplet Actuator Devices and Methods," the entire disclosure of which is incorporated herein by reference. One or both substrates may be fabricated using a printed circuit board (PCB), glass, indium tin oxide (ITO)-coated glass, and/or semiconductor materials as the substrate. When the substrate is ITO-coated glass, the ITO coating is preferably a thickness in the range of about 20 to about 200 nm, preferably about 50 to about 150 nm, or about 75 to about 125 nm, or about 100 nm. In some cases, the top and/or bottom substrate includes a PCB substrate that is coated with a dielectric, such as a polyimide dielectric, which may in some cases also be coated or otherwise treated to make the droplet operations surface hydrophobic. When the substrate includes a PCB, the following materials are examples of suitable materials: MITSUI™ BN-300 (available from MITSUI Chemicals America, Inc., San Jose CA); ARLON™ 11N (available from Arlon, Inc, Santa Ana, CA).; NELCO® N4000-6 and N5000-30/32 (available from Park Electrochemical Corp., Melville, NY); ISOLA™ FR406 (available from Isola Group, Chandler, AZ), especially IS620; fluoropolymer family (suitable for fluorescence detection since it has low background fluorescence); polyimide family; polyester; polyethylene naphthalate; polycarbonate; polyetheretherketone; liquid crystal polymer; cyclo-olefin copolymer (COC); cyclo-olefin polymer (COP); aramid; THERMOUNT® nonwoven aramid reinforcement (available from DuPont, Wilmington, DE); NOMEX® brand fiber (available from DuPont, Wilmington, DE); and paper. Various materials are also suitable for use as the dielectric component of the substrate. Examples include: vapor deposited dielectric, such as PARYLENE™ C (especially on glass) and PARYLENE™ N (available from Parylene Coating Services, Inc., Katy, TX); TEFLON® AF coatings; cytop; soldermasks, such as liquid photoimageable soldermasks (e.g., on PCB) like TAIYO™ PSR4000 series, TAIYO™ PSR and AUS series (available from Taiyo America, Inc. Carson City, NV) (good thermal characteristics for applications involving thermal control), and PROBIMER™ 8165 (good thermal characteristics for applications involving thermal control (available from Huntsman Advanced Materials Americas Inc., Los Angeles, CA); dry film soldermask, such as those in the VACREL® dry film soldermask line (available from DuPont, Wilmington, DE); film dielectrics, such as polyimide film (e.g., KAPTON® polyimide film, available from DuPont, Wilmington, DE), polyethylene, and fluoropolymers (e.g., FEP), polytetrafluoroethylene; polyester; polyethylene naphthalate; cyclo-olefin copolymer (COC); cyclo-olefin polymer (COP); any other PCB substrate material listed above; black matrix resin; and polypropylene. Droplet transport voltage and frequency may be selected for performance with reagents used in specific assay protocols. Design parameters may be varied, e.g., number and placement of on-actuator reservoirs, number of independent electrode connections, size (volume) of different reservoirs, placement of magnets/bead washing zones, electrode size, inter-electrode pitch, and gap height (between top and bottom substrates) may be varied for use with specific reagents, protocols, droplet volumes, etc. In some cases, a substrate of the invention may derivatized with low surface-energy materials or chemistries, e.g., using deposition or in situ synthesis using poly- or per-fluorinated compounds in solution or polymerizable monomers. Examples include TEFLON® AF coatings and FLUOROPEL® coatings for dip or spray coating, and other fluorinated monomers for plasma-enhanced chemical vapor deposition (PECVD). Additionally, in some cases, some portion or all of the droplet operations surface may be coated with a substance for reducing background noise, such as background fluorescence from a PCB substrate. For example, the noise-reducing coating may include a black matrix resin, such as the black matrix resins available from Toray industries, Inc., Japan. Electrodes of a droplet actuator are typically controlled by a controller or a processor, which is itself provided as part of a system, which may include processing functions as well as data and software storage and input and output capabilities. Reagents may be provided on the droplet actuator in the droplet operations gap or in a reservoir fluidly coupled to the droplet operations gap. The reagents may be in liquid form, e.g., droplets, or they may be provided in a reconstitutable form in the droplet operations gap or in a reservoir fluidly coupled to the droplet operations gap. Reconstitutable reagents may typically be combined with liquids for reconstitution. An example of reconstitutable reagents suitable for use with the invention includes those described in Meathrel, et al., U.S. Patent 7,727,466, entitled "Disintegratable films for diagnostic devices," granted on June 1, 2010.

"Droplet operation" means any manipulation of a droplet on a droplet actuator. A droplet operation may, for example, include: loading a droplet into the droplet actuator; dispensing one or more droplets from a source droplet; splitting, separating or dividing a droplet into two or more droplets; transporting a droplet from one location to another in any direction; merging or combining two or more droplets into a single droplet; diluting a droplet; mixing a droplet; agitating a droplet; deforming a droplet; retaining a droplet in position; incubating a droplet; heating a droplet; vaporizing a droplet; cooling a droplet; disposing of a droplet; transporting a droplet out of a droplet actuator; other droplet operations described herein; and/or any combination of the foregoing. The terms "merge," "merging," "combine," "combining" and the like are used to describe the creation of one droplet from two or more droplets. It should be understood that when such a term is used in reference to two or more droplets, any combination of droplet operations that are sufficient to result in the combination of the two or more droplets into one droplet may be used. For example, "merging droplet A with droplet B," can be achieved by transporting droplet A into contact with a stationary droplet B, transporting droplet B into contact with a stationary droplet A, or transporting droplets A and B into contact with each other. The terms "splitting," "separating" and "dividing" are not intended to imply any particular outcome with respect to volume of the resulting droplets (i.e., the volume of the resulting droplets can be the same or different) or number of resulting droplets (the number of resulting droplets may be 2, 3, 4, 5 or more). The term "mixing" refers to droplet operations which result in more homogenous distribution of one or more components within a droplet. Examples of "loading" droplet operations include microdialysis loading, pressure assisted loading, robotic loading, passive loading (e.g., gravity-assisted loading), and pipette loading. The term "incubating" or "incubation" refers to a droplet maintained at a particular temperature or temperature profile for a period of time; the droplet may be retained in a stationary position during incubation, or may be in constant motion, or subjected to periodic droplet operations, such as split-merge-split-merge or transport back and forth or in a loop. Droplet operations may be on-actuator, meaning that they take place on a droplet operations surface of a droplet actuator, or in a droplet operations gap of a droplet actuator, and in either case, the droplet may be separated from one or more surfaces of the droplet actuator by a filler fluid. Droplet operations may be electrode-mediated. In some cases, droplet operations are further facilitated by the use of hydrophilic and/or hydrophobic regions on surfaces, and/or by physical obstacles, and/or by geometry of the droplet actuator, such as a differential in gap height. For examples of droplet operations, see the patents and patent applications cited above under the definition of "droplet actuator." In some cases, droplet operations may be mediated by a differential in droplet actuator gap height or a difference in dimensions which causes droplet deformation as a droplet moves into a conformation that is more energetically stable. Impedance or capacitance sensing or imaging techniques or visual observations may sometimes be used to determine or confirm the outcome of a droplet operation. Examples of such techniques are described in Sturmer et al., International Patent Pub. No. WO/2008/101194, entitled "Capacitance Detection in a Droplet Actuator," published on August 21, 2008, the entire disclosure of which is incorporated herein by reference. Generally speaking, the sensing or imaging techniques may be used to confirm the presence or absence of a droplet at a specific electrode. For example, the presence of a dispensed droplet at the destination electrode following a droplet dispensing operation confirms that the droplet dispensing operation was effective. Similarly, the presence of a droplet at a detection spot at an appropriate step in an assay protocol may confirm that a previous set of droplet operations has successfully produced a droplet for detection. Droplet transport time can be quite fast. For example, in various embodiments, transport of a droplet from one electrode to the next may exceed about 1 sec, or about 0.1 sec, or about 0.01 sec, or about 0.001 sec. In one embodiment, the electrode is operated in AC mode but is switched to DC mode for imaging. It is helpful for conducting droplet operations for the footprint area of droplet to be similar to electrowetting area; in other words, 1x-, 2x- 3x-droplets are usefully controlled operated using 1, 2, and 3 electrodes, respectively. If the droplet footprint is greater than the number of electrodes available for conducting a droplet operation at a given time, the difference between the droplet size and the number of electrodes should typically not be greater than 1; in other words, a 2x droplet is usefully controlled using 1 electrode and a 3x droplet is usefully controlled using 2 electrodes. When droplets include beads, it is useful for droplet size to be equal to the number of electrodes controlling the droplet, e.g., transporting the droplet. However, it should be noted that by varying interfacial tension, transport speed, etc., very large droplets can be transported using electrodes or sets of electrodes having a footprint that is significantly smaller than the droplet foot print; thus, while the foregoing sentence describes a useful rule of thumb, it should not be construed as limiting the invention.

"Filler fluid" means a fluid that is immiscible or substantially immiscible with a droplet. In some embodiments, a filler fluid is associated with a droplet operations substrate of a droplet actuator, which fluid is sufficiently immiscible with a droplet phase to render the droplet phase subject to electrode-mediated droplet operations. For example, the gap of a droplet actuator is typically filled with a filler fluid. The filler fluid may, for example, be a low-viscosity oil, such as silicone oil or hexadecane filler fluid. The filler fluid may fill the entire gap of the droplet actuator or may coat one or more surfaces of the droplet actuator. Filler fluids may be conductive or non-conductive. Filler fluids may, for example, be doped with surfactants or other additives. For example, additives may be selected to improve droplet operations and/or reduce loss of reagent or target substances from droplets, formation of microdroplets, cross contamination between droplets, contamination of droplet actuator surfaces, degradation of droplet actuator materials, etc. Composition of the filler fluid, including surfactant doping, may be selected for performance with reagents used in the specific assay protocols and effective interaction or non-interaction with droplet actuator materials. Examples of filler fluids and filler fluid formulations suitable for use with the invention are provided in Srinivasan et al, International Patent Pub. Nos. WO/2010/027894, entitled "Droplet Actuators, Modified Fluids and Methods," published on March 11, 2010, and WO/2009/021173, entitled "Use of Additives for Enhancing Droplet Operations," published on February 12, 2009; Sista et al., International Patent Pub. No. WO/2008/098236, entitled "Droplet Actuator Devices and Methods Employing Magnetically responsive beads," published on August 14, 2008; and Monroe et al., U.S. Patent Publication No. 20080283414, entitled "Electrowetting Devices," filed on May 17, 2007; the entire disclosures of which are incorporated herein by reference, as well as the other patents and patent applications cited herein. It is noted that any of the library preparation steps described herein may be conducted in a filler fluid, e.g., a filler fluid substantially filling a droplet operations gap of a droplet actuator. In one embodiment, the filler fluid comprises silicone oil having a kinematic viscosity ranging from about 1 to about 6.5 cST, or from about 1.5 to about 5.5 cSt, or from about 2 to about 5 cSt. This silicone oil may be doped with a surfactant. In one embodiment, the filler fluid for conducting library construction includes from about 2 cSt to about 7 cSt silicone oil with from about 0.0001 to about 1% v/v of an oil soluble surfactant, or from about 0.001 to about 0.1% v/v of an oil soluble surfactant, or from about 0.001 to about 0.01% v/v of an oil soluble surfactant, or from about 0.005 to about 0.01% v/v of an oil soluble surfactant. Thus, in one embodiment, the invention provides a kit comprising a droplet actuator cartridge of the invention and a filler fluid having such characteristics. In another embodiment, the filler fluid for conducting library construction includes from about 2 cSt to about 7 cSt silicone oil with from about 0.0001 to about 1% v/v of a fatty acid ester of sorbitan, or from about 0.001 to about 0.1% v/v of a fatty acid ester of sorbitan, or from about 0.001 to about 0.01% v/v of a fatty acid ester of sorbitan, or from about 0.005 to about 0.01% v/v of a fatty acid ester of sorbitan. Thus, in one embodiment, the invention provides a kit comprising a droplet actuator cartridge of the invention and a filler fluid having such characteristics. In another embodiment, the filler fluid for conducting library construction includes from about 2 cSt to about 7 cSt silicone oil with from about 0.0001 to about 1% v/v of a sorbitan ester that is soluble in the silicone oil, or from about 0.001 to about 0.1% v/v of a sorbitan ester that is soluble in the silicone oil, or from about 0.001 to about 0.01% v/v of a sorbitan ester that is soluble in the silicone oil, or from about 0.005 to about 0.01% v/v of a sorbitan ester that is soluble in the silicone oil. Thus, in one embodiment, the invention provides a kit comprising a droplet actuator cartridge of the invention and a filler fluid having such characteristics. In another embodiment, the filler fluid for conducting library construction includes from about 2 cSt to about 7 cSt silicone oil with from about 0.0001 to about 1% v/v of sorbitan trioleate, or from about 0.001 to about 0.1% v/v of an ester of sorbitan trioleate, or from about 0.001 to about 0.01% v/v of sorbitan trioleate, or from about 0.005 to about 0.01% v/v of an ester of sorbitan trioleate. Sorbitan trioleate is available as SPAN® 85 surfactant formulation from Sigma Aldrich. Thus, in one embodiment, the invention provides a kit comprising a droplet actuator cartridge of the invention and a filler fluid having such characteristics. In one embodiment, the filler fluid for conducting library construction includes 2cSt silicone oil with from about 0.01 to about 2% v/v of an oil soluble surfactant, or from about 0.1 to about 1% v/v of an oil soluble surfactant, or from about 0.1 to about 0.5% v/v of an oil soluble surfactant. Thus, in one embodiment, the invention provides a kit comprising a droplet actuator cartridge of the invention and a filler fluid having such characteristics. In another embodiment, the filler fluid for conducting library construction includes 2cSt silicone oil with from about 0.01 to about 2% v/v of an octylphenol ethoxylate surfactant that is soluble in the oil, or from about 0.1 to about 1% v/v of an octylphenol ethoxylate surfactant that is soluble in the oil, or from about 0.1 to about 0.5% v/v of an octylphenol ethoxylate surfactant that is soluble in the oil. For example, a suitabla an octylphenol ethoxylate surfactant is TRITON® X-15, which is an octylphenol ethoxylate having 15 ethylene oxide units. Thus, in one embodiment, the invention provides a kit comprising a droplet actuator cartridge of the invention and a filler fluid having such characteristics. In another embodiment, the surfactant comprises a block copolymer. For example, the block copolymer may include a poly(tetrafluoroethylene) block and poly(dimethylsiloxane). In one embodiment, the poly(tetrafluoroethylene) block may include from about 5 to about 50 repeat units. In one embodiment, the poly(dimethylsiloxane) may have a MW ranging from about 400 to about 10,000 MW. In another embodiment, the surfactant comprises a hydrophilic silicone or siloxane. For example, the surfactant may comprise dimethylsiloxane backbones in which some of the methyl groups are replaced by polyalkylenoxy or pyrrolidone groups with propyl group as a spacer. Further examples of suitable surfactants include polyalkylene oxide silicones, hydroxylic and cationic silicones, poly(alkyleneoxy) functional metal organics and silanes, and trisiloxanes. Further examples include DBE-712 (dimethylsiloxane-ethylene oxide block copolymer), DBP-732 (dimethylsiloxane - (60% propylene oxide-40% ethylene oxide) block copolymer), DBE-224 (dimethylsiloxane-ethylene oxide block copolymer), QMS-435 (35-45% (trimethylphenethyl)methylsiloxane - 55-65% dimethylsiloxane copolymer, chloride salt), CMS-222 ((carbinol functional)methylsiloxane-Dimethylsiloxane copolymer), AKT841 (O-allyloxy(polyethyleneoxy)triisopropxytitanate), SIT8192.0 (N-(3-Triethoxysilylpropyl)-4-hydroxybutyramide), SIM6492.7 (2-[Methoxy(polyethyleneoxy)propyl]trimethoxysilane), SIH6185.0 ((hydroxypolyethyleneoxypropyl)heptamethyltrisiloxane), SIM6492.6 ((methoxypolyethyleneoxypropyl)heptamethyltrisiloxane), and SIA0075.0 ((Acetoxypolyethyleneoxypropyl)heptamethyltrisiloxane), all available from Gelest, Inc., Morrsiville, PA.

"Immobilize" with respect to magnetically responsive beads, means that the beads are substantially restrained in position in a droplet or in filler fluid on a droplet actuator. For example, in one embodiment, immobilized beads are sufficiently restrained in position in a droplet to permit execution of a droplet splitting operation, yielding one droplet with substantially all of the beads and one droplet substantially lacking in the beads.

"Magnetically responsive" means responsive to a magnetic field. "Magnetically responsive beads" include or are composed of magnetically responsive materials. Examples of magnetically responsive materials include paramagnetic materials, ferromagnetic materials, ferrimagnetic materials, and metamagnetic materials. Examples of suitable paramagnetic materials include iron, nickel, and cobalt, as well as metal oxides, such as Fe₃O₄, BaFe₁₂O₁₉, CoO, NiO, Mn₂O₃, Cr₂O₃, and CoMnP.

"Transporting into the magnetic field of a magnet," "transporting towards a magnet," and the like, as used herein to refer to droplets and/or magnetically responsive beads within droplets, is intended to refer to transporting into a region of a magnetic field capable of substantially attracting magnetically responsive beads in the droplet. Similarly, "transporting away from a magnet or magnetic field," "transporting out of the magnetic field of a magnet," and the like, as used herein to refer to droplets and/or magnetically responsive beads within droplets, is intended to refer to transporting away from a region of a magnetic field capable of substantially attracting magnetically responsive beads in the droplet, whether or not the droplet or magnetically responsive beads is completely removed from the magnetic field. It will be appreciated that in any of such cases described herein, the droplet may be transported towards or away from the desired region of the magnetic field, and/or the desired region of the magnetic field may be moved towards or away from the droplet. Reference to an electrode, a droplet, or magnetically responsive beads being "within" or "in" a magnetic field, or the like, is intended to describe a situation in which the electrode is situated in a manner which permits the electrode to transport a droplet into and/or away from a desired region of a magnetic field, or the droplet or magnetically responsive beads is/are situated in a desired region of the magnetic field, in each case where the magnetic field in the desired region is capable of substantially attracting any magnetically responsive beads in the droplet. Similarly, reference to an electrode, a droplet, or magnetically responsive beads being "outside of" or "away from" a magnetic field, and the like, is intended to describe a situation in which the electrode is situated in a manner which permits the electrode to transport a droplet away from a certain region of a magnetic field, or the droplet or magnetically responsive beads is/are situated away from a certain region of the magnetic field, in each case where the magnetic field in such region is not capable of substantially attracting any magnetically responsive beads in the droplet or in which any remaining attraction does not eliminate the effectiveness of droplet operations conducted in the region. In various aspects of the invention, a system, a droplet actuator, or another component of a system may include a magnet, such as one or more permanent magnets (e.g., a single cylindrical or bar magnet or an array of such magnets, such as a Halbach array) or an electromagnet or array of electromagnets, to form a magnetic field for interacting with magnetically responsive beads or other components on the droplet actuator. Such interactions may, for example, include substantially immobilizing or restraining movement or flow of magnetically responsive beads during storage or in a droplet during a droplet operation or pulling magnetically responsive beads out of a droplet.

"Washing" with respect to washing a bead means reducing the amount and/or concentration of one or more substances in contact with the bead or exposed to the bead from a droplet in contact with the bead. The reduction in the amount and/or concentration of the substance may be partial, substantially complete, or even complete. The substance may be any of a wide variety of substances; examples include target substances for further analysis, and unwanted substances, such as components of a sample, contaminants, and/or excess reagent. In some embodiments, a washing operation begins with a starting droplet in contact with a magnetically responsive bead, where the droplet includes an initial amount and initial concentration of a substance. The washing operation may proceed using a variety of droplet operations. The washing operation may yield a droplet including the magnetically responsive bead, where the droplet has a total amount and/or concentration of the substance which is less than the initial amount and/or concentration of the substance. Examples of suitable washing techniques are described in Pamula et al., U.S. Patent 7,439,014, entitled "Droplet-Based Surface Modification and Washing," granted on October 21, 2008, the entire disclosure of which is incorporated herein by reference.

The terms "top," "bottom," "over," "under," and "on" are used throughout the description with reference to the relative positions of components of the droplet actuator, such as relative positions of top and bottom substrates of the droplet actuator. It will be appreciated that the droplet actuator may be functional regardless of its orientation in space.

When a liquid in any form (e.g., a droplet or a continuous body, whether moving or stationary) is described as being "on", "at", or "over" an electrode, array, matrix or surface, such liquid could be either in direct contact with the electrode/array/matrix/surface, or could be in contact with one or more layers or films that are interposed between the liquid and the electrode/array/matrix/surface.

When a droplet is described as being "on" or "loaded on" or "loaded into" a droplet actuator, it should be understood that the droplet is arranged on the droplet actuator in a manner which facilitates using the droplet actuator to conduct one or more droplet operations on the droplet, the droplet is arranged on the droplet actuator in a manner which facilitates sensing of a property of or a signal from the droplet, and/or the droplet has been subjected to a droplet operation on the droplet actuator.

Where chemical reactions are described, it is presumed that the reactions may take place at any temperature and for any duration that achieves the stated result.

### 6 Brief Description of the Drawings

Figure 1 illustrates a flow diagram of an example of a protocol for construction of a nucleic acid library;
Figure 2 shows a photograph of the agarose gel used to calculate the library output data that is shown in Table 3;
Figure 3 shows a plot of the calculated yield from agarose gel analysis of 7 additional runs of the on-actuator library construction protocol;
Figure 4 shows a plot of a comparison of the on-bench and on-droplet actuator implementation of the elution and DNA clean-up steps in the paired-end protocol;
Figures 5A through 5M illustrate top views of an example of a portion of an electrode arrangement of a droplet actuator and show a process of preparing nucleic acid for construction of a nucleic acid library;
Figure 6 illustrates a flow diagram of a method, which is another way of depicting the process of preparing nucleic acid shown in Figures 6A through 6H;
Figure 7 illustrates a flow diagram of a method, which is another way of depicting the alcohol-based bead wash/elute process shown in Figures 6A through 6H;
Figures 8A, 8B, and 8C illustrate top views of an example of a portion of an electrode arrangement of a droplet actuator and show a process of snapping off beads, while leaving behind with the beads the smallest amount of liquid possible;
Figure 9 illustrates a top view of an example of a droplet actuator that is suitable for use in conducting a multiplexed nucleic acid library construction protocol;
Figure 10 illustrates a top view of another example of an electrode arrangement configured for processing of nucleic acid on a droplet actuator for construction of a nucleic acid library;
Figure 11 illustrates a top view of another example of an electrode arrangement configured for processing of nucleic acid on a droplet actuator for construction of a nucleic acid library;
Figure 12 illustrates a top view of another example of an electrode arrangement configured for processing of nucleic acid on a droplet actuator for construction of a nucleic acid library;
Figures 13A and 13B illustrate a top view and a perspective view, respectively, of another example of a droplet actuator suitable for use in conducting a multiplexed nucleic acid library construction protocol;
Figure 13C illustrates a top view of an example implementation of the top substrate of the droplet actuator of Figures 13A and 13B;
Figure 14 illustrates a top view of an example of a bottom substrate of the droplet actuator of

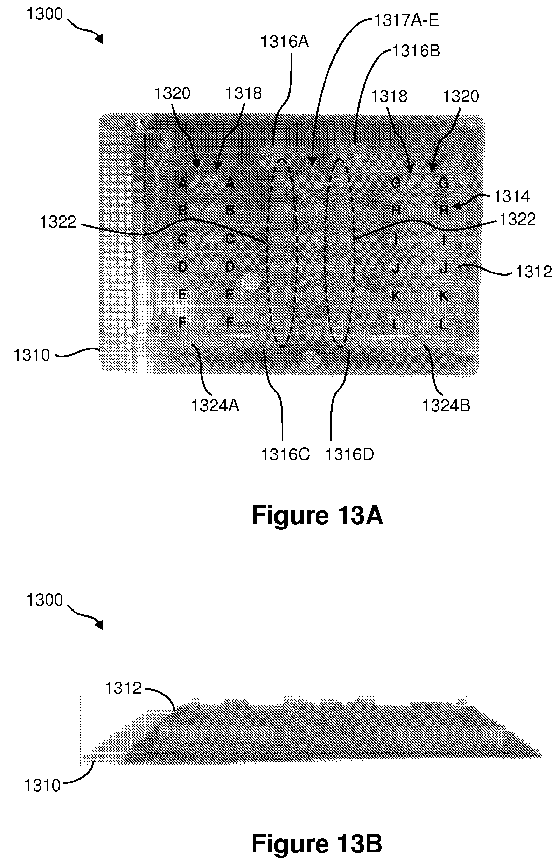

Figures 13A and 13B, which has an electrode arrangement patterned thereon;

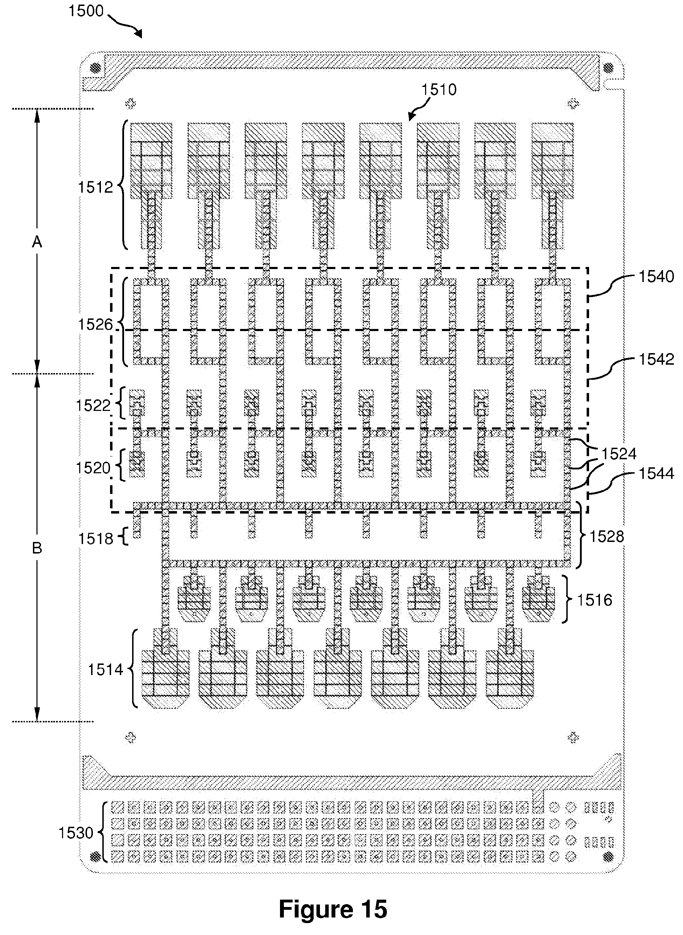

Figure 15 illustrates a top view of an example of a bottom substrate of a droplet actuator that has an electrode arrangement patterned thereon for optimized droplet transporting and routing time;

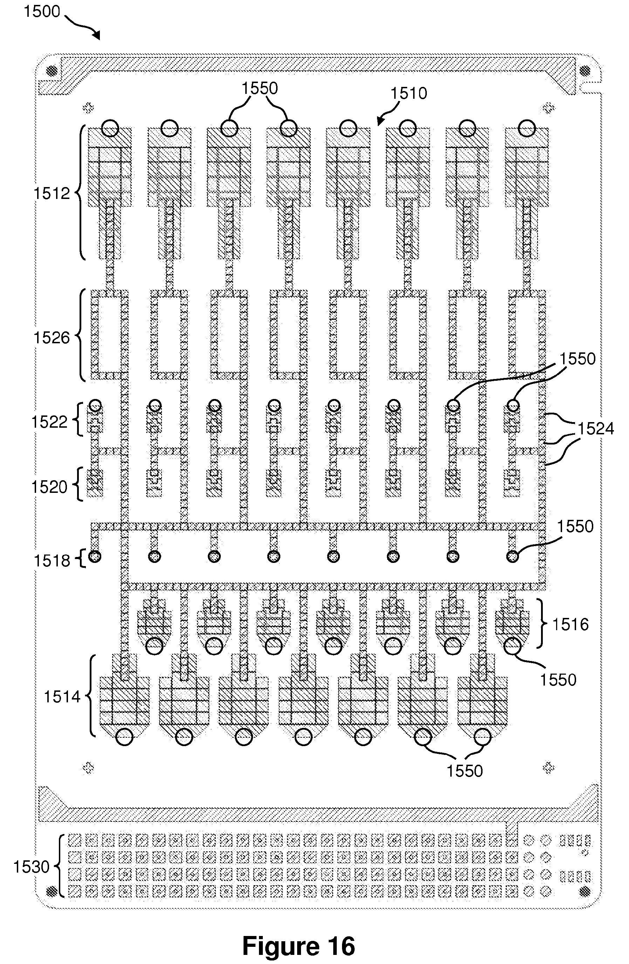

Figure 16 illustrates a top view of the bottom substrate of Figure 15 in relation to openings for filling the on-actuator fluid reservoirs supported by the electrode arrangement of Figure 15;

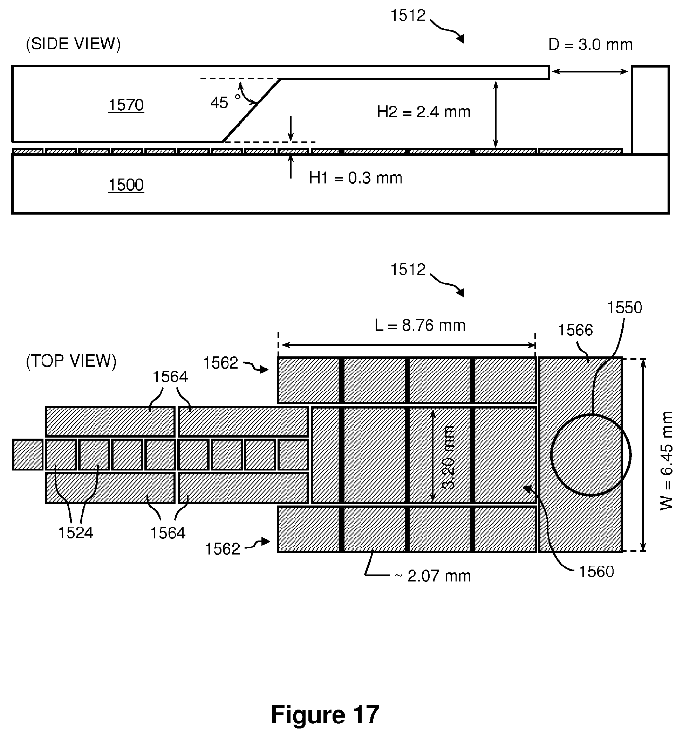

Figures 17, 18, 19, 20, and 21 illustrate views of the various fluid reservoirs that are supported by the electrode arrangement of Figure 15;

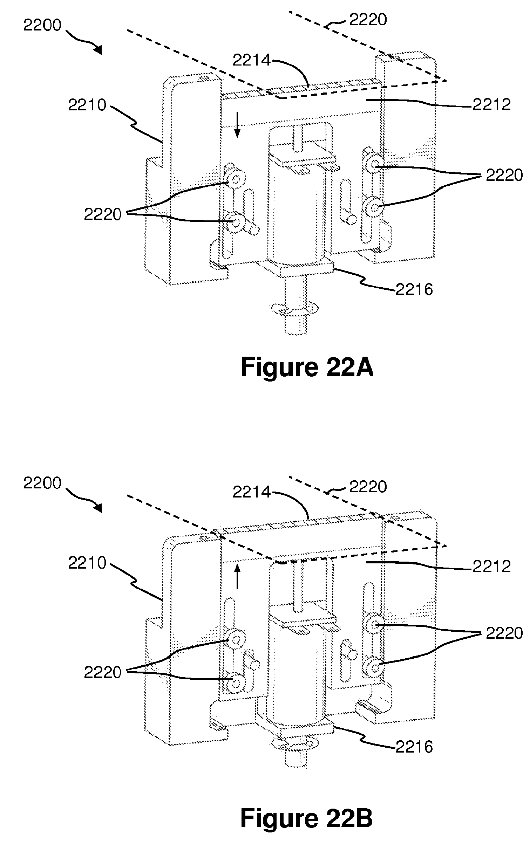

Figures 22A and 22B illustrate perspective views of a magnet actuator;

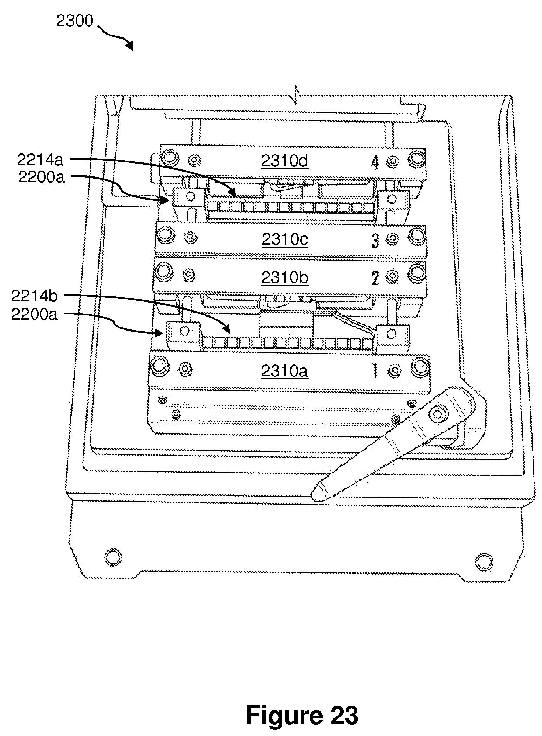

Figure 23 illustrates a top view of an example of a mechanical fixture for holding one or more magnet actuators and one or more heater mechanisms;

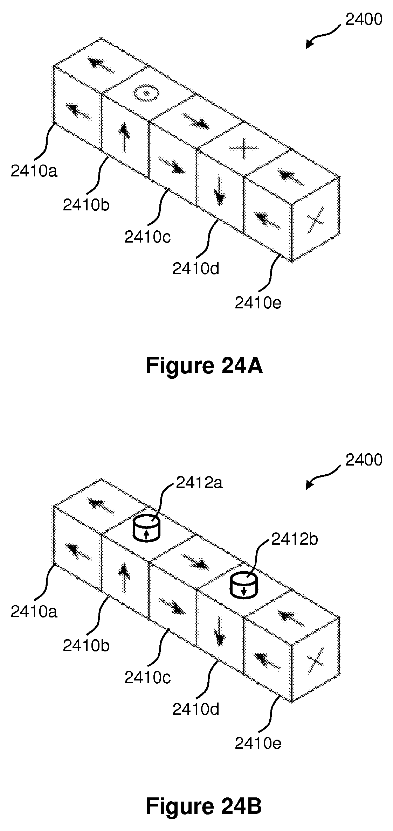

Figures 24A and 24B illustrate a perspective view of examples of a Halbach magnet array;

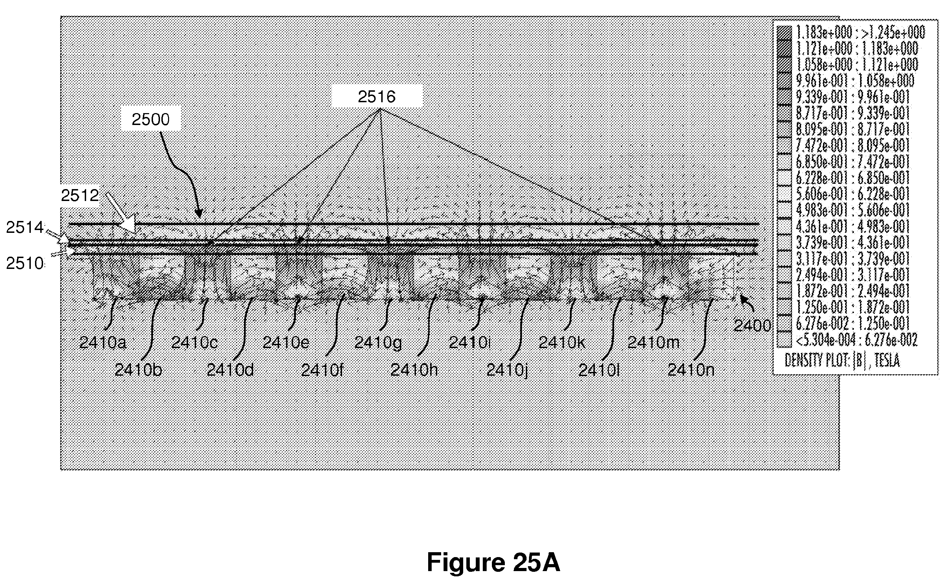

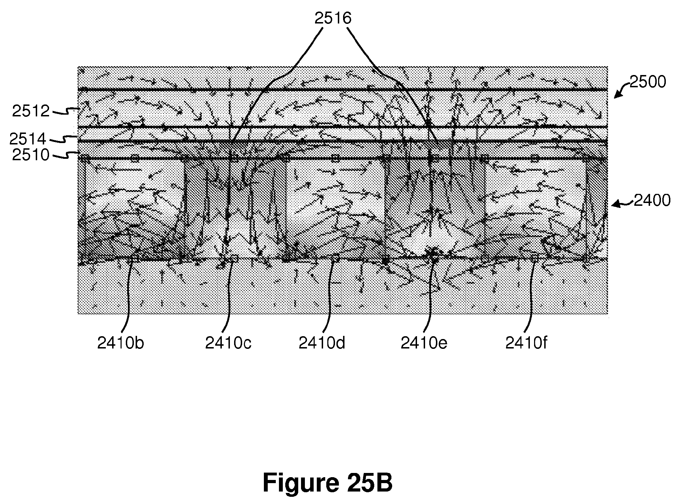

Figures 25A and 25B illustrate the relationship of the magnetic fields of, for example, the Halbach magnet array of Figure 24A to the electrodes of a droplet actuator;

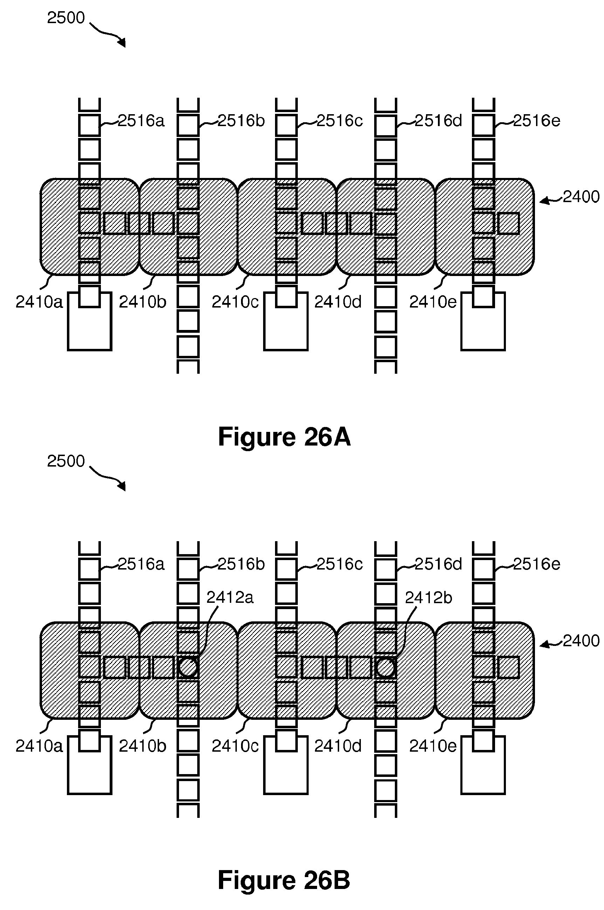

Figures 26A and 26B illustrate another view (i.e., a top view) of the droplet actuator of Figures 25A and 25B in relation to the Halbach magnet array of Figures 24A and 24B;

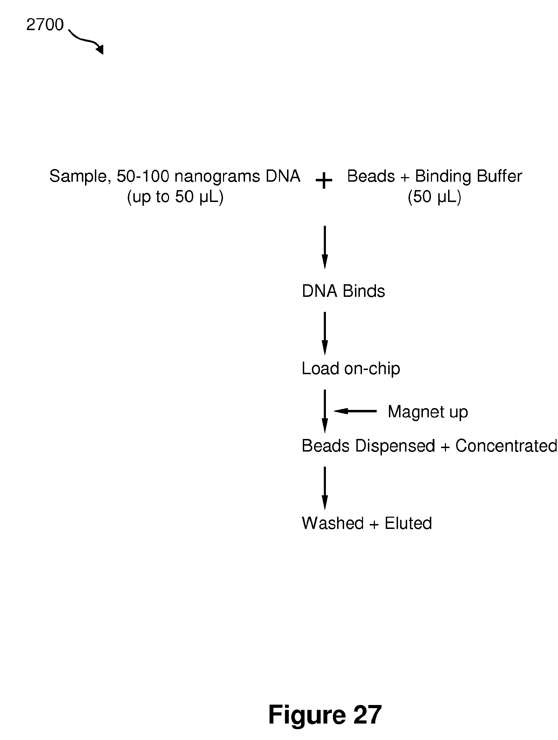

Figure 27 illustrates a flow diagram of a method of sample concentration in a droplet actuator;

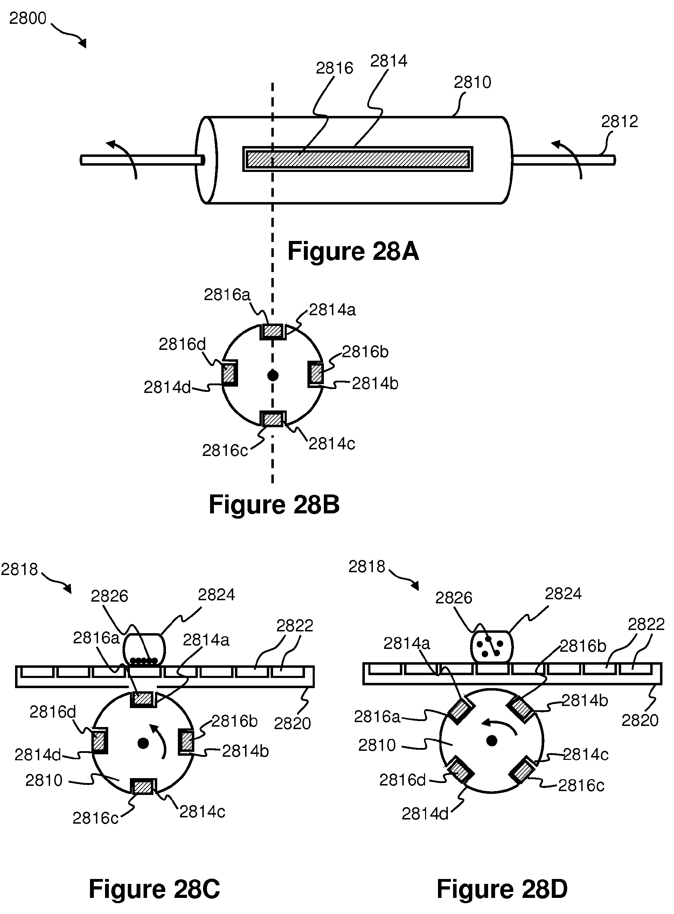

Figures 28A through 28D illustrate a side view and cross-sectional views of a roller assembly that includes an arrangement of other components that may be useful with respect to droplet actuators;

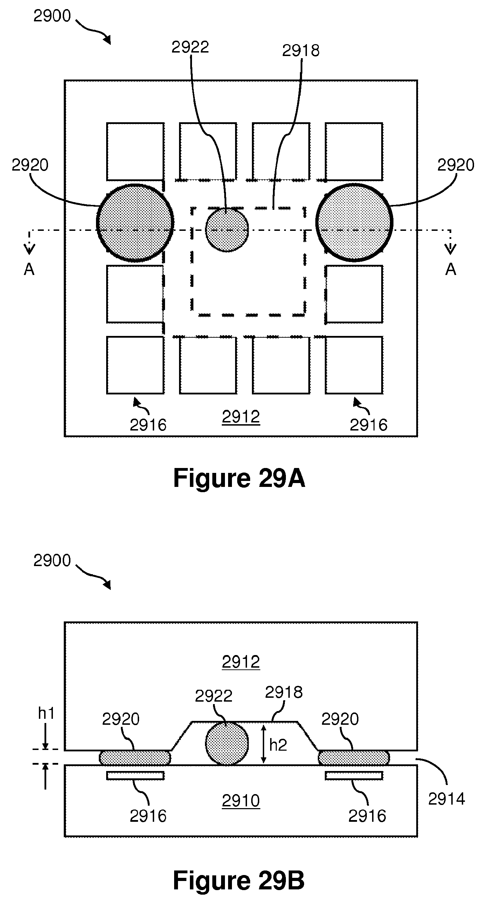

Figures 29A and 29B illustrate a top view and a cross-sectional view, respectively, of an example of a portion of a droplet actuator and show a process of dumping droplets to waste;

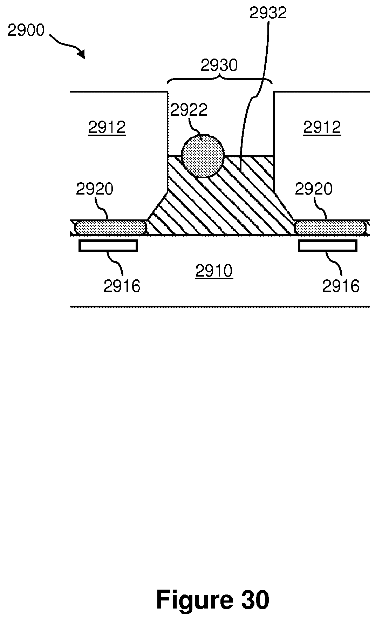

Figure 30 illustrates a cross-sectional view of another embodiment of the droplet actuator of Figures 29A and 29B;

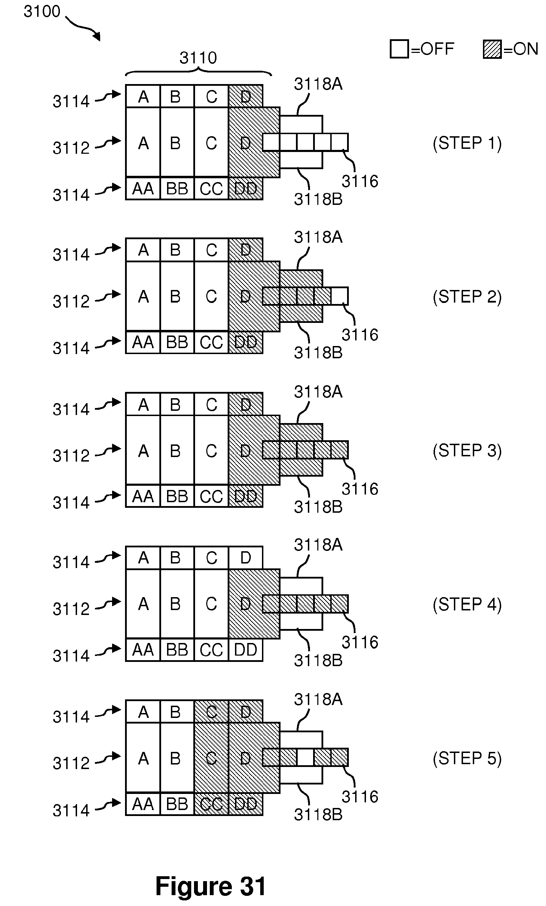

Figure 31 illustrates top views of a portion of an example of an electrode arrangement and a reservoir dispensing sequence for dispensing 2X droplets;

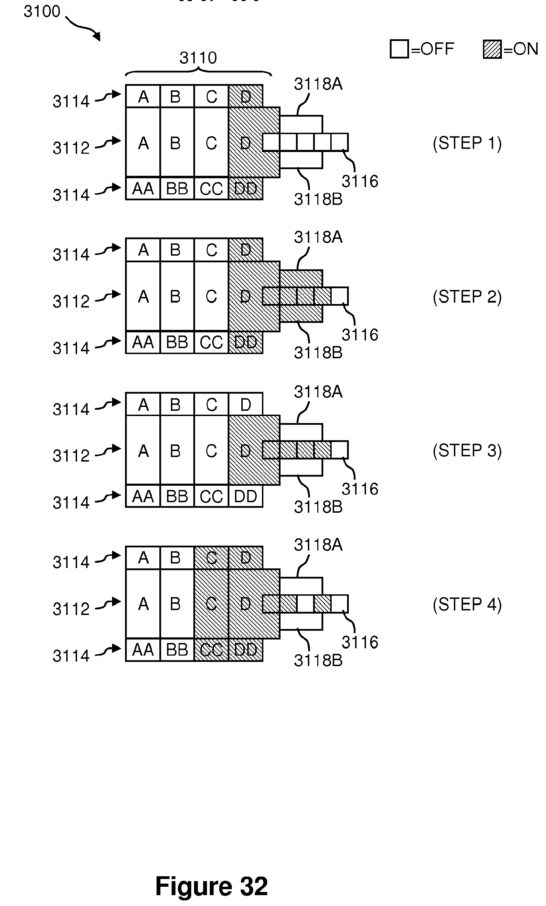

Figure 32 illustrates top views of the electrode arrangement of Figure 31 and a reservoir dispensing sequence for dispensing 1X droplets;

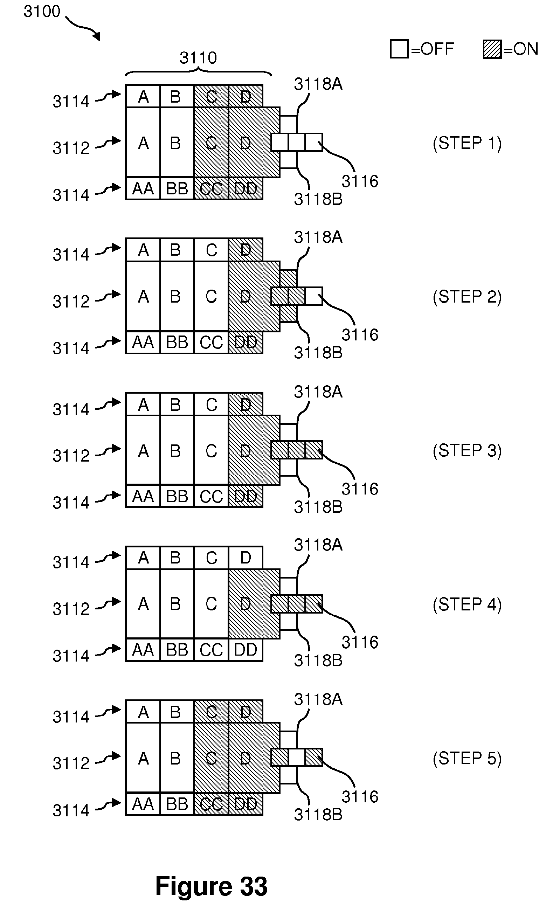

Figure 33 illustrates top views of another embodiment of the electrode arrangement of Figure 31 and another reservoir dispensing sequence for dispensing 1X droplets; and

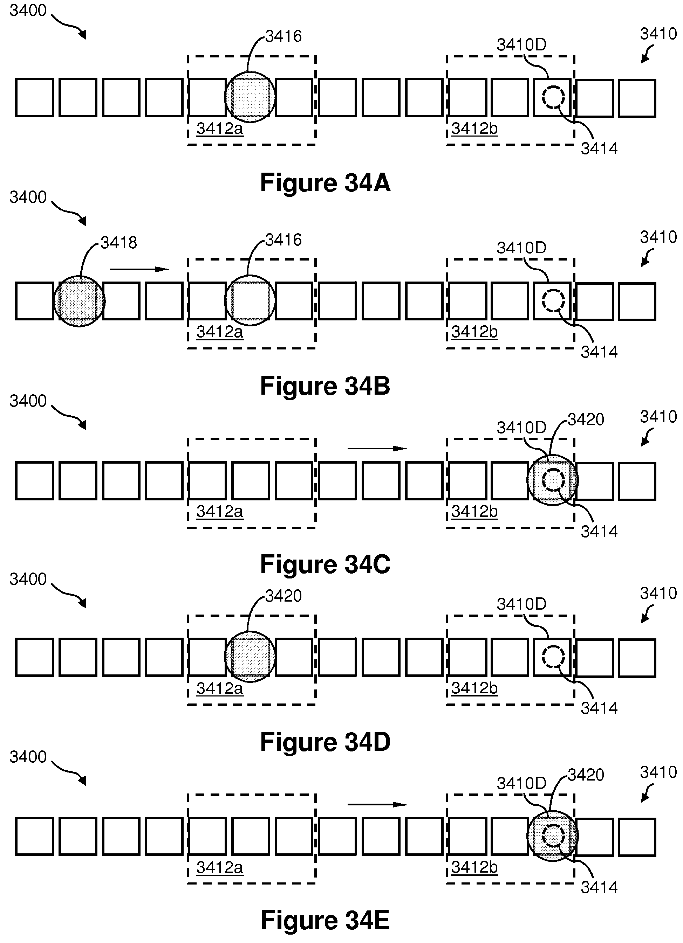

Figures 34A through 34E illustrate top views of an example of a portion of an electrode arrangement of a droplet actuator and show a process of integrating PCR amplification and HRM analysis for allele discrimination on a droplet actuator.

### 7 Detailed Description of the Invention

The invention provides methods for constructing nucleic acid libraries. The methods typically use sample and reagent droplets in immiscible fluids, such as oil. In one aspect, the methods of the invention use fragmented nucleic acid as an input sample, and end with an adapter-ligated nucleic acid library ready for next steps in a nucleic acid sequencing process, e.g., using a next-generation sequencing platform, such as platforms available or in development from F. Hoffmann-La Roche Ltd., Life Technologies Corp., Illumina, Inc., Helicos BioSciences Corp., and Pacific Biosciences of California, Inc.. Additional steps on either end of the process may also be included, such as size fractionation, nucleic acid fragmentation, reverse transcription, nucleic acid amplification, double stranded nuclease treatment, target enrichment, targeted sequence capture, size selection, and quantitation of nucleic acid output (e.g., by gel electrophoresis, qPCR or other methods). In certain embodiments, the chemical techniques of the invention may be executed using a droplet actuator device. Library construction parameters, such as yield of the overall process, yield of particular steps, time-to-result, reagent consumption, and bias, are vastly improved over the existing state of the art. The invention also provides novel devices, techniques and assay steps that have uses beyond nucleic acid library construction.

### 7.1 Library Construction

A typical library construction protocol of the invention includes several steps of enzymatic reactions. Some or all of the steps may be followed by nucleic acid purification. The steps may be performed using unit-sized droplets, e.g., 1X, 2X, 3X, 4X sized droplets. The steps may be conducted while droplets are floating or submersed in an immiscible or substantially immiscible filler fluid, or otherwise completely or partially surrounded by an immiscible or substantially immiscible filler fluid, such as droplets compressed between two substrates in a droplet actuator coated, partially filled, or substantially filled with an immiscible or substantially immiscible filler fluid. Thus, in the present specification, where any droplet operation is described, it should be understood that the droplet operation may be conducted while droplets are floating or submersed in an immiscible or substantially immiscible filler fluid, or otherwise completely or partially surrounded by an immiscible or substantially immiscible filler fluid, or compressed between two substrates in a droplet actuator coated, partially filled, or substantially filled with an immiscible or substantially immiscible filler fluid. Moreover, it should be understood that such filler fluid is preferably an oil-based filler fluid, such as those described herein. Some or all of the droplet operations of the methods of the invention may be executed on a droplet actuator.

The library preparation methods of the invention make use of fragmented nucleic acids. The fragmented nucleic acids may be produced using any nucleic acid fragmentation process, such as physical shearing processes; sonication; enzymatic processes, such as restriction endonucleases; and other chemical processes; as well as combinations of such processes. Fragment sizes may be random or non-random. Fragments may or may not be size fractionated. Fragmentation of nucleic acids may be accomplished on or off a droplet actuator. On a droplet actuator, a droplet including a non-fragmented sample may be merged using droplet operations with one or more droplets including fragmentation reagents to yield a droplet including fragmented nucleic acid. The fragmented nucleic acid may be removed from the droplet actuator for subsequent steps, or subjected to subsequent steps in a sample preparation process or subjected to subsequent steps on a droplet actuator. Examples of sonication set-ups that may be useful for facilitating fragmentation of nucleic acid samples on a droplet actuator are described in Srinivasan et al., U.S. Patent App. No. 61/364,645, entitled "Systems for and Methods of Promoting Cell Lysis in Droplet Actuators," filed on July 15, 2010, the entire disclosure of which is incorporated herein by reference.

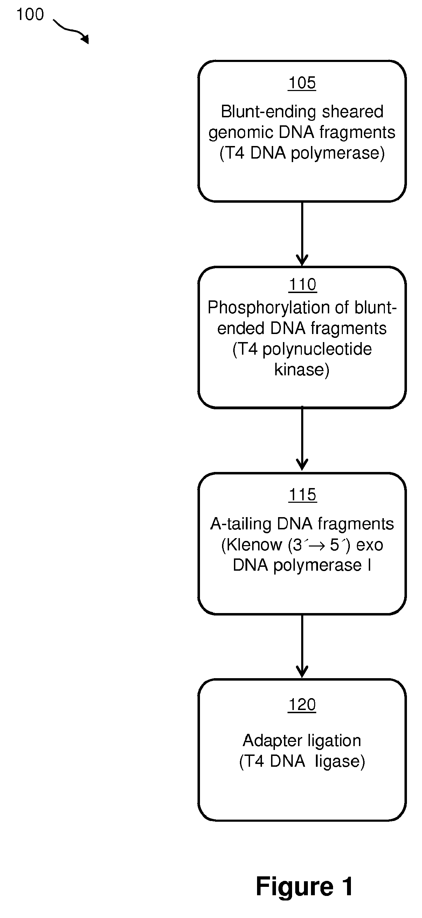

**Figure 1** is a flow diagram showing steps in an exemplary protocol 100 for construction of a nucleic acid library using fragmented nucleic acids. Protocol 100 may include, but is not limited to, the following steps:
In blunt ending step 105, nucleic acid fragments with 5'- and/or 3'- overhangs are blunt-ended using T4 DNA polymerase which has both a 3'→5' exonuclease activity and a 5'→3' polymerase activity. On a droplet actuator, this step may be accomplished by using droplet operations to dispense and combine a sample droplet and a droplet comprising blunt ending reagents. After blunt-ending, the nucleic acid may be purified (e.g., a bead-based washing protocol) to remove the enzyme and unincorporated dNTPs which may interfere with the subsequent steps. On the droplet actuator, this washing step may be accomplished by capturing the nucleic acid on beads and conducting a droplet-based washing protocol, as described herein. The nucleic acid may then be released from the beads. The blunt ending step 105 may be omitted where a fragmentation scheme is used that produces blunt ended fragments.

In phosphorylation step 110, T4 polynucleotide kinase may be used to attach a phosphate to the 5'-hydroxyl terminus of the blunt-ended nucleic acid. On a droplet actuator, this step may be accomplished by using droplet operations to dispense a droplet comprising phosphorylation reagents and merging that droplet with a droplet comprising the blunt ended nucleic acid produced by step 105. After blunt-ending, the nucleic acid may be purified (e.g., using a bead-based washing protocol) to remove the enzyme and excess reagent which may interfere with the subsequent steps. On the droplet actuator, this washing step may be accomplished by capturing the nucleic acid on beads and conducting a droplet-based washing protocol, as described herein. The nucleic acid may then be released from the beads. Note that blunt ending step 105 and phosphorylation step 110 may be readily combined. For example, in the ILLUMINA® Paired-end DNA Sample Prep Kit, and the NEBNEXT® DNA Sample Prep Reagent Set 1, and other commercially available kits, blunt ending and phosphorylation reagents are commonly combined. Nucleic acid purification for the combined steps may be accomplished using a bead-based merge-and-split droplet washing protocol as described for the individual steps. Note also that if the blunt ending step 105 and phosphorylation step 110 are separated, it is not necessary to do a wash step between them. A droplet comprising the blunt ending reagents may be dispensed and merged, using droplet operations, with the sample droplet; then, a droplet comprising the phosphorylation enzymes may be dispensed and merged, using droplet operations, with the droplet including the sample and blunt ending reagents. The nucleic acid in the droplet produced thereby may then be captured and purified as described herein.

In A-tailing step 115, Klenow (3'→5') exo-DNA polymerase I may be used to add A-tails on both ends of the phosphorylated, blunt-ended nucleic acid fragments. The reaction preferably occurs at about 37 °C. In the presence of dATP, Klenow (3'→5') exo-DNA polymerase I catalyzes non-template 3' additions. On the droplet actuator, this step may be accomplished by using droplet operations to dispense a droplet comprising A-tailing reagents and merging that droplet with a droplet comprising the phosphorylated nucleic acid produced by step 110. On the droplet actuator, this reaction may be accomplished at temperatures as low as room temperature or as high as 37 °C or higher in some cases. After the A-tailing reaction, Klenow DNA polymerase I may be inactivated, e.g., chemically inactivated by conducting droplet operations to merge the reaction droplet with a droplet comprising an inactivation reagent, or heat inactivated by incubating the reaction droplet at a temperature and duration selected to achieve the desired activation. The temperature is typically elevated above room temperature, e.g., from about 35 to about 75 °C for from about 20 to about 60 minutes. Alternatively, the nucleic acid may be purified and the enzyme removed using a bead-based washing protocol in which the nucleic acid may be captured on beads which are subjected to the washing protocol.

In adapter ligation step 120, T4 DNA ligase may be used to couple nucleic acid adapters to the A-tailed nucleic acid fragments. T4 DNA ligase catalyzes the formation of a phosphate bond between cohesive end termini that present a juxtaposed 5'-phosphate and 3'-hydroxyl termini in duplex nucleic acid. The reaction may, for example, occur at a temperature and for a duration selected to achieve the desired adapter coupling, e.g., at about 20 °C for about 30 minutes. On the droplet actuator, this step may be accomplished by using droplet operations to dispense a droplet comprising adapters and ligation reagents and merging that droplet with a droplet comprising the A-tailed nucleic acid produced by step 115. After ligation, the nucleic acid may be purified (e.g., using a bead-based washing protocol) to remove unincorporated adapters which will interfere with the subsequent PCR amplification steps. On the droplet actuator, this washing step may be accomplished by capturing the nucleic acid on beads and conducting a droplet-based washing protocol, as described herein. The nucleic acid may then be released from the beads. In certain embodiments, the ratio of adapter to nucleic acid is greater than about 10:1, or greater than about 15:1, or greater than about 20:1, or greater than about 25:1, or greater than about 30:1 molar or higher. In yet another embodiment the ratio of adapter to nucleic acids is from about 1:1 to about 10:1. The released nucleic acid may then be amplified using any nucleic acid amplification technique. The amplification may be conducted on the droplet actuator, e.g., using a flow-through thermal cycling process, or the nucleic acid may be removed from the droplet actuator for amplification in a separate device. However, in one aspect of the invention, the applicants have discovered that the processes of the invention provide a yield which is far greater than the typical library construction process. Consequently, in certain embodiments, the methods of the invention provide for sequencing the nucleic acid library following library construction without an intervening amplification step. In certain embodiments, the methods of the invention provide for sequencing the nucleic acid library following library construction with minimal amplification, e.g., 20 or fewer cycles, or 15 or fewer cycles, or 10 or fewer cycles, or 5 or fewer cycles, or just one or two cycles. Thus, in certain embodiments, the method of the invention includes removing a library droplet from the droplet actuator and loading the library onto a sequencing machine without an intervening amplification step or with minimal amplification, e.g., 20 or fewer cycles, or 15 or fewer cycles, or 10 or fewer cycles, or 5 or fewer cycles, or just one or two cycles. In certain embodiments, the methods of the invention provide for sequencing the nucleic acid library following library construction without an intervening enrichment amplification step.

In one embodiment, adapter ligation step 120 may be accomplished at a temperature which is below room temperature, e.g., from about 15 to about 24 °C, or from about 17 to about 23 °C, or from about 19 to about 21 °C, or at about 20 °C.

In another alternative embodiment, blunt ending step 105, phosphorylation step 110, and A-tailing step 115 are combined. On the droplet actuator, this embodiment involves using droplet operations to dispense a droplet including blunt ending, phosphorylation, and A-tailing reagents, and merging the dispensed droplet with a nucleic acid sample droplet, to accomplish blunt ending, phosphorylation, and A-tailing of the sample nucleic acid. The nucleic acid may be captured on beads and washed, e.g., as described elsewhere in this specification, in order to provide purified nucleic acid for a subsequent adapter ligation step.

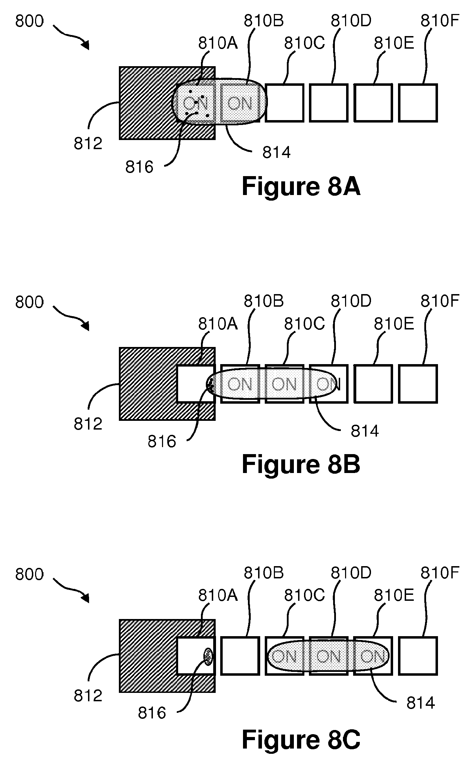

In another embodiment, a nucleic acid purification step is performed only after the adapter ligation step. In yet another embodiment, one or more capture and elution steps may be performed after any of the steps in the library construction process. For example, after blunt ending, phosphorylation, and/or A-tailing steps, the droplet may be combined with a droplet comprising nucleic acid capture beads. The beads may be restrained using a magnet, and excess liquid in the droplet surrounding the beads may be "snapped off" by transporting the droplet away from the beads. The beads will be left behind with a miniscule amount of liquid surrounding them. They may be picked up by transporting another droplet into contact with them, thus merging this miniscule amount of liquid with the liquid of the new droplet and suspending the beads in the newly merged droplet. Thus, for example, the new droplet may include reagents for eluting the captured nucleic acid from the beads. The beads may again be restrained using a magnet, and excess liquid in the droplet surrounding the beads may be "snapped off" by transporting the droplet away from the beads. This droplet will include the eluted nucleic acid, which may then be subjected to subsequent steps in the library preparation process. Beads may be restrained during the snapping off process and may thereafter be released and resuspended in a subsequent droplet by displacement of the magnet or switching off an electromagnet. Examples of suitable snapping off techniques are described elsewhere herein, e.g., see the description of Figures 8A, 8B, and 8C.

Any required temperature conditions in this and other protocols of the invention may be produced by heating or cooling a droplet operations surface, and/or a region of filler fluid in a droplet operations gap of a droplet actuator. Droplets may be transported using droplet operations into the heated or cooled region of the droplet operations surface or gap and retained there for the requisite period of time. Alternatively, an entire droplet actuator cartridge or the region of the cartridge containing the droplets can be heated or cooled for the requisite incubation period. Moreover, the droplets may be removed from the droplet actuator, e.g., by displacing the droplet into a fluid path that exits the droplet operations surface or gap, for heating or cooling outside the droplet operations gap or away from the droplet operations surface. The droplet may thereafter be returned to the gap or surface following cooling for conducting subsequent steps.

An example of an on-bench protocol for construction of a paired-end library is shown in Table 1. In this example, reaction kits for each enzymatic step, i.e., blunt-ending, A-tailing, and adapter ligation are used (E1210S, M0212S and M2200S, respectively, New England Biolabs). Reagents may be diluted as needed to provide a final 1X concentration for use in the assay. The blunting, A-tailing, and adapter ligation mixes may be modified to include a surfactant, such as a polysorbate surfactant, such as 0.01 - 0.1% Tween-20 (also known as polysorbate 20), or other surfactants at concentrations described herein.

| **Table 1.** Bench protocol for the 3-step paired-end library construction | | | | | |
|---|---|---|---|---|---|
| Blunt-ending | | A-tailing | | Adapter ligation | |
| 10X Blunting buffer | 2.5 | 10X buffer | 5 µL | 2X buffer | 50 µL |
| 1 mM dNTP | µL | 1 mM dATP | 1.65 | 50 µM each P1 & | 3 µL |
| Blunting enzyme mix | 2.5 | Klenow (3'-5' exo) | µL | P2 adapter mix | |
| Water | µL | mix Water | 1 µL | DNA quick ligase | 5 µL |
| gDNA | 1 µL | Blunt-ended DNA | 2.35 | mix | |
| | 9 µL | | µL | Water | 2 µL |
| | 10 µL | | 40 µL | A-tailed DNA | 40 µL |
| Incubated at 25 °C for 30 mien Cleaned up with CHARGESWITCH® beads Eluted into 40 µL elution buffer | | Incubated at about 37 °C for 30 min Cleaned up with CHARGESWITCH® beads Eluted into 40 µL elution buffer | | Incubated at 25 °C for 5 min Cleaned up with CHARGESWITCH® beads Eluted into 40 µL elution buffer | |

Another example of a nucleic acid fragment library construction protocol is a high-density in vitro transposition protocol. In the transposition protocol, a hyperactive derivative of the Tn5 transposase may be used to catalyze integration of synthetic oligonucleotides (i.e., engineered transposons) into target DNA at a high density. In this example, a transposase, such as Nextera's Transposome™ technology, may be used to generate random dsDNA breaks. The Transposome™ complex includes free transposon ends and a transposase. When this complex is incubated with dsDNA, the DNA is fragmented and the transferred strand of the transposon end oligonucleotide is covalently attached to the 5' end of the DNA fragment. In some platform-specific applications (e.g., Illumina sequencing platform), the transposon ends may be appended with sequencing primer sites. By varying buffer and reaction conditions (e.g., concentration of Transposome™ complexes), the size distribution of the fragmented and tagged DNA library may be controlled. Nextera technology may be used to generate di-tagged libraries, with optional bar coding, compatible with sequencing platforms, such as Roche/454 or Illumina/Solexa sequencing platforms.

A digital microfluidic protocol for transposase-catalyzed library construction may include, but is not limited to, the following steps: Nucleic acid may be fragmented and tagged using a transposase enzyme complex that includes transposase and free transposon ends (e.g., Nextera's Transposome™ complex). In some platform-specific applications (e.g., Illumina sequencing platform), the transposon ends may be appended with sequencing primer sites. Transposon integration and strand transfer occur via a staggered, dsDNA break within the target nucleic acid. The target nucleic acid may be tagged at the 5' end with the transposon sequence. The reaction preferably occurs at 55 °C. On a droplet actuator, this step may be accomplished by using droplet operations to dispense and combine a sample droplet and a droplet comprising transposase enzyme reagents and reaction buffer. The transposase enzyme reagents may be selected for platform-specific applications (e.g., Nextera™ Enzyme Mix for Illunina-compatible libraries; Nextera™ Enzyme Mix for Roche 454-compatible libraries). After fragmentation and tagging, the nucleic acid may be purified (e.g., a bead-based washing protocol) to remove the enzyme and reagents which may interfere with the subsequent steps. On the droplet actuator, this washing step may be accomplished by capturing the nucleic acid on beads and conducting a droplet-based washing protocol, as described herein. The nucleic acid may then be released from the beads.

Suppression PCR with a four-primer reaction may be used to add platform-specific oligonucleotide adapters and optional bar codes to the fragmented and tagged nucleic acid. The resulting di-tagged library may be enriched for fragments containing both tags. In one example, suppression PCR with a four-primer reaction may be used to add for Roche 454-compatible libraries. Optional bar coding may be added between the upstream PCR adapter and the transposon. In another example, suppression PCR with a four-primer reaction may be used to add Illumina-compatible adapter sequences (i.e., bridge PCR-compatible adapters; bPCR). Optional bar coding may be added between the downstream PCR adapter and the appended transposon. On a droplet actuator, this step may be accomplished by using droplet operations to dispense and combine a sample droplet and a droplet comprising PCR reagents and a platform-specific primer cocktail (e.g., Nextera's Illunina-compatible primer cocktail or Nextera's Roche 454-compatible primer cocktail). The amplification may be conducted on the droplet actuator by thermocycling the reaction droplet between temperature control zones for a limited number of cycles (e.g., about 5 to about13 cycles). In one example, the thermocycling protocol may include incubations of 3 min at 72 °C and 30 sec at 95 °C, followed by 13 cycles of 10 sec at 95 °C, 30 sec at 72 °C, and 3 min at 72 °C. Alternatively, the nucleic acid may be removed from the droplet actuator for amplification in a separate device.

The di-tagged library may be purified (e.g., bead-based washing protocol) to remove unincorporated adapters and bar codes which may interfere with the subsequent PCR amplification steps. On the droplet actuator, this washing step may be accomplished by capturing the nucleic acid on beads and conducting a droplet-based washing protocol, as described herein. The nucleic acid may then be released from the beads. The enriched, di-tagged library may be amplified, for example, by emulsion PCR (emPCR) on the Roche 454 platform; bridge PCR (bPCR) on the Illunina platform; on the droplet actuator; or using other amplification methods.

The amplified library may be subsequently sequenced using the appropriate primers. In another embodiment, the di-tagged library may be size selected (e.g., >300 bp size) prior to amplification and sequencing. In one example, the di-tagged library may be sized on the droplet actuator by gel electrophoresis. In another example, the di-tagged library may be removed from the droplet actuator for size separation in a separate device such as a nicrofluidic chip-based automated size selection platform from Caliper.

Standard sequencing libraries for the Illumina sequencing platform have been generated without the use of PCR amplification (PCR-free) in order to reduce associated biases. A similar approach may be used for transposase-based libraries. In one example, the flowcell bridge PCR primer (bPCR) sequences may be included in the adapters that are added during the transposition reaction. After transposition, a nick translation reaction may be performed resulting in Illumina-ready libraries. The PCR-free, transposase-based library construction protocol substantially reduces the time required for converting nucleic acid to a sequencing-ready fragment library.

A digital nicrofluidic protocol for PCR-free, transposase-catalyzed library construction may include, but is not limited to, the following steps: Nucleic acid may be fragmented and tagged using a transposase enzyme complex that includes transposase and free transposon ends (e.g., Nextera's Transposome™ complex). For the Illumina sequencing platform, the transposome adapter sequences may include flowcell bPCR sequences and appended sequencing primer sites. Transposon integration and strand transfer occur via a staggered, dsDNA break within the target nucleic acid. The target nucleic acid may be tagged at the 5' end with the transposon sequence. The reaction preferably occurs at 55 °C. On a droplet actuator, this step may be accomplished by using droplet operations to dispense and combine a sample droplet and a droplet comprising transposase enzyme reagent and reaction buffer. DNA polymerase may be used in a nick translation reaction to repair the single-stranded gap generated in the fragmentation and tagging reaction. On the droplet actuator, this step may be accomplished by using droplet operations to combine the sample droplet and a droplet comprising DNA polymerase and reaction buffer (e.g., FailSafe PCR Master Mix and DNA polymerase available from Epicentre). After nick translation, the DNA may be purified (e.g., a bead-based washing protocol) to remove the enzyme and reagents which may interfere with the subsequent steps. On the droplet actuator, this washing step may be accomplished by capturing the nucleic acid on beads and conducting a droplet-based washing protocol, as described herein. The nucleic acid may then be released from the beads. The di-tagged library may be ready for amplification bridge PCR (bPCR) on the Illunina platform. The amplified library may be subsequently sequenced using the appropriate primers.

The digital nicrofluidic library construction protocol was evaluated using a 278 bp bacterial DNA fragment from methicillin-resistant *Staphylococcus aureus* (MRSA). DNA end repair (blunt-ending), dA-tailing of DNA fragments, and adapter ligation were performed using NEBnext DNA Sample Prep Master Mix Set 1 available from New England BioLabs. The set includes NEBnext End Repair, dA-Tailing and Quick Ligation modules. DNA samples (1, 10, 30, 100, 300, or 1000 ng of 278 bp MRSA DNA) and reagents were prepared on-bench and subsequently loaded into fluid dispensing reservoirs of a droplet actuator. The experiment was performed 4 times and run on 4 separate droplet actuators.

A working solution of NEBnext End Repair (2X enzyme and buffer concentration) was prepared by combining End Repair reaction buffer (3 µL), End Repair enzyme mix (1.5 µL), 0.5% Tween 20 (1.5 µL), and sterile water (9 µL) in a final volume of 15 µL. A working solution of NEBnext dA-Tailing (2X enzyme and buffer concentration) was prepared by combining dA-Tailing reaction buffer (3 µL), Klenow fragment (exo-) (1.8 µL), 0.5% Tween 20 (1.5 µL), and sterile water (8.7 µL) in a final volume of 15 µL. A working solution of NEBnext Quick Ligation (3X enzyme and buffer concentration) was prepared by combining Quick Ligation reaction buffer (9 µL), Quick T4 DNA ligase (4.5 µL), and 0.5% Tween 20 (1.5 µL) in a final volume of 15 µL. A magnetically responsive bead solution was prepared by combining 300 µL of Agencourt AMPure XP beads and 3 µL 1% Tween 20. Adapters were prepared at a 10:1 molar ratio of adapter to DNA at a final concentration of 0.1 % Tween 20. The concentration of the adapters varied depending on the DNA concentration.

DNA samples were prepared by combining 1, 10, 30, 100, 300, or 1000 ng of 278 bp MRSA DNA (up to 23 µL), Agencourt AMPure XP beads (1.2 µL), bead binding buffer (25 µL), 5% Tween 20 (1 µL), and sterile water to a final volume of 50 µL. The beads and DNA samples were incubated with shaking at room temperature for 10 minutes.

The DNA samples (1, 10, 30, 100, 300, or 1000 ng of 278 bp MRSA DNA) and prepared reagents were loaded into reservoirs of a droplet actuator. Prior to dispensing and processing, each DNA sample was concentrated on-actuator using a single step bead concentration protocol. The magnetically responsive beads with bound DNA thereon were immobilized using a magnet positioned below the sample dispensing reservoir electrodes. The supernatant liquid (about 50 µL) was split off using droplet operations and transported away from the immobilized beads that were retained by the magnetic field. The DNA was subsequently eluted from the magnetically responsive beads in a 1X DNA sample droplet.

The digital nicrofluidic protocol used to evaluate the library output (yield) included the following steps conducted using electrowetting-mediated droplet operations in a droplet operations gap of a droplet actuator: A 1X DNA sample droplet was combined using droplet operations with a 1X NEBnext End Repair droplet to yield a 2X reaction droplet. After 30 minute incubation at room temperature, the 2X reaction droplet was combined using droplet operations with a 4X magnetically responsive bead containing droplet to yield a 6X DNA sample/bead droplet. After 10 minute incubation at room temperature, the beads were immobilized using a magnet and a 6X supernatant droplet is split off to yield a 0X sample/bead droplet. The supernatant droplet was transported to waste. The end-repaired DNA was eluted from the beads in a 1X sample droplet. The 1X end-repaired DNA sample droplet was combined using droplet operations with a 1X NEBnext dA-Tailing droplet to yield a 2X reaction droplet. After 30 minute incubation at about 37 °C, the 2X reaction droplet was combined using droplet operations with a 4X magnetically responsive bead containing droplet to yield a 6X DNA sample/bead droplet. After 10 minute incubation at room temperature, the beads were immobilized using a magnet and a 6X supernatant droplet is split off to yield a 0X sample/bead droplet. The supernatant droplet was transported to waste. The A-tailed DNA was eluted from the beads in a 1X sample droplet. The 1X A-tailed DNA sample droplet was combined using droplet operations with a 1X NEBnext Quick Ligation droplet and a 1X adapter droplet to yield a 3X reaction droplet. After 30 minute incubation at room temperature, the 3X reaction droplet was combined using droplet operations with a 2X magnetically responsive bead containing droplet to yield a 5X DNA sample/bead droplet. After 10 minute incubation at room temperature, the beads were immobilized using a magnet and a 5X supernatant droplet is split off to yield a 0X sample/bead droplet. The supernatant droplet was transported to waste. The beads were washed 3 times with a 2X wash buffer droplet using a droplet-based bead washing protocol. The adapter ligated DNA was eluted from the beads in a 1X sample droplet. The 1X adapter ligated DNA sample droplet was transported using droplet operations to a sample collection reservoir on the droplet actuator and removed from the droplet actuator for determination of library construction yield.

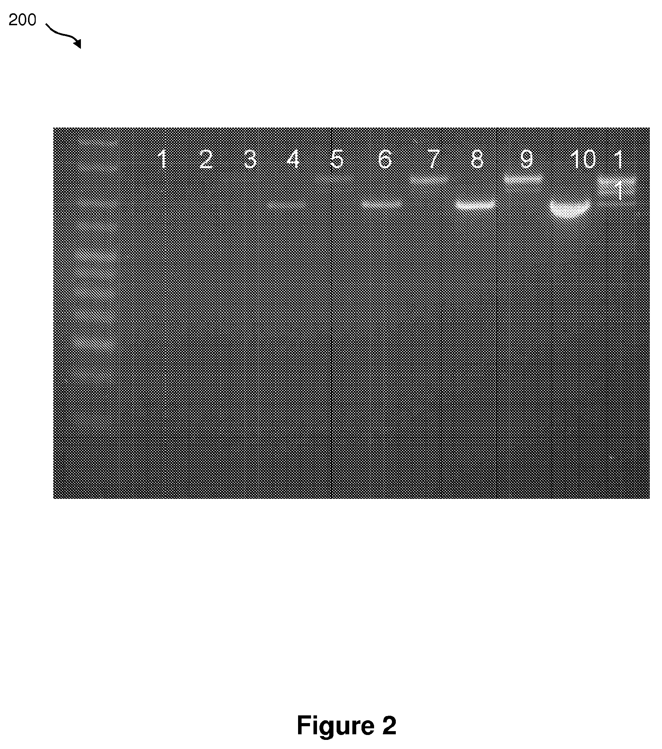

The results of the calculated yield from an agarose gel analysis of the on-actuator library construction protocol are shown in Table 2. For the gel analysis, 9 µL of 1, 10, 30, and 100 ng input samples; 5 µL of 300 ng input sample; and 1.5 µL of 1000 ng input sample were adjusted with water to 10 µL prior to loading samples on the gel. MRSA DNA (278 bp fragment) was loaded onto the gel at 0.9, 9, 27, 90, and 150 ng and used as a standard. **Figure 2** shows an exemplary photograph of the agarose gel used to calculate the library output data shown in Table 2. The library samples and MRSA standard DNA were loaded onto the agarose gel in the following order (from left to right): 1 ng input sample, 0.9 ng MRSA standard, 10 ng input sample, 9 ng MRSA standard, 30 ng input sample, 27 ng MRSA standard, 100 ng input sample, 90 ng MRSA standard, 300 ng input sample, 150 ng MRSA standard, and 1000 ng input sample. Gel analysis was performed using Image J software to determine library output.

| **Table 2.** Calculated yield from gel analysis | |
|---|---|
| Input MRSA (ng) | Library output (ng) |
| 1000 | 643.6 |
| 300 | 134.6 |
| 100 | 48.1 |
| 30 | 21.4 |
| 10 | 5.6 |

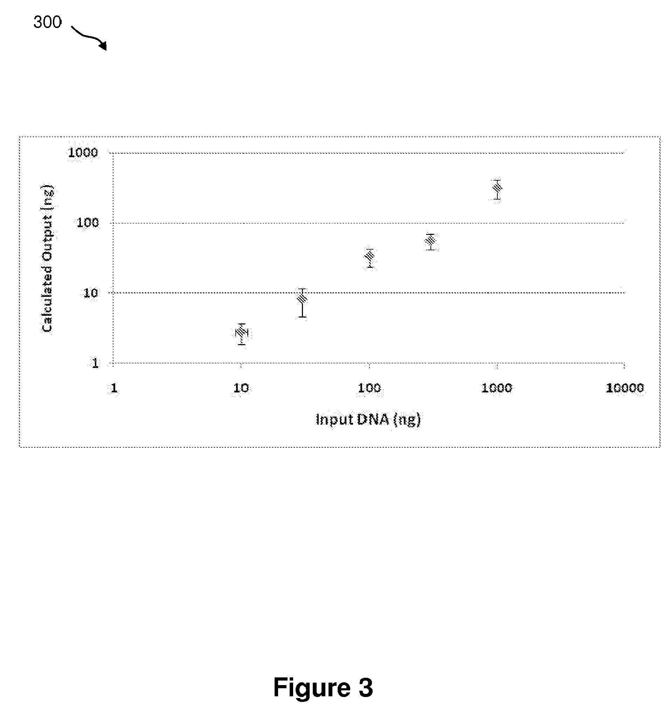

The experiment was repeated an additional 7 times on 7 separate droplet actuators. **Figure 3** shows a plot of the calculated yield from agarose gel analysis of 7 additional runs of the on-actuator library construction protocol using 10, 30, 100, 300, or 1000 ng of 278 bp MRSA DNA. The data in Figure 3 is summarized in Table 3.

| **Table 3.** Calculated yield from additional 7 library amplifications | | | |
|---|---|---|---|
| Input (ng) | Average Output (ng) | Std Dev | Yield (%) |
| 1000 | 320 | 97 | 32.0 |
| 300 | 56 | 14 | 18.7 |
| 100 | 34 | 10 | 33.6 |
| 30 | 8 | 4 | 27.4 |
| 10 | 3 | 1 | 27.6 |

Droplet manipulation protocols, reaction biochemistry, and other system parameters may be further selected to improve the performance of the automated digital nicrofluidic library construction protocol. Selection of biochemical reaction conditions may, for example, be facilitated by assessing the performance of each reaction step.

In one embodiment, the quantitative recovery or yield of DNA after each step of the protocol may be measured on-actuator using either incorporated radioactive phosphate or a fluorescent label bound to the incorporated nucleotide. For example, in the blunt-ending step (referring to the protocol of Figure 1), T4 polynucleotide kinase attaches a phosphate to the 5'-hydroxyl terminus of the blunt-ended DNA. By using radio-labeled ATP (γ-³²P ATP) in the kinase reaction buffer, the terminal phosphate attached to the blunt-ended DNA will be radio-labeled and may be readily monitored. Similarly, the γ-phosphate in the dATP may be radio-labeled for monitoring the A-tailing step. All the nucleotides in the adapter sequence may be radio-labeled in their α-phosphate and the adapter ligation step may be monitored quantitatively with high sensitivity.

In another embodiment, the adapter-ligated sequence quality (e.g., no degradation), bias (e.g., insert size, GC content), and reproducibility may be evaluated. In this example, library construction may be performed using DNA amplicons of different sizes and GC content. The quality of the adapter-ligated sequence may be assessed by collecting the processed fragments and evaluating the DNA sequences using a commercial sequencer. The library construction process may be evaluated for reproducibility by performing the protocol on the same DNA sample multiple times. Variability among different reservoirs on the droplet actuator may be evaluated by loading the same DNA sample into different on-actuator reservoirs.

Digital nicrofluidic operational parameters, such as incubation time, number of wash cycles, droplet transport speed, and reagent loading volumes, may be selected by one of skill in the art in view of the instant disclosure. Because the mixing length scales and the volume-to-surface ratio are smaller in a digital nicrofluidic droplet system compared to a bench system, the incubation time in droplet system may be substantially reduced. A substantially reduced incubation time for enzymatic reactions combined with an automated protocol (i.e., less hands-on-time) may reduce the overall time of the library construction process.

### 7.2 Nucleic Acid Purification

Protocols for library construction on a droplet actuator may include bead-based nucleic acid purification steps. The beads may be magnetically responsive or not substantially magnetically responsive. In one embodiment, any of the enzymatic steps described herein may be followed with a bead-based nucleic acid purification step. Nucleic acid purification and clean-up steps are often the yield-limiting steps in a library construction protocol.

In one example charge switch beads, such as CHARGESWITCH® PCR cleanup beads (available from Invitrogen by Life Technologies), may be used. CHARGESWITCH® PCR cleanup beads are paramagnetically responsive beads that can capture nucleic acid typically from 90 bp to 4 kbp at pH 5, and release the captured nucleic acid at pH > 8. In one example, the use of CHARGESWITCH® beads in the clean-up steps of a digital nicrofluidic library construction protocol may be selected by adjusting the buffering capacity and pH of the elution buffer used to elute bound DNA from the beads. Tables 4 and 5 show the effects of elution buffer composition and number of washes on percent nucleic acid recovery. pH of buffers was adjusted using 12M HCl.

| **Table 4.** Elution conditions and % recovery in the paired-end library construction protocol (on-actuator vs. on-bench recovery) | | | | | | |
|---|---|---|---|---|---|---|
| Elution Condition | Dilution | Amount input to PCR if 100% recovery | Actual amount recovered from actuator | % Recovered on-actuator | Actual amount recovered from bench re-elution | % Recovered on-bench |
| 10 mM Tris pH 8.5 55 C 2 washes | 100X | 240 pg | 618 fg | 0.3% | 300 pg | 125% |
| | 10,000X | 2.4 pg | 10 fg | 0.4% | 3.8 pg | 158% |
| | 100,000X | 240 fg | 12 ag | <0.1% | N/A | N/A |
| 100 mM Tris pH 9.5 55 C 2 washes | 100X | 240 pg | 1900 fg | 0.8% | 370 pg | 154% |
| | 10,000X | 2.4 pg | 40 fg | 1.7% | 4.1 pg | 171% |
| | 100,000X | 240 fg | N/A | N/A | N/A | N/A |
| 100 mM Tris pH 9.5 55 C 6 washes | 100X | 240 pg | N/A | N/A | 480 fg | 0.2% |
| | 10,000X | 2.4 pg | 2.9 pg | 120% | 5.6 fg | 0.2% |
| | 100,000X | 240 fg | 369 fg | 153% | N/A | N/A |
| 10 mM Tris pH 9.5 55 C 6 washes | 100X | 240 pg | 1200 fg | 0.5% | N/A | N/A |
| | 10,000X | 2.4 pg | 16 fg | 0.7% | 1.07 pg | 45% |
| | 100,000X | 240 fg | 13 ag | <0.1% | N/A | N/A |
| 50 mM Tris pH 9.0 55 C 6 washes | 100X | 240 pg | N/A | N/A | 15 pg | 6.3% |
| | 10,000X | 2.4 pg | 6 pg | 250% | 64 fg | 2.7% |
| | 100,000X | 240 fg | 510 fg | 212% | N/A | N/A |

| **Table 5.** Optimization of elution conditions in the paired-end library construction protocol | | | | | | |
|---|---|---|---|---|---|---|
| Elution Condition | Dilution | Amount input to PCR if 100% recovery | Actual amount recovered from actuator | % Recovered on-actuator | Calculated amount | % of 48 ng |
| 10 mM Tris pH 8.5 55 C 2 washes | 100X | 240 pg | 618 fg | 0.3% | 30.4 ng | 63% |
| | 10,000X | 2.4 pg | 10 fg | 0.4% | 38.2 ng | 80% |
| | 100,000X | 240 fg | 12 ag | <0.1% | N/A | N/A |
| 100 mM Tris pH 9.5 55 C 2 washes | 100X | 240 pg | 1900 fg | 0.8% | 37 ng | 77% |
| | 10,000X | 2.4 pg | 40 fg | 1.7% | 41.3 ng | 86% |
| | 100,000X | 240 fg | N/A | N/A | N/A | N/A |
| 100 mM Tris pH 9.5 55 C 6 washes | 100X | 240 pg | N/A | N/A | 48 pg | 0.1% |
| | 10,000X | 2.4 pg | 2.9 pg | 120% | 56 pg | 0.1% |
| | 100,000X | 240 fg | 369 fg | 153% | N/A | N/A |
| 10 mM Tris pH 9.5 55 C 6 washes | 100X | 240 pg | 1200 fg | 0.5% | N/A | N/A |
| | 10,000X | 2.4 pg | 16 fg | 0.7% | 107 ng | 223% |
| | 100,000X | 240 fg | 13 ag | <0.1% | N/A | N/A |
| 50 mM Tris pH 9.0 55 C 6 washes | 100X | 240 pg | N/A | N/A | 1.5 ng | 3.1% |
| | 10,000X | 2.4 pg | 6 pg | 250% | 64 fg | 1.3% |
| | 100,000X | 240 fg | 510 fg | 212% | N/A | N/A |

The digital Microfluidic protocol used to evaluate the elution buffer chemistry and number of washes included the following steps conducted using electrowetting-mediated droplet operations in a droplet operations gap of a droplet actuator: A ∼350 nL droplet containing 48 ng of a 278 bp MRSA dsDNA (138 ng/µL) was combined using droplet operations with a ∼350 nL droplet that contains CHARGESWITCH® beads (25 mg/mL) in binding buffer (pH 5) to yield a ∼700 nL droplet. After 1 minute incubation at room temperature, the ∼700 nL droplet was combined with a ∼700 nL wash buffer droplet, beads were immobilized using a magnet, and the droplet split to yield a bead-containing droplet and a supernatant droplet. After multiple wash cycles (2 or 6 cycles) were performed, the CHARGESWITCH® beads were resuspended in a ∼350 nL wash buffer droplet. The bead containing droplet was then combined with a ∼350 nL elution buffer droplet to yield a ∼700 nL elution droplet. After a 2-minute incubation, the CHARGESWITCH® beads were immobilized within the magnetic field of a permanent magnet and the ∼700 nL elution droplet was transported away from the beads into an on-actuator reservoir. The ∼700 nL elution droplet was recovered from the reservoir and adjusted to 10 µL volume with water. If the recovery of DNA was 100%, then there should be 4.8 pg of DNA input to the qPCR assay. The CHARGESWITCH® beads were recovered from the droplet actuator and another elution using 20 µL elution buffer was performed on the bench to elute any DNA not eluted on the droplet actuator. A quantitative PCR (qPCR) assay was performed to estimate the recovery of DNA. Samples were diluted 100X, 10,000X and 100,000X. Five µL of each sample was analyzed in a 50 µL qPCR assay. Samples were analyzed against a standard curve for the 278 MRSA amplicons. If the efficiency of DNA recovery was 100%, then input DNA into the qPCR assay should be 240 pg, 2.4 pg and 240 fg (100X, 10,000X and 100,000X dilutions, respectively).

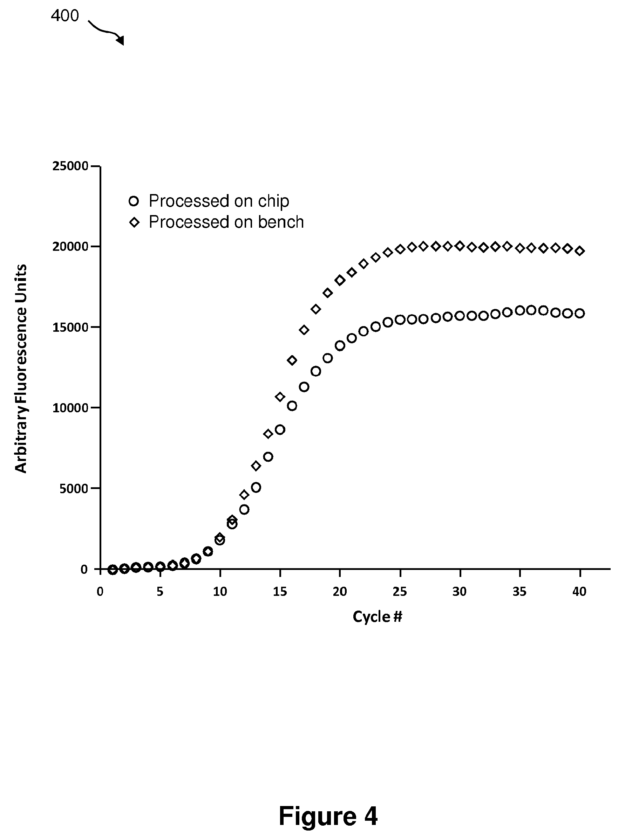

**Figure 4** shows a plot 400 of a comparison of the on-bench and on-droplet actuator implementation of the elution and nucleic acid clean-up steps. The elution conditions were used in a full paired-end library construction protocol performed on-bench and on the droplet actuator. The data show that on-bench and on-droplet actuator elution is comparable.

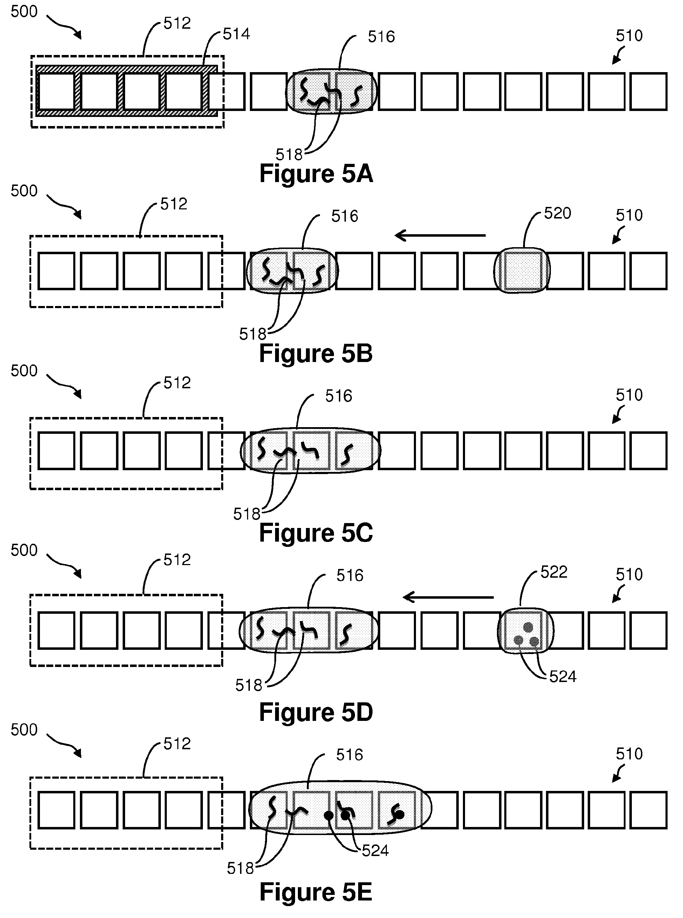

**Figures 5A through 5M** illustrate top views of an example of a portion of an electrode arrangement 500 of a droplet actuator and show a process of preparing nucleic acid for construction of a library. The method of Figures 5A through 5M is an example of a library construction protocol in which nucleic acid is immobilized on magnetically responsive beads, and a movable magnet and a series of merge and split operations are used to purify the nucleic acid between each step in the library construction protocol.

Electrode arrangement 500 may include an arrangement of droplet operations electrodes 510 (e.g., electrowetting electrodes). Droplet operations are conducted by droplet operations electrodes 510 on a droplet operations surface or in a droplet operations gap. A bead immobilization zone 512 may be associated with electrode arrangement 500. A movable magnet 514 may be aligned with and moved into and out of bead immobilization zone 512. Magnet 514 may be used for retaining magnetically responsive beads during droplet operations. In particular, magnet 514 may arranged such that at least one droplet operations electrode 510 and, optionally, other electrodes, is within the magnetic field. Magnet 514 may, for example be a permanent magnet or an electromagnet.

Sample droplet 516 may be transported using droplet operations along droplet operations electrodes 510. Sample droplet 516 may, for example, be a 2X droplet, meaning that its footprint in the droplet operations gap is approximately 2 times the area of one droplet operations electrode 510. Sample droplet 516 may contain nucleic acid 518 to be processed for construction of a nucleic acid library. In one embodiment, a nucleic acid sample may be sheared, and the sheared nucleic acid may be loaded into the droplet operations gap of a droplet actuator. Nucleic acids may, for example, be randomly fragmented by hydrodynamic shear or mechanical forces or fragmented by enzymatic digestion. In another embodiment, a nucleic acid sample, such as nucleic acid, may be randomly fragmented on a droplet actuator. In one example, a low frequency current may be used to agitate a sample droplet such that nucleic acid within the droplet is sheared. In another example, a droplet may be alternately expanded and contracted to create fluidic patterns within the droplet that is sufficient to shear nucleic acid. In another example, application of a low frequency current and alternating expansion and contraction of a droplet may be used to shear nucleic acid. On-actuator shearing of nucleic acid may be controlled to give fragments of a predictable size range. Sheared nucleic acid 518 may be size selected prior to processing for construction of a nucleic acid fragment library. In one example, sheared nucleic acid 518 may be separated by gel electrophoresis. An example of a process of preparing sheared nucleic acid for construction of a library on a droplet actuator may include, but is not limited to, the following steps.

Figure 5A shows sample droplet 516 that is positioned on electrode path 510 away from bead immobilization zone 512 and magnet 514. Magnet 514 may, for example, be situated on an instrument on which the droplet actuator is mounted, mounted on the droplet actuator itself, situated in the droplet operations gap, or in any other position which permits inmiobilization of beads in the droplet during the execution of a droplet-based bead washing protocol as described herein. Figures 5B and 5C show an incubation process in which reagent droplet 520 is merged using droplet operations with sample droplet 516. As illustrated, reagent droplet 520 is a 1X droplet (e.g., about 250 to about 500 nL), meaning that its footprint is approximately the area of one droplet operations electrode 510. In Figure 5C, merged sample droplet 516 is now a 3X droplet, meaning that its footprint is approximately 3 times the area of one droplet operations electrode 510. Reagent droplet 520 may include enzymes, e.g., blunt-ending reagents. Merged sample droplet 516 is incubated at a requisite temperature and for a requisite time to achieve the desired reaction, e.g., a temperature and time selected for facilitating blunt-ending of the nucleic acid fragments.

Figures 5D and 5E illustrate capture of the nucleic acids. A 1X reagent droplet 522 that includes magnetically responsive beads 524 selected to capture the nucleic acids or a certain subset of the nucleic acids is merged using droplet operations controlled by electrodes 514 with 3X merged sample droplet 516. Merged sample droplet 516 is now a 4X sample droplet, meaning that its footprint is approximately 4 times the area of one droplet operations electrode 510. Merged sample droplet 516 is incubated at room temperature for a period of time that is sufficient the desired nucleic acid fragments to bind to magnetically responsive beads 524.

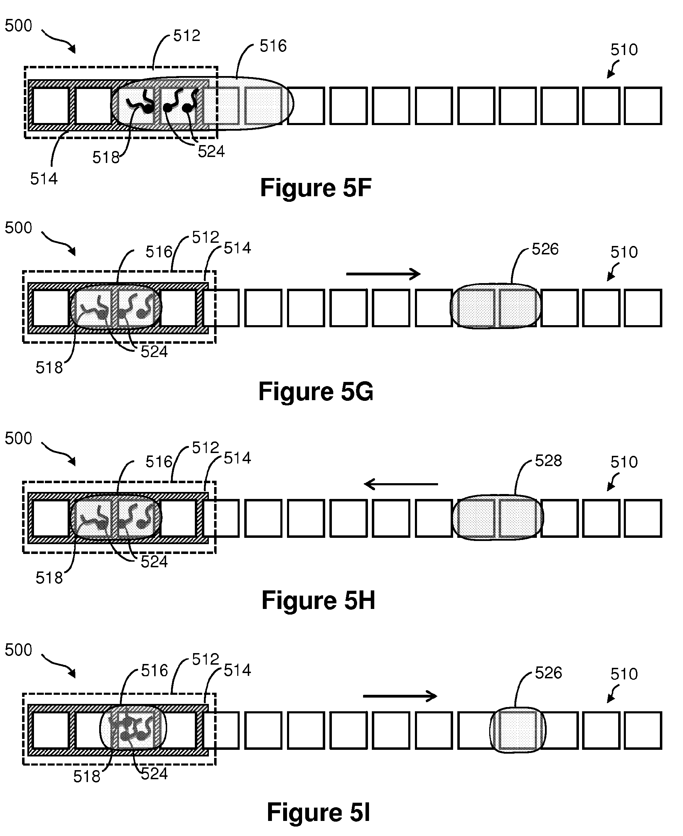

Figures 5F, 5G and 5H show a bead washing process, in which 4X merged sample droplet 516, which has magnetically responsive beads 524 and bound nucleic acid 518 therein, is transported using droplet operations along electrode path 510 into bead immobilization zone 512. Magnet 514 is moved into position within bead immobilization zone 512 such that 4X merged sample droplet 516 is within the magnetic field of magnet 514. Magnetically responsive beads 524 are immobilized or otherwise restrained or substantially immobilized by the magnetic field of magnet 514. A 2X supernatant droplet 526 is split off using droplet operations to form a 2X merged sample droplet, which is retained at a droplet operations electrode 510 that is within the magnetic field of magnet 514. This can be accomplished by activating 5 underlying electrodes to elongate the droplet across the 5 electrodes, and then deactivating the center electrode. The 5 electrodes may be arranged relative to the magnet such that substantially all magnetically responsive beads are arranged in one end of the elongated droplet and following splitting they remain together in one of the daughter droplets. The other daughter droplet will be composed substantially of supernatant liquid and can be transported away. Similar operations can be used for splits of other droplet sizes, e.g., deactivating the third of four electrodes underlying a 4X droplet to yield one 1X and one 3X droplet, with the magnetically responsive beads being retained in either the 1X or the 3X droplet. In Figure 5G, a 2X wash buffer droplet 528 is transported along droplet operations electrodes 510 and combined using droplet operations with 2X merged sample droplet 516 to form a 4X merged sample/wash droplet 516. Merged sample/wash droplet 516 may be divided using droplet operations into two droplets (e.g., 2X/2X or 1X/3X): one droplet that has magnetically responsive beads 524 therein and one droplet without a substantial amount of magnetically responsive beads 524 (e.g., supernatant droplet). The steps shown in Figures 5F, 5G and 5H may be repeated multiple times (e.g., 6 times) until a sufficient degree of purification is achieved. Figure 5I shows an optional step in which a 1X supernatant droplet 526 is split off a 2X droplet using droplet operations to form a 1X sample droplet 516, which is retained at a droplet operations electrode 510 that is within the magnetic field of magnet 514. This may be accomplished by elongating the 2 X droplet along 3 underlying electrodes to elongate the droplet, and then deactivating the central electrode. The 3 electrodes may be arranged relative to the magnet such that substantially all magnetically responsive beads are arranged in one end of the elongated droplet, and following splitting, they remain together in one of the daughter droplets. The other daughter droplet will be composed substantially of supernatant liquid and can be transported away.

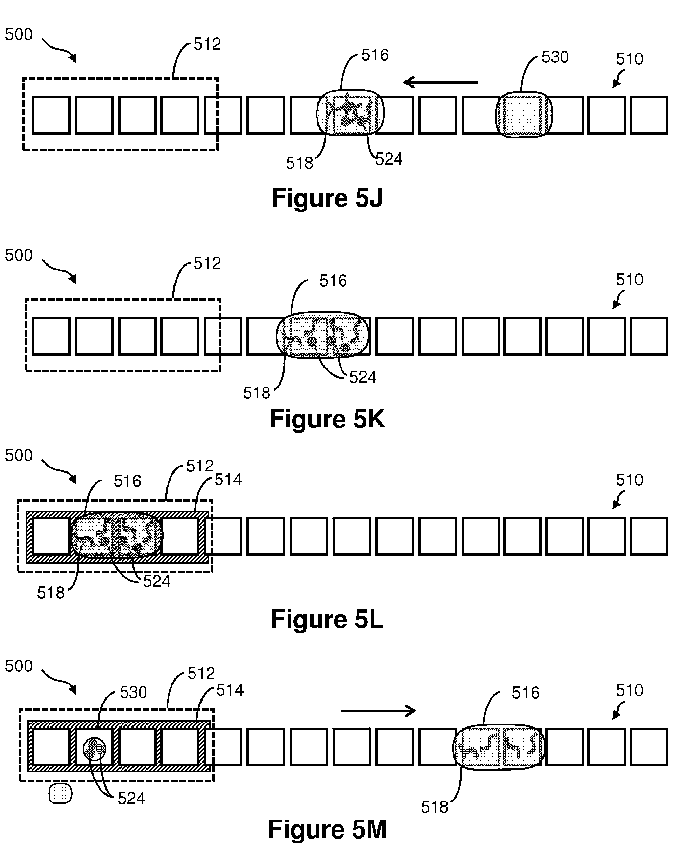

Figures 5J and 5K show an incubation process in which a 1X elution buffer droplet 530 is merged using droplet operations with 1X sample droplet 516. Magnet 514 is moved away from bead immobilization zone 512. Sample droplet 516, which has magnetically responsive beads 524 and eluted nucleic acid 518 therein, is transported using droplet operations away from the droplet operations electrode 510 that is within the magnetic field of magnet 514 and away from bead immobilization zone 512. 1X elution buffer droplet 530 is merged using droplet operations with sample droplet 516 to form a 2X merged sample/elution droplet. Merged sample/elution droplet 516 is incubated at room temperature for a period of time (e.g., about 1 min) sufficient to elute bound nucleic acid 518 from magnetically responsive beads 524.

Figure 5L shows 2X merged sample/elution droplet 516, which has magnetically responsive beads 524 and bound nucleic acid 518 therein, transported using droplet operations to a droplet operations electrode 510 that is within the magnetic field of magnet 514 (i.e., into bead immobilization zone 512). Magnet 514 is moved into position within bead immobilization zone 512 such that 2X merged sample/elution droplet 516 is within the magnetic field of magnet 514. Magnetically responsive beads 524 are immobilized by the magnetic field of magnet 514.

Figure 5M shows a 2X sample droplet 516 that includes substantially all of blunt-ended nucleic acid 518 split off (i.e., full bead snap-off) using droplet operations and transported away from at the droplet operations electrode 510 that is within the magnetic field of magnet 514 and away from magnet 514. Magnetically responsive beads 524 remain immobilized on at least one droplet operations electrode 510 that is within the magnetic field of magnet 514 by the magnetic field of magnet 514. Sample droplet 516, which includes blunt-ended nucleic acid 518, may be transported for further processing, i.e., A-tailing and adapter ligation. Another droplet can be transported into contact with the remaining beads, which will typically have a small amount of liquid surrounding them; the droplet will merge with this small amount of liquid, and the beads can be transported away. Optionally, the magnet can be deactivated or moved away during this operation in order to permit the beads to be transported away.

The steps shown in Figures 5B through 5M may be repeated for the subsequent processing reactions, e.g., A-tailing and adapter ligation, of the library construction protocol. Referring to Figures 5B and 5C, 2X sample droplet 516 that includes substantially all of blunt-ended nucleic acid 518 is merged using droplet operations with a second 1X reagent droplet 520 to form a 3X merged sample droplet. In this step, reagent droplet 520 includes enzyme and reaction components for an A-tailing reaction. Incubation of merged sample droplet 516 is performed at 37° C for about 30 minutes. Merged sample droplet 516 may be processed (i.e., enzyme and bead incubations and bead washing) as described in reference to Figures 5D through 5M. Sample droplet 516, which includes A-tailed nucleic acid 518, may be transported for further processing, i.e., adapter ligation.

Referring to Figures 5B and 5C, 2X sample droplet 516, which includes substantially all of A-tailed nucleic acid 518, is merged using droplet operations with a third 1X reagent droplet 520 to form a 3X merged sample droplet. In this step, reagent droplet 520 includes enzyme and reaction components for adapter ligation. Incubation of merged sample droplet 516 is performed at room temperature for about 5 to about 30 minutes. Merged sample droplet 516 may be processed (i.e., enzyme and bead incubations and bead washing) as described in reference to Figures 5D through 5M. Sample droplet 516, which includes substantially all of adapter-ligated nucleic acid 518, may be transported for further processing. In one example, nucleic acid 518 in sample droplet 516 may be amplified by PCR. The number of PCR amplification cycles used may be sufficiently limited (e.g., about 8 to 15 cycles) to minimize sequence bias of the amplification protocol. In another example, nucleic acid 518 in sample droplet 516 may be transported to an output reservoir for subsequent analysis by quantitative PCR and gel electrophoresis, and in some embodiments, collection for input into a next-generation sequencing process.

In one embodiment a dye may be added in the filler fluid or the droplet to facilitate visualization of the droplet for removal from the droplet operations gap of the droplet actuator. In one embodiment the processed sample droplet is removed from the droplet operations gap through an opening in the droplet actuator, such opening may be in top substrate, bottom substrate, or between the two substrates, such as a fluid path via an opening in the gasket.

In one embodiment, the library preparation protocols of the invention may make use of beads which are able to bind nucleic acid at a first pH and elute nucleic acid at a second pH. For example, a first droplet which is output from any of the enzyme steps of the library preparation protocols of the invention may be combined with a second droplet including beads which are able to bind nucleic acid at a first pH yielding a combined third droplet in which nucleic acid is captured on the beads. One or more additional droplets may be combined with the third droplet as necessary to ensure capture of nucleic acid on the beads. The third droplet may then be subject to a droplet-based bead washing protocol. A wash droplet may be combined with the third droplet; alternatively, the third droplet may be split prior to introduction of the wash droplet, yielding a bead-containing droplet and a supernatant droplet; and the wash droplet may be combined with the bead-containing droplet. In either case, the beads may be immobilized, the combined droplet may be split to yield a supernatant droplet and a bead-containing droplet. The bead-containing droplet preferably includes all or substantially all of the beads. The merge-immobilize-and-split technique may be repeated as necessary to achieve a desired reduction in contaminants in the resulting bead-containing droplet, preferably retaining all or substantially all of the beads in the process. The wash droplets may include a buffer having a pH which is suitable to retain the nucleic acid on the beads. Following washing, the droplet including the washed beads may be combined using droplet operations with one or more droplets having a pH selected to elute the nucleic acid from the beads. The beads may be restrained, and the surrounding supernatant pulled from around the beads by transporting the droplet away from the restrained beads. In an alternative embodiment, this process may be used to isolate other molecules of interest, such as RNA, proteins, peptides, macromolecules, or small molecules. Importantly, the inventors have discovered that this process may be effectively conducted using droplets surrounded by oil. In one embodiment, some or all of the droplet operations necessary for accomplishing the process are accomplished using electrowetting-mediated droplet operations. Beads may be restrained using physical barriers or obstacles and/or by using a magnet to restrain magnetically responsive beads. Examples of beads suitable for use with this process are described in Baker, U.S. Patent 6,914,137, entitled "Isolation of nucleic acids," issued on July 5, 2005, which is incorporated by reference for its teaching concerning the composition of such beads and conditions for capturing and eluting substances, such as DNA, using such beads. The CHARGESWITCH® beads described above are an example of such beads.

In another embodiment, the nucleic acid purification techniques of the invention include combining a droplet comprising nucleic acid for purification with a droplet comprising beads which adsorb (e.g., non-covalently binding) nucleic acid molecules in a reversible manner. Examples of such beads include those described in McKernan, et al., U.S. Patent 6,534,262, entitled "Solid phase technique for selectively isolating nucleic acids," granted on March 18, 2003, the entire disclosure of which is incorporated herein by reference for its disclosure concerning beads (referred to in that patent as particles) for capturing nucleic acids and chemistry for eluting nucleic acids from such beads. For example, solid phase reversible immobilization beads, such as SPRI® beads (available from Agencourt Bioscience Corp.) may be used. SPRI® beads are constructed using a core shell process that begins with a polystyrene core that is first coated with a layer of magnetite (iron) and then finally encapsulated with a polymer layer that contains carboxyl functional groups. In one embodiment SPRI® beads may be provided in a buffer droplet including from about 0.01 to about 0.1 % Tween-20, or TE buffer with from about 0.01 to about 0.1% Tween-20, or Tris buffer with from about 0.01 to about 0.1% Tween-20. Note that these buffers may also be used to wash the SPRI® beads. SPRI® beads may be kept in suspension by periodic mixing within the reservoir, e.g., by activating and deactivating electrowetting electrodes causing the reagent droplet to move around within the reservoir, or to move in and out of the reservoir, or to be transported back and forth through a fluid path extending from an external reservoir into the droplet operations gap, or transported in and out of a fluid path, e.g., using capillary forces to force the droplet at least partially into the fluid path and electrowetting forces to pull the droplet out of the fluid path.

The library construction protocol of the invention provides consistent and efficient recovery of nucleic acid at each purification step in the protocol. In one embodiment, nucleic acid is immobilized on magnetically responsive beads, and a movable magnet and a series of merge and split operations are used to purify the nucleic acid between each step in the library construction protocol. In another embodiment, nucleic acid is immobilized on magnetically responsive beads (e.g., SPRI® beads) and a magnet and a series of droplet merge-and-split wash steps using alcohol wash droplets are used to purify the nucleic acid between each step in the library construction protocol. The organic wash droplet may be composed of from about 1 to about 100%, or about 10 to about 90%, or about 30 to about 80%, or about 50 to about 75% of any polar protic solvent, with the remainder being water and other substances, including a surfactant, such as those described herein. In one embodiment, the solvent portion of the droplet is an alcohol and/or carboxylic acid. In another embodiment, the solvent is an alcohol and/or carboxylic acid having from 1 to 8 carbons, or from 1 to 6 carbons. The alcohol and/or carboxylic acid may be cyclic, linear, or branched. Examples include hexanol, pentanol, butanol, propanol, isopropanol, ethanol, methanol, formic acid, acetic acid, propionic acid, butyric acid, valeric acid.

The wash droplet may include at least about 50%, 60%, or 80% v/v alcohol. The alcohol may, for example, have from 1 to 8 carbons, or from 1 to 6 carbons. The alcohol may be cyclic, linear, or branched. Examples of suitable alcohols include hexanol, pentanol, butanol, propanol, isopropanol, ethanol, and methanol.

The wash droplet may include from about 0.01 to about 1% v/v surfactant, or with about 0.01 to about 0.1% v/v surfactant. Suitable surfactants include anionic surfactants, such as bile salts; cationic surfactants, such as quaternary ammonium salts; non-ionic surfactants, such as alkanoyl-N-hydroxyethylglucanide, alkanoyl-N-methylglucanide, alkyl glycosides, cycloalkanoyl hydroxyethylglucanide, cycloalkyl glycosides, polyoxyethylenes; and Zwitterionic surfactants, such as alkyl betaines, alkyl phosphocholines, alkyl sulfobetaines, cycloalkyl phosphocholines, phosphoethanolamines, non-detergent sulfobetaines, sulfobetaines. Polysorbate surfactants are preferred.

The wash droplet may optionally include one or more salt, such as an inorganic salt and/or an organic salt. The one or more salts may, for example, include one or more aluminum, ammonium, barium, beryllium, calcium, cesium, lithium, magnesium, potassium, rubidium, sodium, or strontium salts. The one or more salts may, for example, include one or more chloride, fluoride, oxide, oxoanion, carbonate, bicarbonate, hydroxide, nitrate, phosphate, sulfate salts. The one or more salts may be provided in the wash droplet solution in any concentration which does not eliminate the suitability of the solution for use in a droplet actuator as a wash droplet. For example, one or more salts may be provided in the wash droplet solution in a concentration ranging from about 0.001 to about 100 mM, or from about 0.001 to about 10 mM, or from about 0.001 to about 1 mM.

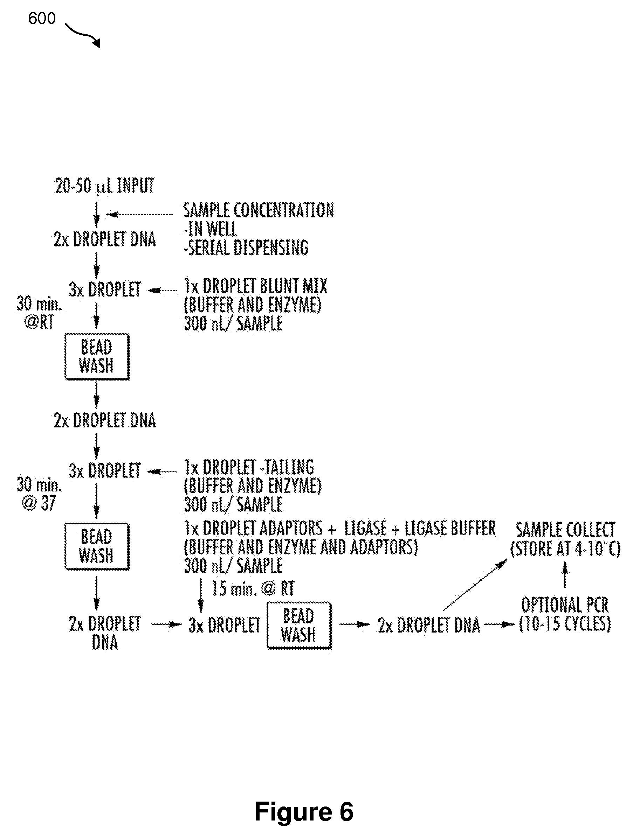

**Figure 6** illustrates a flow diagram of a method 600 of preparing a nucleic acid library according to a protocol that uses magnetically responsive beads (e.g., SPRI® beads) and an alcohol-based bead washing protocol, the number of unit-sized droplets in the reaction droplet volumes may be varied. A sheared nucleic acid sample (e.g., 20-50 µL) may be loaded into a sample input reservoir of a droplet actuator. The nucleic acid sample may be concentrated on-actuator prior to processing. Sample concentration may, for example, be performed using an in-reservoir concentration protocol or using a serial dispensing concentration protocol.

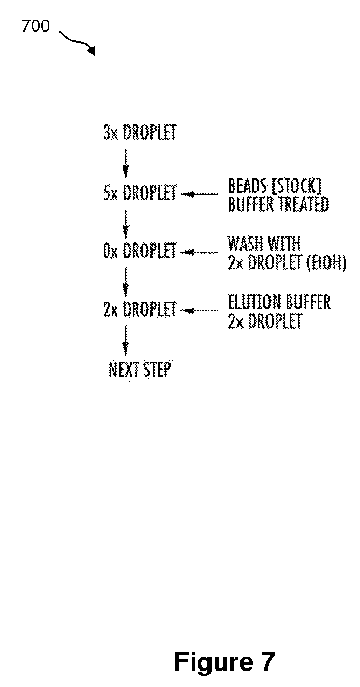

A 2X sample droplet may be dispensed and combined using droplet operations with a 1X reagent droplet (e.g., 300 nL/sample) that includes enzymes (e.g., T4 DNA polymerase) and reagents for blunt-ending sheared nucleic acid fragments, yielding a 3X merged sample droplet. In some embodiments, polynucleotide kinase and reagents may be included in the reaction droplet at this step. The merged 3X sample droplet may be incubated at room temperature for a period of time that is sufficient for the blunt-ending reaction to come to completion, e.g., about 30 minutes. The blunt-ended nucleic acid may be captured (5X sample/SPRI® beads droplet), washed (0X sample/bead droplet), and eluted (2X eluted sample droplet) as described in reference to Figure 7.

The 2X eluted sample droplet, which includes substantially all of blunt-ended nucleic acid, may be merged using droplet operations with a second 1X reagent droplet (300 nL/sample) to form a 3X merged sample droplet. In this step, the 1X reagent droplet includes enzyme and reaction (3x buffer and enzyme) components for an A-tailing reaction. Incubation of the merged sample droplet may be performed at about 37 °C for about 30 minutes. The processed nucleic acid may be captured (5X sample/SPRI® beads droplet), washed (0X sample/bead droplet) and eluted (2X eluted sample droplet) as described in reference to Figure 7.

The 2X eluted sample droplet, which includes substantially all of the A-tailed nucleic acid, is merged using droplet operations with a third 1X reagent droplet to yield a 3X merged sample droplet. In this step, the 1X reagent droplet (∼300 nL/sample) includes enzyme and reaction components for adapter ligation (e.g., adapters, ligase, and ligase buffer). Incubation of the 3X merged sample droplet may be performed at room temperature for about 5 to about 30 minutes. The processed nucleic acid may be captured (5X sample/SPRI® beads droplet), washed (0X sample/bead droplet) and eluted (2X eluted sample droplet) as described in reference to Figure 7. In one embodiment, the 2X eluted sample droplet, which includes substantially all of the processed nucleic acid, may be transported using droplet operations to a sample collection and removal reservoir. The 2X eluted sample droplet may be stored in the sample collection and removal reservoir at from about 4 to about 10 °C. In another embodiment, the processed nucleic acid (e.g., the adapter ligated DNA) may be amplified using PCR (e.g., 10-15 cycles) prior to transporting the sample droplet to a sample collection and removal reservoir.

**Figure 7** illustrates a flow diagram of bead-based wash/elute process 700. A 3X droplet (e.g., a 3X sample droplet with nucleic acid therein) may be merged using droplet operations with a 2X reagent droplet that includes magnetically responsive beads (e.g., SPRI® beads) to yield a 5X merged sample/bead droplet. The merged sample/bead droplet may be incubated at room temperature for a period of time that is sufficient for nucleic acid fragments to bind to the magnetically responsive beads (e.g., about 5 minutes). The 5X merged sample/bead droplet may be washed using a bead washing protocol, except a 5X supernatant droplet may be split off using droplet operations to yield a 0X sample/bead droplet (i.e., beads with bound nucleic acid). A 2X wash droplet (i.e., EtOH) may be transported using droplet operations along droplet operations electrodes and passes across the 0X sample/bead droplet, which has bound nucleic acid. A 2X elution buffer droplet (e.g., a water droplet) may be transported using droplet operations and combined with the washed 0X sample/bead droplet to yield a 2X elution buffer droplet. The elution buffer droplet may be incubated at room temperature for a period of time (e.g., about 1 min) sufficient to elute bound nucleic acid from the magnetically responsive beads. A 2X eluted sample droplet that includes substantially all of the nucleic split off (i.e., full bead snap-off). The 2X eluted sample droplet, which includes the nucleic acid, may be transported for further processing, i.e., A-tailing reaction and adapter ligation in a library construction protocol.

The methods of the invention may be further adapted to provide for total automated construction of small (e.g., about 200 bp) and large (e.g., about 400 bp to about 10 kb) nucleic acid libraries. In one embodiment, the methods of the invention may be adapted to include automated fragmentation of nucleic acids, i.e., RNA or DNA, on a droplet actuator. In one example, the methods of the invention may be adapted to include fragmentation and reverse transcription of RNA for a RNA-based library. In this example, nucleic acid fragmentation may be performed using a buffer-based fragmentation. In another example, fragmentation of nucleic acid (e.g., bacterial or eukaryotic) may be performed using an enzyme-based fragmentation reaction. For example, a fragmentase such as NEBNext™ dsDNA Fragmentase™ may be used to generate dsDNA breaks in a time-dependent manner to yield about 100-800 bp DNA fragments. NEBNext dsDNA Fragmentase contains two enzymes, one enzyme randomly generates nicks on the dsDNA and the other enzyme recognizes the nicked site and cuts the opposite DNA strand across from the nick, producing dsDNA breaks. In another example, a transposase, such as Nextera's Transposome™ technology, may be used to generate dsDNA breaks. The Transposome™ complex includes free transposon ends and a transposase. When this complex is incubated with dsDNA, the DNA is fragmented and the transferred strand of the transposon end oligonucleotide is covalently attached to the 5' end of the DNA fragment. By varying the concentration of Transposome complexes, the size distribution of the fragmented and tagged DNA library may be controlled. A sample droplet may be combined in a droplet operations gap with a droplet comprising fragmentation reagents, and the resulting droplet may be incubated to yield fragmented nucleic acid. The requisite droplet operations may be performed in a filler fluid.

In another embodiment, the methods of the invention may be adapted to include quantitation of input (unprocessed sample) and output (processed sample) nucleic acid. In one example, qPCR may be used to determine the concentration of DNA in each processed library sample. Using the qPCR data, each processed sample may be used as is, or diluted using an on-actuator dilution protocol, or further amplified using an on-actuator PCR protocol to achieve appropriate ranges of concentrations prior to pooling of samples.

In another embodiment, the methods of the invention may be adapted to provide for quality assurance testing of the constructed library. In one example, a probe-based hybridization assay such as TaqMan or Molecular Beacon may be used to verify the quality of the completed library. TaqMan and Molecular Beacon probes may be used for real-time or endpoint PCR analysis.

Because of the flexibility and programmability of digital microfluidics, library construction protocols of the invention may be readily adapted for use with a number of different next-generation sequencing platforms. Examples of next-generation sequencing platforms include, but are not limited to, Illunina, 454, SOLiD, PacBio and Ion Torrent.

**Figures 8A, 8B, and 8C** illustrate top views of an example of a portion of an electrode arrangement 800 of a droplet actuator and show a process of snapping off beads, while leaving behind with the beads the smallest amount of liquid possible. Electrode arrangement 800 may include an arrangement of droplet operations electrodes 810 (e.g., electrowetting electrodes).

Droplet operations are conducted atop droplet operations electrodes 810 on a droplet operations surface. In one example, electrode arrangement 800 includes droplet operations electrodes 810A, 810B, 810C, 810D, 810E, and 810F that are arranged in a line or path. Further, a magnet 812 is positioned, for example, such that droplet operations electrode 810A is within its magnetic field. Magnet 812 may be a permanent magnet or electromagnet.

An aspect of the method of the invention of snapping off beads is that it uses an electrowetting surface area that is greater than the footprint of the droplet, which is key to leaving behind with the beads the smallest amount of liquid possible. In one example, when the droplet is a 1X droplet, the electrowetting surface area used in the process of snapping off beads is 2X. In another example, when the droplet is a 2X droplet, the electrowetting surface area used in the process of snapping off beads is 3X. In another example, when the droplet is a 3X droplet, the electrowetting surface area used in the process of snapping off beads is 4X, and so on. By way of example, Figures 8A, 8B, and 8C show the scenario of beads being snapping off a 2X droplet while using a 3X electrowetting surface area. In this example, the process of snapping off beads, while leaving behind with the beads the smallest amount of liquid possible, may include, but is not limited to, the following steps.

Referring to Figure 8A, in this step, a 2X droplet 814 that contains a certain amount of magnetically responsive beads 816 is positioned atop droplet operations electrode 810A and 810B. This is because, in this step, droplet operations electrodes 810A and 810B are turned ON, while droplet operations electrodes 810C, 810D, 810E, and 810F are turned OFF. Because droplet operations electrode 810A is within the magnetic field of magnet 812, the magnetically responsive beads 816 that are in 2X droplet 814 are attracted toward droplet operations electrode 810A.

Referring to Figure 8B, in this step, droplet operations electrode 810A, 810E, and 810F are turned OFF, while droplet operations electrodes 810B, 810C, and 810D are turned ON. This causes the 2X droplet 814 to stretch across three droplet operations electrodes 810. The tip of this stretched 2X droplet 814 is at about the edge of magnet 812 and still holding the magnetically responsive beads 816. The magnetically responsive beads 816 are held at the tip of this stretched 2X droplet 814 because of the magnetic field of magnet 812. The stretching action of the 2X droplet 814 across three droplet operations electrodes, which is a 3X electrowetting surface area, causes the tip of the 2X droplet 814 to take on a somewhat pointed geometry. Essentially, pulling access liquid away from the edge of magnet 812, while still retaining the magnetically responsive beads 816.

Referring to Figure 8C, in this step, droplet operations electrodes 810A, 810B, and 810F are turned OFF, while droplet operations electrodes 810C, 810D, and 810E are turned ON. This causes the 2X droplet 814, which is still stretched across a 3X electrowetting surface area, to move further away from magnet 812. This causes the magnetically responsive beads 816 to be snapped off at about the edge of magnet 812, while leaving behind with the beads the smallest amount of liquid possible. The 2X droplet 814, which is still stretched across a 3X electrowetting surface area, is substantially bead free.

An important challenge in making library preparation chemistry work on a droplet nicroactuator involves the typical need for conducting droplet operations using organic solvent wash droplets. In various embodiments, the organic wash droplet may be composed of from about 1 to about 100%, or about 10 to about 90%, or about 30 to about 80%, or about 50 to about 75% of any polar protic solvent, with the remainder being water and other substances, including a surfactant, such as those described herein. In one embodiment, the solvent is one or more alcohols and/or carboxylic acids. In another embodiment, the solvent is one or more alcohols and/or carboxylic acids having from 1 to 8 carbons, or from 1-6 carbons. The alcohols and/or carboxylic acids may be cyclic, linear, or branched. Examples include hexanol, pentanol, butanol, propanol, isopropanol, ethanol, methanol, formic acid, acetic acid, propionic acid, butyric acid, valeric acid. Droplet actuator modifications and techniques for dispensing and conducting droplet operations using such solvents are discussed elsewhere herein.

### 7.3 Droplet Actuator Configuration and Assembly

A droplet actuator may, for example, include a bottom substrate and a top substrate. Electrodes may be provided on either or both substrates and arranged for conducting droplet operations. The droplet actuator may be adapted for multiplexed library construction and for application of a specific library construction protocol. For example, composition of the filler fluid and surfactant doping concentration may be selected for performance with reagents used in the nucleic acid library construction protocol based on the instant disclosure. Droplet transport voltage and frequency may also be selected for performance with reagents used in the library construction protocol based on the instant disclosure. In one example, the droplet actuator is configured for construction of 10, 20, 30, 40, 50 or more different libraries in parallel. Design parameters may be varied by one of skill in the art in view of the instant disclosure, e.g., number and placement of on-actuator reservoirs, number of independent electrode connections, size (volume) of different reservoirs, placement of magnets/bead washing zones, electrode size, inter-electrode pitch, and gap height (between top and bottom substrates).

The droplet actuator may be designed to fit onto an instrument deck that houses certain components that may be useful with respect to droplet actuators, such as one or more magnets for immobilization of magnetically responsive beads and one or more heater assemblies for controlling the temperature within certain reaction and/or washing zones. The magnets associated with the instrument deck may be permanent magnets. The magnets may be fixed in position or they may be movable.

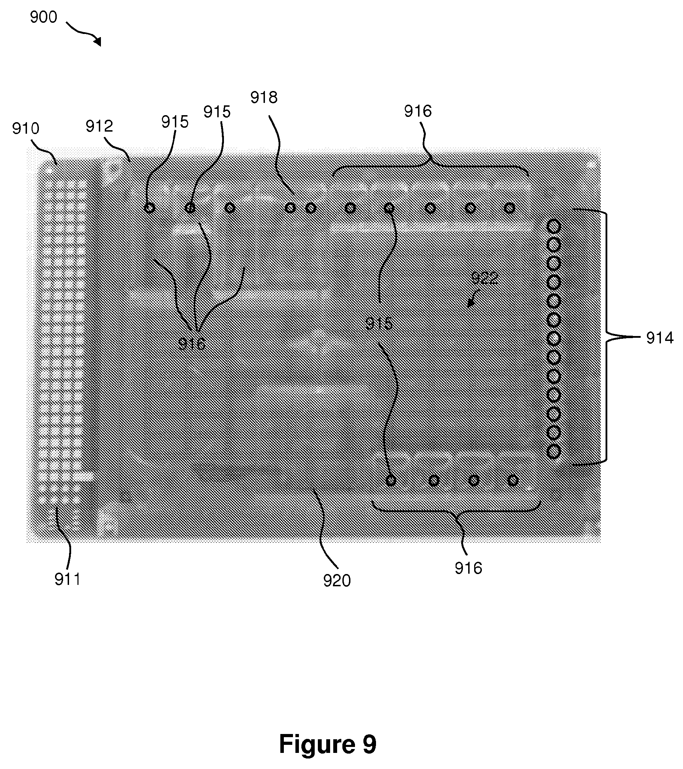

Figure 9 illustrates a top view of an example of a droplet actuator 900 that is suitable for use in conducting a multiplexed library construction protocol. Droplet actuator 900 may include a bottom substrate 910 and a top substrate 912 that are separated by a droplet operations gap (not shown). Bottom substrate 910 may, for example, be a printed circuit board (PCB). Top substrate 912 may be formed, for example, of glass, injection-molded plastic, silicon. Top substrate 912 and may be coated on the side facing the droplet operations gap with a conductor, such as a conductive ink or indium tin oxide (ITO), and may further be coated with a hydrophobic coating, such as a fluoropolymer coating.

Referring to top substrate 912 of Figure 9, top substrate 912 includes a series of liquid reservoirs 916 of various sizes and shapes, each designed to accept a volume of liquid. The liquids may, for example, include the various reagents necessary for conducting a library preparation protocol of the invention. Examples include blunt ending reagents, phosphorylation reagents, A-tailing reagents, adapter ligation reagents, wash buffers, nucleic acid capture beads, and nucleic acid amplification reagents. Each reservoir includes an opening establishing a fluid path from the reservoir into the droplet operations gap. Reagents flow through openings 915 into the droplet operations gap, where they may be subjected to electrode-mediated droplet operations, such as electrowetting-mediated droplet operations. Using droplet operations, , e.g., using electrowetting-mediated or dielectrophoresis-mediated droplet operations, the reagents may be dispensed into sub-droplets and transported into contact with other reagent droplets and/or sample droplets in accordance with a droplet operations protocol, such as a library construction protocol. Top substrate 912 may also include one or more pipette injection openings 914 for injecting liquids, such as sample liquids, e.g., sheared nucleic acid, optionally immobilized on magnetically responsive beads.

Bottom substrate 910 includes an electrowetting electrode arrangement 922. Electrode arrangement 922 includes dispensing electrodes arranged to receive liquid flowed into the droplet operations gap via openings 915 or pipette injection sites 914. Dispensing electrodes of electrode arrangement 922 are interconnected through, for example, a path, line, and/or array of droplet operations electrodes (e.g., electrowetting electrodes). Electrodes are wired to contacts 910.

In one example, nucleic acid samples may be loaded into the droplet operations gap via sample injection sites 914, e.g., by manually or robotically pipetting the samples into through the sample injection sites into the droplet operations gap. Different nucleic acid samples may be processed in different electrode lanes on droplet actuator 900 to reduce the possibility of contamination between the samples. In another embodiment, each sample may include its own lane, and each lane may include an opening for extracting the sample from that lane so that it is not necessary for samples to be extracted from a common opening. Thus, at the end of the library construction protocol, the adapter-ligated nucleic acid from each of the lanes may be cleaned up and collected in different outlets or outlet reservoirs (not shown).

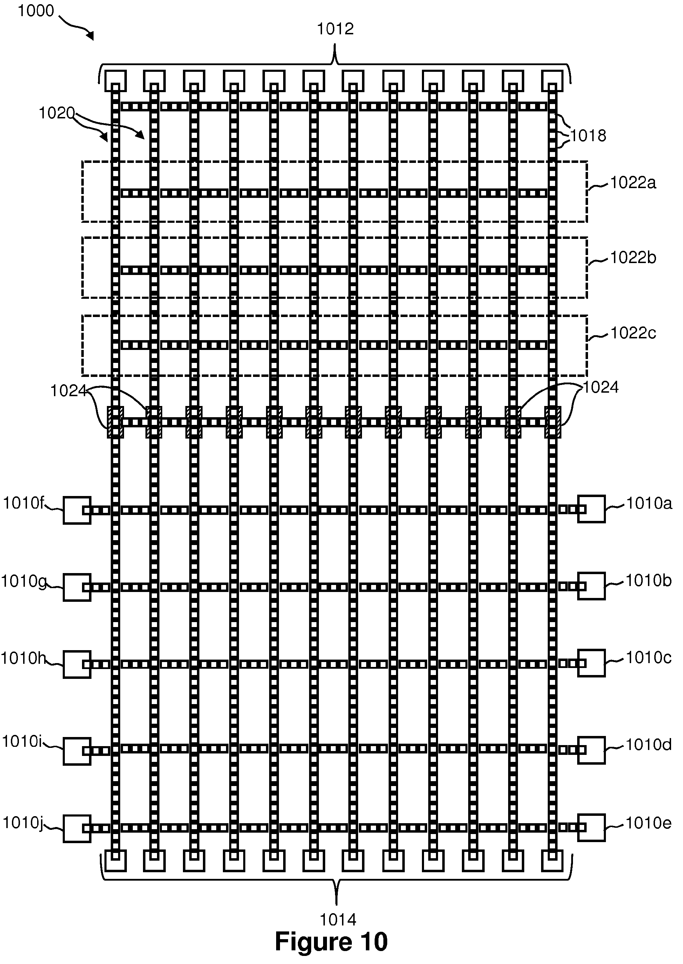

**Figure 10** illustrates a top view of another example of an electrode arrangement 1000 configured for processing a nucleic acid sample for construction of a nucleic acid library. In this example, electrode arrangement 1000 is configured for processing up to 12 different nucleic acid samples in parallel for construction of twelve different nucleic acid libraries. Electrode arrangement 1000 includes dispensing electrodes 1010 and 1012, as well as a series of sample collection electrodes 1014. Dispensing electrodes 1010 and 1012 and sample collection electrodes 1014 may be aligned to receive liquids from or flow liquids into openings or fluid paths coupling external reservoirs (e.g., as described with respect to Figure 9) with the droplet operations gap. In operation, dispensing electrodes 1010 may be used for dispensing reagents. Examples include blunt ending reagents, phosphorylation reagents, A-tailing reagents, adapter ligation reagents, wash buffers, nucleic acid capture beads, and nucleic acid amplification reagents. Dispensing electrodes 1012 are used for dispensing sample samples, as shown here, 12 sample input dispensing electrodes 1012 for 12 nucleic acid samples. Sample output collection electrodes 1014 are used for receiving sample fluids, as shown here, twelve sample output collection electrodes 1014 for receiving processed nucleic acid libraries for recovery from the droplet operations gap. Reagent dispensing electrodes 1010, sample input dispensing electrodes 1012, and sample output collection electrodes 1014 are interconnected through an arrangement, such as a path or array, of droplet operations electrodes 1018 (e.g., electrowetting electrodes). A path of droplet operations electrodes 1018 extending from each sample input dispensing electrode 1012 and its corresponding sample output collection electrode 1014 forms dedicated electrode lanes 1020 (e.g., 12 dedicated electrode lanes 1020). Dedicated electrode lanes 1020 provide individual reaction zones for processing different nucleic acid samples. The use of dedicated lanes for sample droplets minimizes cross-contamination among different nucleic acids. Each nucleic acid sample traverses a path in the droplet operations gap that does not cross the path of any other sample, thereby minimizing the possibility of cross contamination. Further, while reagent droplets do traverse the sample paths, the protocol may be executed such that reagent droplets always traverse the sample paths before the sample droplets have traversed the same paths, therefore, eliminating the possibility that reagent droplets may be a source of nucleic acid contamination between sample lanes.

Electrode arrangement 1000 may be provided with one or more temperature control zones 1022. In one example, three temperature control zones 1022 may be used (i.e., temperature control zones 1022a, 1022b, and 1022c). Temperature control elements (not shown) establish the temperature of a region of filler fluid (not shown) in the temperature control zones 1022. As noted elsewhere, the temperature control elements may in some embodiments be associated with a deck for mounting the droplet actuator cartridge and electrically coupling the cartridge to a system that controls operations of the cartridge, such as droplet operations and detection. A temperature control zone may be provided at about 37 °C, which is a temperature sufficient suitable for enzymatic activity in an A-tailing reaction. The temperature control zones may be established at temperature suitable for conducting amplification of the nucleic acid samples or products. While three temperature control zones 1022 are shown, any suitable number of temperature control zones 1022 may be associated with electrode arrangement 1000.

The droplet actuator may include or be associated with one or more magnets 1024 (e.g., twelve magnets 1024) may be positioned in proximity to respective dedicated electrode lanes 1020 for retaining magnetically responsive beads. Each magnet 1024 may, for example, be a permanent magnet or an electromagnet. In one embodiment, magnets 1024 may be a movable into proximity with, and away from, a corresponding dedicated electrode lane 1020. Each magnet 1024 is positioned in a manner which ensures spatial immobilization of nucleic acid-attached beads during washing between enzymatic reactions and bead removal following elution of processed nucleic acid. In some embodiments, mixing and incubations may be performed in dedicated electrode lanes 1020 away from the magnet.

Either or both substrates may include electrodes arranged for conducting one or more droplet operations in this enclosure. The droplet operations may, for example, be electrowetting-mediated, dielectrophoresis-mediated, and/or optoelectrowetting-mediated droplet operations. The droplet operations may alternatively, or additionally, be mediated by other mechanisms, such as devices that induce hydrodynamic fluidic pressure, such as those that operate on the basis of mechanical principles (e.g. external syringe pumps, pneumatic membrane pumps, vibrating membrane pumps, vacuum devices, centrifugal forces, piezoelectric/ultrasonic pumps and acoustic forces); electrical or magnetic principles (e.g. electroosmotic flow, electrokinetic pumps, ferrofluidic plugs, electrohydrodynamic pumps, attraction or repulsion using magnetic forces and magnetohydrodynamic pumps); thermodynamic principles (e.g. gas bubble generation/phase-change-induced volume expansion); other kinds of surface-wetting principles (e.g. electrowetting, and optoelectrowetting, as well as chemically, thermally, structurally and radioactively induced surface-tension gradients); gravity; surface tension (e.g., capillary action); electrostatic forces (e.g., electroosmotic flow); centrifugal flow (substrate disposed on a compact disc and rotated); magnetic forces (e.g., oscillating ions causes flow); magnetohydrodynamic forces; and vacuum or pressure differential. In certain embodiments, combinations of two or more of the foregoing techniques may be employed to conduct a droplet operation in a droplet actuator of the invention.

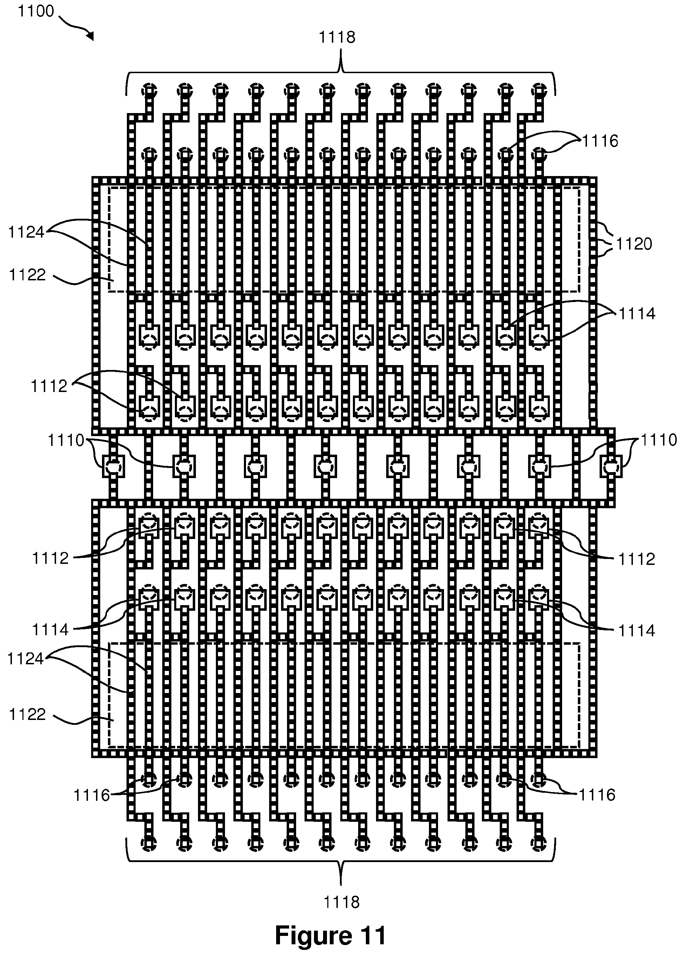

**Figure 11** illustrates a top view of another example of an electrode arrangement 1100 configured for processing of nucleic acid on a droplet actuator for construction of a nucleic acid library. In this example, reagent dispensing electrodes are positioned in a central region of the electrode arrangement and two sets of 12 each sample input dispensing electrodes and sample processing electrodes are on each end. The arrangement of dispensing electrodes, droplet operations electrodes (e.g., sample processing electrodes), and collection electrodes is such that a reagent droplet is dispensed and transported to a dedicated sample processing area (i.e., dedicated library construction lanes). Reaction products (i.e., processed nucleic acid) and waste products (e.g., wash droplets) are transported on the dedicated library construction lanes to dedicated sample collection and waste collection reservoirs, respectively. Electrode arrangement 1100 is configured for processing up to 24 different nucleic acid samples in parallel in dedicated reaction lanes for construction of 24 different nucleic acid libraries. A unique adapter-barcode tag that identifies each nucleic acid library may be coupled to each nucleic acid sample during the library preparation process.

Electrode arrangement 1100 includes multiple dispensing electrodes. For example, electrode arrangement 1100 may include one or more reagent dispensing electrodes 1110, e.g., 11 reagent dispensing electrodes 1110, for dispensing different reagent fluids (e.g., blunt-ending reagents, A-tailing reagents, adapter ligation reagents, bead solutions, wash buffer, elution buffer). Electrode arrangement 1100 may also include one or more sample input dispensing electrodes 1112 for dispensing sample fluids (e.g., 24 sample input dispensing electrodes 1112 for dispensing nucleic acid). Electrode arrangement 1100 may also include one or more adapter dispensing electrodes 1114 for dispensing adapter-barcode tags (e.g., 24 adapter dispensing electrodes 1114). Each arrangement of dispensing electrodes 1110, 1112, and 1114 may include a dedicated reagent transport lane 1111, 1112, and 1115, including electrodes arranged to transport dispensed reagent droplets from the dispensing electrodes 1110, 1112, and 1114 to their respective reaction lanes 1124 without requiring reagents to traverse any other reaction lanes. In an assembled droplet actuator, dispensing electrodes 1110, 1112, and 1114 will be associated with a fluid path that flows reagents into sufficient proximity with the dispensing electrodes that the dispensing electrodes may be utilized for dispensing droplets of the reagents onto a droplet operations surface or into a droplet operations gap. For example, the fluid path may be a path from a top substrate reservoir into the droplet operations gap. Dispensing electrodes 1110 are arranged to dispense droplets from either side of the arrangement, thus independently feeding reagents in two directions along dedicated transport lanes 1111 to dedicated reaction paths 1124.

Electrode arrangement 1100 may also include multiple collection electrodes. For example, electrode arrangement 1100 may include one or more sample output collection electrodes 1116 for receiving sample fluids (e.g., 24 sample output collection electrodes 1116 for receiving processed nucleic acid droplets). Electrode arrangement 1100 may also include one or more waste collection electrodes 1118 for collecting waste fluids (e.g., 24 waste collection electrodes 1118). Each arrangement of collection electrodes 1116 and 1118 may be contiguous with a dedicated reagent transport lane 1117 and 1119, respectively, including electrodes arranged to transport dispensed reagent droplets from the reaction lanes 1124 to collection electrodes 1116 and 1118 without requiring droplets to traverse any other reaction lanes. Sample output collection electrodes 1116 and/or waste collection reservoirs 1118 may be associated with a fluid path that flows reagents from these collection electrodes into a fluid path or reservoir or out of the droplet actuator for collection. For example, the fluid path may be a path from a collection electrode into a reservoir exterior to the droplet operations gap, or into a reservoir within a region of the droplet operations gap.

Reagent dispensing electrodes 1110, sample input dispensing electrodes 1112, adapter dispensing electrodes 1114, sample output collection electrodes 1116, and waste collection electrodes 1118 are interconnected through an arrangement, such as a path or array, of droplet operations electrodes 1120 (e.g., electrowetting electrodes). The arrangement of droplet operations electrodes 1120 provides a reaction zone 1122. In particular, an array of droplet operations electrodes 1120 extending from each sample input dispensing electrode 1112, adapter dispensing electrode 1114 and their corresponding sample output collection electrode 1116 and waste collection electrode 1118 forms dedicated electrode lanes 1124, e.g., two dedicated electrode lanes 1124 for each sample input. Dedicated electrode lanes 1124 within reaction zone 1122 provide individual reaction lanes for processing different nucleic acid samples (i.e., library construction) and "turn-off" lanes for shuttling reaction droplets during certain reaction steps (e.g., bead removal steps). The use of dedicated lanes for sample droplets and centralized reagent dispensing minimizes cross-contamination among different nucleic acids.

One or more magnets (not shown) may be located in proximity to dedicated electrode lanes 1124 for retaining magnetically responsive beads. The magnet may, for example, be a permanent magnet or an electromagnet. In one example, the magnet may be a movable magnet that may be moved into proximity with and away from its respective dedicated electrode lane 1124. Each magnet is positioned in a manner which ensures spatial immobilization of nucleic acid-attached beads during washing between enzymatic reactions and bead removal following elution of processed nucleic acid. Mixing and incubations may be performed in dedicated electrode lanes 1124 away from the magnet.

Reaction zone 1122 may also include one or more temperature control zones (not shown). Temperature control elements (e.g., heaters or heat sinks, not shown) may be located in proximity to dedicated electrode lanes 1124 within reaction zone 1122. The temperature control elements may be used to control the temperature of filler fluid (not shown) in vicinity of the temperature control zones.

In one embodiment, sample input dispensing electrodes may be designed for a small sample input volume. For example, a 1 µL sample volume may be loaded onto a droplet actuator and processed as a 660 nL droplet.

In another embodiment, sample input dispensing electrodes and reservoirs may be designed for a larger sample input volume. In one example, a bead concentration protocol may be used to concentrate and collect nucleic acid from a sample volume in an off-actuator sample reservoir. Magnetically responsive beads may be added to the large sample volume, e.g., about 10 to about 20 µL, prior to loading the sample onto a sample reservoir on the droplet actuator. The large volume sample may then be processed on-actuator using a bead concentration protocol into a 330 nL droplet. In another example, a large sample volume may be concentrated in an on-actuator sample reservoir. In this example, a series of 660 nL droplets may be sequentially incubated with magnetically responsive beads in an on-actuator sample reservoir. Mixing electrodes may be used to facilitate bead mixing. Because of the flexibility and programmability of a digital nicrofluidics, a sample processing protocol may be readily adapted for on-actuator, off-actuator or any combination of sample processing.

In another embodiment, each nucleic acid library (e.g., using the illustrated electrode arrangement 1100 up to 24 different nucleic acid libraries) may be collected at individual dedicated sample output collection electrodes 1116. Sample output collection reservoirs may, for example, be filled with buffer solution to facilitate removal of the processed nucleic acid sample from the reservoir. In one example, the sample output collection reservoir may be manually filled (e.g., manual pipetting) with buffer. In another example, the sample output reservoir may be filled with buffer using robotic instrumentation. Sample collection may, for example, be performed by manually removing (e.g., manual pipetting) the processed sample from each sample output collection reservoir (not shown). In another example, sample collection may be performed using robotic instrumentation to remove each processed sample from individual output collection reservoirs (not shown).

In another embodiment, one or more different nucleic acid libraries may be merged using droplet operations such that they are pooled on the droplet actuator prior to collection at a sample output collection electrode. Prior to pooling one or more nucleic acid libraries, the quantity of processed nucleic acid in each sample may be determined to ensure equivalent concentrations of all individual samples in the pool. In one example, qPCR may be used to determine the concentration of nucleic acid in each processed sample. Using the qPCR data, each processed sample may be used as is, or diluted using an on-actuator dilution protocol, or further amplified using an on-actuator PCR protocol to achieve appropriate ranges of concentrations prior to pooling of samples.

In another example, an on-actuator nucleic acid quantitation protocol may be used prior to processing the nucleic acid sample. In this example, the concentration of input nucleic acid for each sample is determined and adjusted (if necessary) for each sample prior to processing in a nucleic acid library construction protocol.

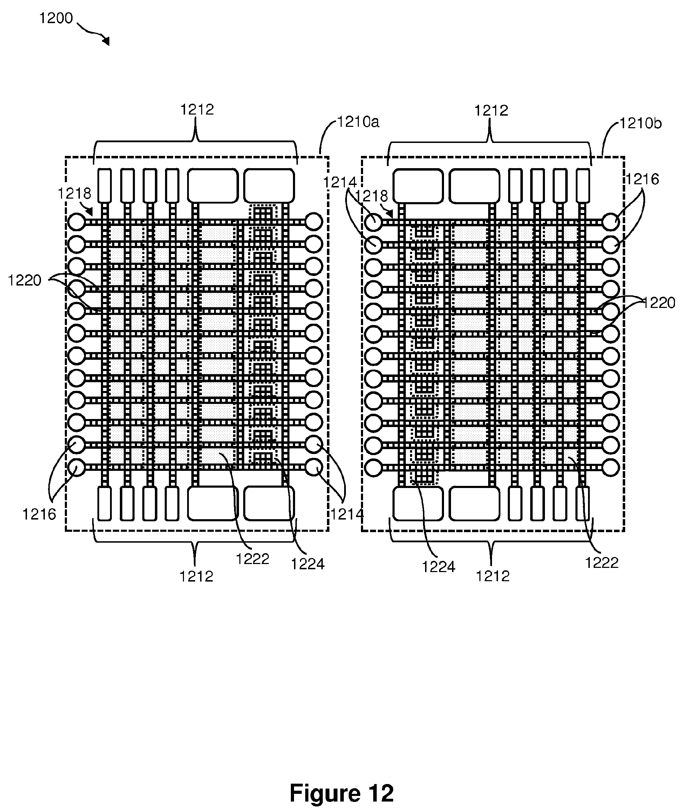

**Figure 12** illustrates a top view of another example of an electrode arrangement 1200 configured for processing of nucleic acid on a droplet actuator for construction of a nucleic acid library. In this example, electrode arrangement 1200 is configured for processing up to 12 different nucleic acid samples in parallel in each of two separate processing modules for up to 24 different nucleic acid libraries. In one embodiment, the processing modules are physically separated on the droplet actuator such that one processing module may be used independently of the other processing module. For example, one portion of the droplet actuator that includes one library processing module may be filled with a filler fluid (e.g., silicone oil) and used for processing up to 12 different nucleic acid libraries. Another portion of the droplet actuator that includes a second library processing module may be left unused for future use.

In one embodiment, electrode arrangement 1200 may include two sample processing modules 1210a and 1210b. While two sample processing modules 1210 are shown, any number and combination of sample processing modules may be used. Each sample processing module 1210 includes multiple electrodes. For example, each sample processing module 1210 may include one or more reagent dispensing electrodes 1212, e.g., 12 reagent dispensing electrodes 1212, for dispensing different reagent fluids (e.g., blunt-ending reagents, A-tailing reagents, adapter ligation reagents, bead solutions, wash buffer, elution buffer). Each sample processing module 1210 may also include one or more sample input dispensing electrodes 1214 for dispensing sample fluids (e.g., 12 sample input dispensing electrodes 1214 for dispensing nucleic acid). Each sample processing module 1210 may also include one or more sample output collection electrodes 1216 for receiving sample fluids (e.g., 12 sample output collection electrodes 1216 for receiving processed nucleic acid droplets). In another embodiment, sample output collection electrodes 1216 may be used as adapter dispensing electrodes.

Reagent dispensing electrodes 1212, sample input dispensing electrodes 1214, and sample output collection electrodes 1216 are interconnected through an arrangement, such as a path or array, of droplet operations electrodes 1218 (e.g., electrowetting electrodes). A path of droplet operations electrodes 1218 extending from each sample input dispensing electrode 1214 and its corresponding sample output collection electrode 1216 forms dedicated electrode lanes 1220, e.g., 12 dedicated electrode lanes 1220. Dedicated electrode lanes 1220 provide individual reaction zones for processing different nucleic acid samples. The use of dedicated lanes for sample droplets minimizes cross-contamination among different nucleic acids.

Electrode arrangement 1200 may include one or more temperature control zones 1222. In one example, three temperature control zones 1222 may be used (i.e., temperature control zones 1222a, 1222b, and 1222c). Temperature control elements (not shown) control the temperature of filler fluid (not shown) in vicinity of temperature control zones 1222.

One or more magnets 1224 (e.g., 12 magnets 1224) may be positioned in proximity to respective dedicated electrode lanes 1220 for retaining magnetically responsive beads. The positioning of magnets 1224 relative to dedicated electrode lanes 1220 provides "turn-off" areas for shuttling reaction droplets during certain reaction steps (e.g., bead removal steps). Each magnet 1224 may, for example, be a permanent magnet or an electromagnet. Each magnet 1224 may be a movable magnet that may be moved into proximity with and away from its respective dedicated electrode lane 1220. Each magnet 1224 is positioned in a manner which ensures spatial immobilization of nucleic acid-attached beads during washing between enzymatic reactions and bead removal following elution of processed nucleic acid. Mixing and incubations may be performed in dedicated electrode lanes 1220 away from the magnet.

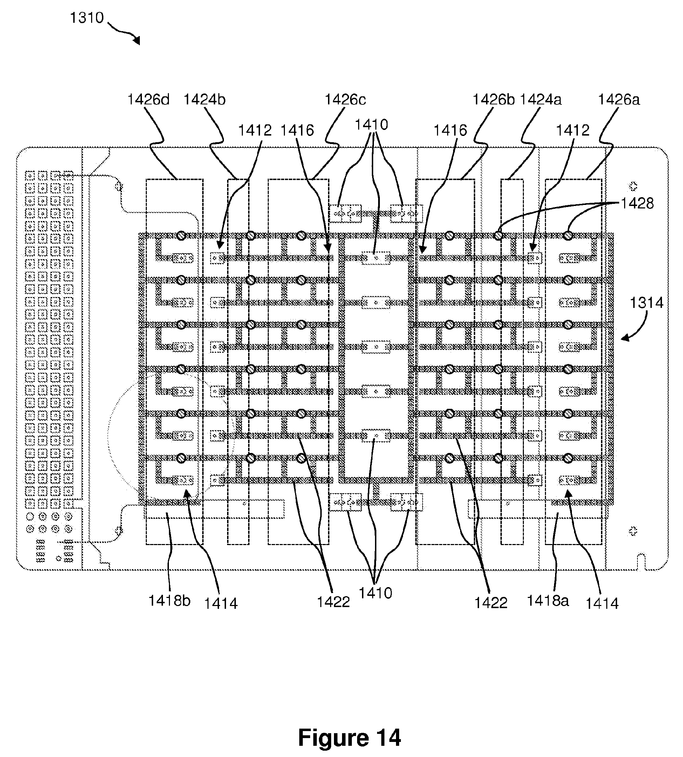

**Figures 13A and 13B** illustrate a top view and a perspective view, respectively, of an assembled droplet actuator 1300 suitable for use in conducting a multiplexed nucleic acid library construction protocol. Droplet actuator 1300 may include a bottom substrate 1310 and a top substrate 1312 that are separated by a droplet operations gap. Bottom substrate 1310 may, for example, be a PCB, plastic or silicon chip. Bottom substrate 1310 may include an electrode arrangement 1314 configured for processing of nucleic acid for construction of a nucleic acid library. More details of electrode arrangement 1314 are described with reference to Figure 14. Droplet operations are conducted atop electrode arrangement 1314 on a droplet operations surface. In a preferred embodiment, bottom substrate 1310 is a PCB that is about 0.03125 inches thick and fabricated without copper flood (i.e., no copper layer on the PCB between the electrodes), which reduces interference with magnetic fields used to control magnetically responsive beads in droplets in the droplet operations gap between bottom substrate 1310 and a top substrate 1312 and reduces transmission of thermal energy via the bottom substrate. In another embodiment, bottom substrate 1310 may be a PCB that is about 0.0625 inches thick.

Top substrate 1312 may, for example, be formed of a molded material, such as polycarbonate. Integrated into top substrate 1312 may be multiple fluid dispensing reservoirs. For example, top substrate 1312 may include one or more reagent dispensing reservoirs for dispensing different reagent fluids (e.g., blunt-ending reagents, A-tailing reagents, adapter ligation reagents, bead solutions, wash buffer, elution buffer). In one example, top substrate 1312 includes reagent dispensing reservoirs 1316A, 1316B, 1316C, and 1316D, along with reagent dispensing reservoirs 1317A, 1317B, 1317C, 1317D, and 1317E. Top substrate 1312 may also include one or more sample input reservoirs for dispensing sample fluids. For example, top substrate 1312 includes, a first row (or column) of sample input reservoirs 1318A through 1318F and a second row (or column) of sample input reservoirs 1318G through 1318L. Top substrate 1312 may also include one or more adapter dispensing reservoirs for dispensing adapter-barcode tags. For example, top substrate 1312 includes, a first row (or column) of adapter dispensing reservoirs 1320A through 1320F and a second row (or column) of adapter dispensing reservoirs 1320G through 1320L.

Top substrate 1312 may also include multiple collection reservoirs. For example, top substrate 1312 may include one or more sample output collection reservoirs 1322 for receiving processed sample fluids, e.g., two rows (or columns) of 6 sample output collection reservoirs 1322 for receiving processed nucleic acid droplets. Top substrate 1312 may also include one or more waste collection reservoirs 1324 for collecting waste fluids, e.g., waste collection reservoirs 1324A and 1324B.

Reagent dispensing reservoirs 1316, sample input reservoirs 1318, and adapter dispensing reservoirs 1320 are associated with openings or fluid paths that are arranged to flow fluid into proximity with fluid dispensing electrodes that are arranged on bottom substrate 1310, such as described in reference to Figure 14. Sample output collection reservoirs 1322 and waste collection reservoirs 1324 substantially are associated with openings or fluid paths that are arranged to flow fluid into sample output collection reservoirs 1322 and waste collection reservoirs 1324 from fluid collecting electrodes that are arranged on bottom substrate 1310, such as described in reference to Figure 14.

An area that is at least about 1 cm wide around the periphery of bottom substrate 1310 provides a bond area for bonding bottom substrate 1310 to top substrate 1312. Additionally, one or more "heat stakes" may be used for mechanically attaching bottom substrate 1310 to top substrate 1312. Heat staking (or thermoplastic staking) is a well-known method of mechanical bonding. In one example, the bond line may be about < 1 cm wide and about 425 microns high.

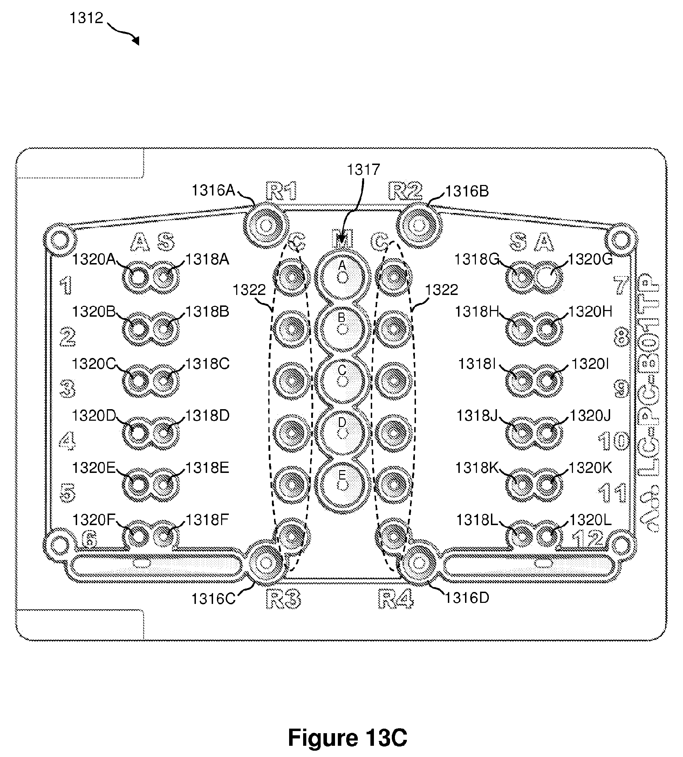

**Figure 13C** illustrates a top view of an example implementation of top substrate 1312 of droplet actuator 1300. The openings in top substrate 1312 are for pipette loading, as there are no top substrate reservoirs. This view shows the openings of reagent dispensing reservoirs 1316A, 1316B, 1316C, and 1316D; reagent dispensing reservoirs 1317A, 1317B, 1317C, 1317D, and 1317E; sample input reservoirs 1318A through 1318F; sample input reservoirs 1318G through 1318L; adapter dispensing reservoirs 1320A through 1320F, and adapter dispensing reservoirs 1320G through 1320L. Referring to Figures 13A, 13B, and 13C, examples of loading volumes and droplets for the reservoirs of droplet actuator 1300 are shown in Table 6.

| **Table 6.** Example loading volumes and droplets for the reservoirs of droplet actuator 1300 | | | |
|---|---|---|---|
| **Reservoir** | **Reagent Loading Volume (µL)** | **# Droplets Required (in terms of 1X)** | **Total Volume of Droplets (µL)** |
| 1316A | Blunt Ending Enzyme: 50 µL | 5 (one 1X per lane) | about 2 µL |
| 1316B | Beads: 100 µL | 60 (six 2X per lane) | about 20 µL |
| 1316C | A-Tailing Enzyme: 50 µL | 5 (one 1X per lane) | about 2 µL |
| 1316D | Ligase: 50 µL | 5 (one 1X per lane) | about 2 µL |
| 1317A | Elution Buffer: 165/-lL | 40 (eight 1X per lane) | about 14 µL |
| 1317B,1317C, 1317D,1317E | 70% Ethanol: 165 µL each | 120 (twelve 2X per lane) | about 40 µL |
| 1318G through 1318L | DNA on beads: 20 µL each | 10 each (five 2X per lane) | about 3.3 µL |
| 1320G through 1320L | 10x DNA adapters: 1.5 µL each | 1 each (one 1X per lane) | about .330 µL |

**Figure 14** illustrates a top view of an example of bottom substrate 1310 of droplet actuator 1300 of Figures 13A and 13B, which has electrode arrangement 1314 patterned thereon. In this example, reagent dispensing electrodes are positioned at a central portion of electrode arrangement 1314, flanked on each side by a set of 6 sample input dispensing electrodes and a set of 6 sample processing electrodes.

Electrode arrangement 1314 includes multiple dispensing electrodes. For example, electrode arrangement 1314 may include one or more reagent dispensing electrodes 1410, e.g., 9 reagent dispensing electrodes 1410, for dispensing different reagent fluids (e.g., blunt-ending reagents, A-tailing reagents, adapter ligation reagents, bead solutions, wash buffer, elution buffer). Electrode arrangement 1314 may also include one or more sample input dispensing electrodes 1412 for dispensing sample fluids (e.g., two rows (or columns) of 6 sample input dispensing electrodes 1412 for dispensing nucleic acid). Electrode arrangement 1314 may also include one or more adapter dispensing electrodes 1414 for dispensing adapter-barcode tags (e.g., two rows (or columns) of 6 adapter dispensing electrodes 1414).

Electrode arrangement 1314 may also include multiple collection electrodes. For example, Electrode arrangement 1314 may include one or more sample output collection electrodes 1416 for receiving processed sample fluids (e.g., two rows (or columns) of 6 sample output collection electrodes 1416 for receiving processed nucleic acid droplets). Electrode arrangement 1314 may also include one or more waste collection electrodes 1418 for collecting waste fluids (e.g., waste collection electrodes 1418a and 1418b). The reagent dispensing electrodes 1410, sample dispensing electrodes 1412, adapter dispensing electrodes 1414, sample output collection electrodes 1416, and waste collection electrodes 1418 are substantially aligned with the fluid reservoirs that are integrated into top substrate 1312 of droplet actuator 1300 of Figures 13A, 13B, and 13C.

Reagent dispensing electrodes 1410, sample input dispensing electrodes 1412, adapter dispensing electrodes 1414, sample output collection electrodes 1416, and waste collection electrodes 1418 are interconnected through an arrangement of droplet operations electrodes 1420 (e.g., electrowetting electrodes). For example, a path of droplet operations electrodes 1420 extending from each sample input dispensing electrode 1412 and its corresponding sample output collection electrode 1416 forms dedicated electrode lanes 1422, e.g., 13 dedicated electrode lanes 1422. Dedicated electrode lanes 1422 provide individual reaction lanes for processing different nucleic acid samples in a library construction protocol. The use of dedicated lanes for sample droplets and centralized reagent dispensing minimizes cross-contamination among different nucleic acids.

One or more bead immobilization zones 1424 (e.g., bead immobilization zones 1424a and 1424b) for performing certain process steps may be provided in relation to electrode arrangement 1314. For example, bead immobilization zones 1424 may be formed by an array of magnets, such as a Halbach array, that are located in sufficient proximity to the droplet actuator that the resultant magnetic field substantially restrains magnetically responsive beads in droplets in the droplet operations gap during droplet operations using such droplets. Magnetic fields at bead immobilization zones 1424, which are created by the magnets, may be used for retaining magnetically responsive beads. In one example, the arrangement of magnets may be a movable array that may be moved into proximity to and away from bead immobilization zone 1424, as described with reference to Figures 23A, 23B, and 24. The magnet array is positioned in a manner which ensures retention of magnetically responsive beads during certain process steps, such as sample concentration, washing between enzymatic reactions, and bead removal following elution of processed nucleic acid.

One or more temperature control zones 1426 (e.g., temperature control zones 1426a through 1426d) may be provided in the droplet actuator for performing certain process steps. For example, one or more heater elements (not shown), e.g., heater bars, may be provided in proximity to the assembled droplet actuator to create the one or more temperature control zones 1426 in the droplet operations gap. The heater bars may be used for thermal control of filler fluid that is in the gap of droplet actuator 1300 and heating or cooling droplets that are being transported along electrode paths of the droplet actuator through temperature control zones 1426. One or more heating pads 1428 may be used to control the flow of heat in temperature control zones 1426.

**Figure 15** illustrates a top view of an example of a bottom substrate 1500 of a droplet actuator that has an electrode arrangement 1510 patterned thereon for optimized droplet transporting and routing time. A droplet actuator may be formed using bottom substrate 1500, which may be a PCB, and an associated top substrate (not shown) that are separated by a gap. Electrode arrangement 1510 may be formed of various electrodes, such as reservoir electrodes and/or droplet operations electrodes.

A main aspect of electrode arrangement 1510 of the invention is that it includes, for example, two portions that are electrically independent of one another. For example, Figure 15 shows that electrode arrangement 1510 may be electrically partitioned into a portion A and a portion B. For example, droplet operations may occur in portion A of electrode arrangement 1510 in parallel with and independent of droplet operations occurring in portion B, and vice versa. Because droplet operations may occur in parallel and independently in portions A and B of electrode arrangement 1510, droplet transporting and routing time may be optimized. That is, current substrate designs may require sequential droplet transporting and routing operations. By contrast, the bottom substrate 1500 and electrode arrangement 1510 of the invention allows droplet transporting and routing time to be minimized because droplet operations may occur in parallel and independently in portions A and B.

In the example shown in Figure 15, when bottom substrate 1500 is assembled with a top substrate (not shown) to form a droplet actuator, electrode arrangement 1510 supports certain on-actuator fluid reservoirs of various capacities. For example, with respect to other fluid reservoirs of bottom substrate 1500, electrode arrangement 1510 may support multiple large-capacity fluid reservoirs 1512, such as eight large-capacity fluid reservoirs 1512 arranged in a line. In one example, the large-capacity fluid reservoirs 1512 may be used for holding sample fluid and/or waste fluid. With respect to other fluid reservoirs of bottom substrate 1500, electrode arrangement 1510 may also support multiple medium-capacity fluid reservoirs 1514, such as seven medium-capacity fluid reservoirs 1514 arranged in a line. In one example, the medium-capacity fluid reservoirs 1514 may be reagent reservoirs for holding wash reagents, elution buffer reagents, buffer reagents (for collecting droplets), enzyme reagents, certain bead-containing reagents, and the like. With respect to other fluid reservoirs of bottom substrate 1500, electrode arrangement 1510 may also support multiple small-capacity fluid reservoirs 1516, such as seven small-capacity fluid reservoirs 1516 arranged in a line. In one example, the small-capacity fluid reservoirs 1516 may be reagent reservoirs for holding certain enzyme reagents.

Additionally, electrode arrangement 1510 of bottom substrate 1500 may support other reservoirs, such as, but not limited to, a line of eight collection reservoirs 1518, a line of eight temporary storage reservoirs 1520, and a line of eight adapter reservoirs 1522. The temporary storage reservoirs 1520 are temporary liquid holding reservoirs. In one example, the temporary storage reservoirs 1520 are used for temporarily holding bead solution. In one example, the adapter reservoirs 1522 are used for holding adapter solution. More details of the on-actuator reservoirs that are supported by electrode arrangement 1510 are described with reference to Figures 16 through 21.

Various lines, paths, and/or arrays of droplet operations electrodes 1524 (e.g., electrowetting electrodes) are used to interconnect large-capacity fluid reservoirs 1512, medium-capacity fluid reservoirs 1514, small-capacity fluid reservoirs 1516, collection reservoirs 1518, temporary storage reservoirs 1520, and/or adapter reservoirs 1522. In one example, Figure 15 shows one or more mixing loops 1526 that are formed by certain arrangements of droplet operations electrodes 1524. For example, a line of eight mixing loops 1526 is implemented near respective large-capacity fluid reservoirs 1512. In another example, Figure 15 shows a distribution loop 1528 that is formed by another arrangement of droplet operations electrodes 1524. For example, distribution loop 1528 is implemented near the medium-capacity fluid reservoirs 1514 and small-capacity fluid reservoirs 1516. In one example, elution droplets may be processed at distribution loop 1528.

Generally, the collection reservoirs 1518, temporary storage reservoirs 1520, and adapter reservoirs 1522 are arranged between the eight mixing loops 1526 and the distribution loop 1528. The seven medium-capacity fluid reservoirs 1514 and seven small-capacity fluid reservoirs 1516 are feeding one side of distribution loop 1528. The eight large-capacity fluid reservoirs 1512 are feeding the eight mixing loops 1526, respectively.

Bottom substrate 1500 also includes certain input/output (I/O) pads 1530. I/O pads 1530 are contacts that are electrically connected to the electrodes by wiring traces in the PCB. In one example, I/O pads 1530 are used for applying electrowetting voltages to droplet operations electrodes 1524 and/or to any reservoir electrodes.

Additionally, Figure 15 shows that bottom substrate 1500 may include multiple temperature zones that are controlled by certain thermal control elements (not shown). In one example, a temperature zone 1540 is provided at the area of mixing loops 1526. This zone may be, for example, a designated reverse transcription (RT) zone. Additionally, a temperature zone 1542 is provided at the area of mixing loops 1526 and adapter reservoirs 1522. This zone may be, for example, a designated heating zone. Further, a temperature zone 1544 is provided at the area of temporary storage reservoirs 1520 and distribution loop 1528. This zone may be, for example, a designated loading zone. In one example, temperature zone 1540 may be held at from about xx °C to about xx °C, temperature zone 1542 may be held at from about xx °C to about xx °C, and temperature zone 1544 may be held at from about xx °C to about xx °C.

With respect to portion A and portion B of electrode arrangement 1510, large-capacity fluid reservoirs 1512 and mixing loops 1526 are in portion A. Medium-capacity fluid reservoirs 1514, small-capacity fluid reservoirs 1516, collection reservoirs 1518, temporary storage reservoirs 1520, adapter reservoirs 1522, and distribution loop 1528 are in portion B.

In operation, in portion A of electrode arrangement 1510, enzymatic and/or binding reactions may occur. Mixing and/or sample concentration steps may occur in the large-capacity fluid reservoirs 1512, which may be holding sample fluid and/or waste fluid. Additionally, certain incubation operations (e.g., up to about 30 minutes of incubation time) may occur in portion A.

At the same time, in portion B of electrode arrangement 1510, certain droplets may be pre-dispensed from medium-capacity fluid reservoirs 1514 and small-capacity fluid reservoirs 1516, which are reagent reservoirs, then await processing at distribution loop 1528. Pre-dispensed droplets from medium-capacity fluid reservoirs 1514 and small-capacity fluid reservoirs 1516 minimizes the time it takes to put them into action (minimizes transporting and routing time).

Sometimes, it may be hard to maintain beads in suspension for long periods of time (e.g., 3 hours). Therefore, portion B of electrode arrangement 1510 may be used to pre-dispense a few droplets (e.g., 8 droplets) of beads from any of the reservoirs to temporary storage reservoirs 1520. The smaller volumes of beads are easier to keep in suspension. In one example, the collection reservoirs 1518 are used for collecting the processed sample, which is the final product of the assay protocol.

A top substrate (not shown) is arranged in relation to bottom substrate 1500 of Figure 15 to form a droplet actuator. **Figure 16** illustrates a top view of bottom substrate 1500 of Figure 15 in relation to openings in the top substrate (not shown) for filling the on-actuator fluid reservoirs supported by electrode arrangement 1510. For example, Figure 16 shows multiple openings 1550, one opening 1550 for each fluid reservoir. In one example, the openings 1550 for large-capacity fluid reservoirs 1512, medium-capacity fluid reservoirs 1514, and small-capacity fluid reservoirs 1516 may have a diameter of about 3 millimeters (mm). The openings 1550 for collection reservoirs 1518 and adapter reservoirs 1522 may have a diameter of about 2 mm. More details of large-capacity fluid reservoirs 1512, medium-capacity fluid reservoirs 1514, small-capacity fluid reservoirs 1516, collection reservoirs 1518, temporary storage reservoirs 1520, and adapter reservoirs 1522 are described with reference to Figures 17, 18, 19, 20, and 21. In one embodiment all operations may happen within a reservoir space. For example, all mixing and binding may occur within large-capacity fluid reservoirs 1512. For example, the front portions of large-capacity fluid reservoirs 1512 may be used to perform binding and elution. In such an embodiment, mixing loops 1526 in each sample lane are available to populate additional unit cells.

Figure 17 illustrates a side view and top view of a portion of bottom substrate 1500 that includes one large-capacity fluid reservoir 1512. Large-capacity fluid reservoir 1512 is formed of an arrangement of multiple individually controlled electrodes, which collectively form large-capacity fluid reservoir 1512 within electrode arrangement 1510 of Figure 15.

For example, along the center of large-capacity fluid reservoir 1512 may be four segmented reservoir electrodes 1560. These four segmented reservoir electrodes 1560 in the center may be flanked on each side by four smaller reservoir flanking electrodes 1562. This arrangement of individually controlled electrodes is arranged in relation to a path, line, and/or array of the droplet operations electrodes 1524 (e.g., electrowetting electrodes). Additionally, the line of droplet operations electrodes 1524 may be flanked on each side by one or more path flanking electrodes 1564. Further, a loading electrode 1566 may be arranged in relation to opening 1550 of large-capacity fluid reservoir 1512.

An aspect of the large-capacity fluid reservoir 1512 of the invention is that it is segmented into multiple individually controlled electrodes. A benefit of the segmented electrode design is that, using certain electrode activation sequences, complex mixing operations may occur atop the multiple reservoir electrodes in the large-capacity fluid reservoir 1512. Another benefit of the segmented electrode design is that the segmentation of the large-capacity fluid reservoir 1512 provides capability to handle different volumes of fluid. In one example, large-capacity fluid reservoir 1512 may handle a fluid volume ranging from about 20 microliters (µL) to about 130 µL. The loading electrode 1566 that is positioned substantially at opening 1550 is sized and shaped to hold the intended minimum volume of fluid (e.g., about 20 µL). In one example, the diameter of opening 1550 for large-capacity fluid reservoir 1512 is about 3 mm.

The side view in Figure 17 shows bottom substrate 1500 in relation to a top substrate 1570. There is a gap height H1 along droplet operations electrodes 1524. In this example, there is a step in the profile of top substrate 1570 so that a gap height H2 at the area of the multiple reservoir electrodes is greater than the gap height H1. There may be a taper in the profile of top substrate 1570 to facilitate the transition from gap height H2 to gap height H1. The change in gap height and the taper may be useful for pulling fluid back into the reservoir without activating any reservoir electrodes. In one example, H1 may be from about 50 to about 600 µm, or from about 200 to about 400 µm, or about 300 µm. In one example, H2 may be from about 1000 to about 5000 µm, or from about 2000 to about 3000 µm, or about 2800 µm.

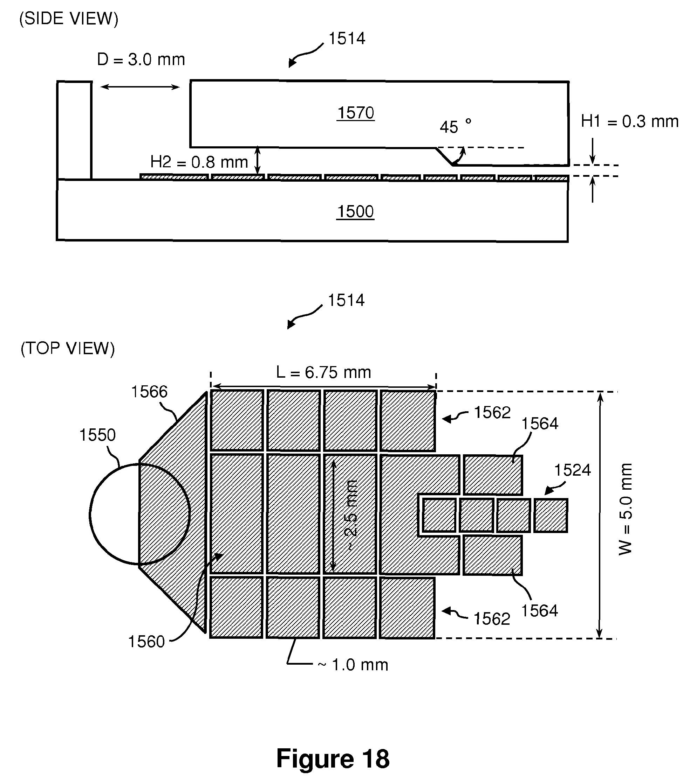

Figure 18 illustrates a side view and top view of a portion of bottom substrate 1500 that includes one medium-capacity fluid reservoir 1514. Medium-capacity fluid reservoir 1514 is also formed of an arrangement of multiple individually controlled electrodes, which collectively form medium-capacity fluid reservoir 1514 within electrode arrangement 1510 of Figure 15.

Although sized and/or shaped uniquely from those of large-capacity fluid reservoir 1512 of Figure 17, medium-capacity fluid reservoir 1514 also includes the segmented reservoir electrodes 1560, the reservoir flanking electrodes 1562, the droplet operations electrodes 1524, and the path flanking electrodes 1564. Medium-capacity fluid reservoir 1514 also includes the loading electrode 1566 in relation to opening 1550. The benefits (e.g., mixing capability and capability to handle different volumes of fluid) of the segmented electrode design of medium-capacity fluid reservoir 1514 are substantially the same as described with respect to large-capacity fluid reservoir 1512 of Figure 17.

In one example, medium-capacity fluid reservoir 1514 may handle a fluid volume ranging from about 8 µL to about 100 µL. The loading electrode 1566 that is positioned substantially at opening 1550 is sized and shaped to hold the intended minimum volume of fluid (e.g., about 8 µL). In one example, the diameter of opening 1550 for medium-capacity fluid reservoir 1514 is about 3 mm.

The side view in Figure 18 shows bottom substrate 1500 in relation to top substrate 1570. There is a gap height H1 along droplet operations electrodes 1524. In this example, there is a step in the profile of top substrate 1570 so that a gap height H2 at the area of the multiple reservoir electrodes is greater than the gap height H1. There may be a taper in the profile of top substrate 1570 to facilitate the transition from gap height H2 to gap height H1. The change in gap height and the taper may be useful for pulling fluid back into the reservoir without activating any reservoir electrodes. In one example, H1 may be from about 50 to about 600 µm, or from about 200 to about 400 µm, or about 300 µm. In one example, H2 may be from about 500 to about 1000 µm, or from about 700 to about 900 µm, or about 800 µm.

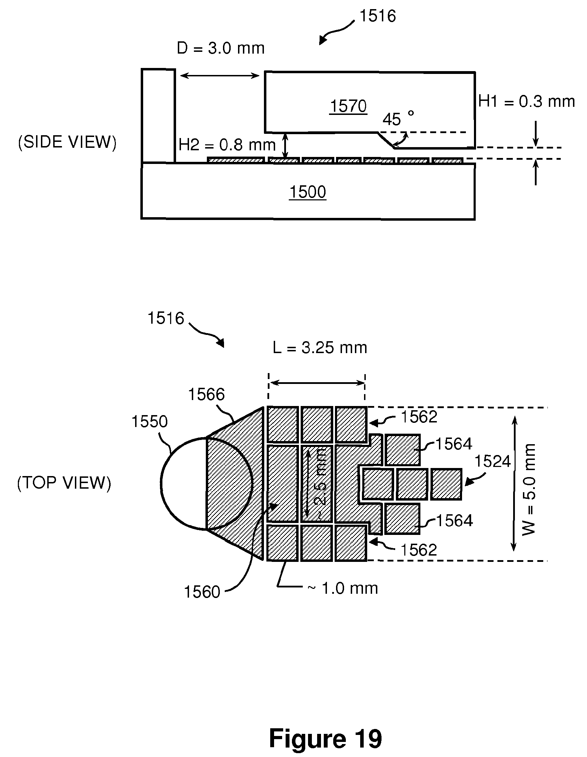

**Figure 19** illustrates a side view and top view of a portion of bottom substrate 1500 that includes one small-capacity fluid reservoir 1516. Small-capacity fluid reservoir 1516 is also formed of an arrangement of multiple individually controlled electrodes, which collectively form small-capacity fluid reservoir 1516 within electrode arrangement 1510 of Figure 15.

Although sized and/or shaped uniquely from those of large-capacity fluid reservoir 1512 of Figure 17, small-capacity fluid reservoir 1516 also includes the segmented reservoir electrodes 1560, the reservoir flanking electrodes 1562, the droplet operations electrodes 1524, and the path flanking electrodes 1564. Small-capacity fluid reservoir 1516 also includes the loading electrode 1566 in relation to opening 1550. The benefits (e.g., mixing capability and capability to handle different volumes of fluid) of the segmented electrode design of small-capacity fluid reservoir 1516 are substantially the same as described with respect to large-capacity fluid reservoir 1512 of Figure 17.

In one example, small-capacity fluid reservoir 1516 may handle a fluid volume ranging from about 6 µL to about 20 µL. The loading electrode 1566 that is positioned substantially at opening 1550 is sized and shaped to hold the intended minimum volume of fluid (e.g., about 6 µL). In one example, the diameter of opening 1550 for medium-capacity fluid reservoir 1514 is about 3 mm.

The side view in Figure 19 shows bottom substrate 1500 in relation to top substrate 1570. There is a gap height H1 along droplet operations electrodes 1524. In this example, there is a step in the profile of top substrate 1570 so that a gap height H2 at the area of the multiple reservoir electrodes is greater than the gap height H1. There may be a taper in the profile of top substrate 1570 to facilitate the transition from gap height H2 to gap height H1. The change in gap height and the taper may be useful for pulling fluid back into the reservoir without activating any reservoir electrodes. In one example, H1 may be from about 50 to about 600 µm, or from about 200 to about 400 µm, or about 300 µm. In one example, H2 may be from about 500 to about 1000 µm, or from about 700 to about 900 µm, or about 800 µm.

The path flanking electrodes 1564, which are positioned lateral to droplet operations electrodes 1524, function to increase electrowetting force to pull liquid from the reservoir to the dispensing area by increasing the electrowetting area. In this example, the misalignment of path flanking electrodes 1564 in relation to droplet operations electrodes 1524 helps liquid to advance to the next electrodes.

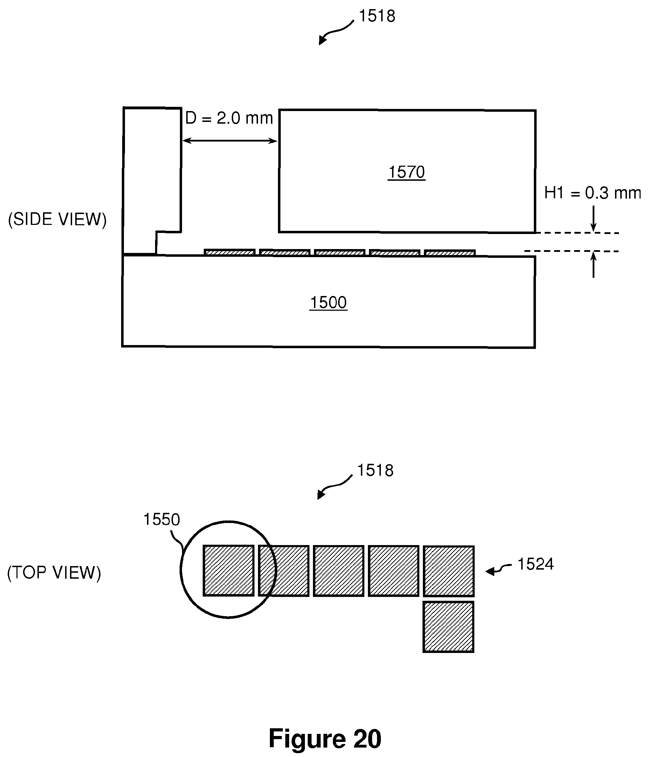

**Figure 20** illustrates a side view and top view of a portion of bottom substrate 1500 that includes one collection reservoir 1518. Collection reservoir 1518 is formed of an arrangement of droplet operations electrodes 1524. In one example, the diameter of opening 1550 for collection reservoir 1518 is about 2 mm. The side view in Figure 20 shows bottom substrate 1500 in relation to top substrate 1570. There is a gap height H1 along droplet operations electrodes 1524. In this example, there is no step in the profile of top substrate 1570. Therefore, there is a substantially uniform gap height H1 along the droplet operations electrodes 1524. In one example, H1 may be from about 50 to about 600 µm, or from about 200 to about 400 µm, or about 300 µm.

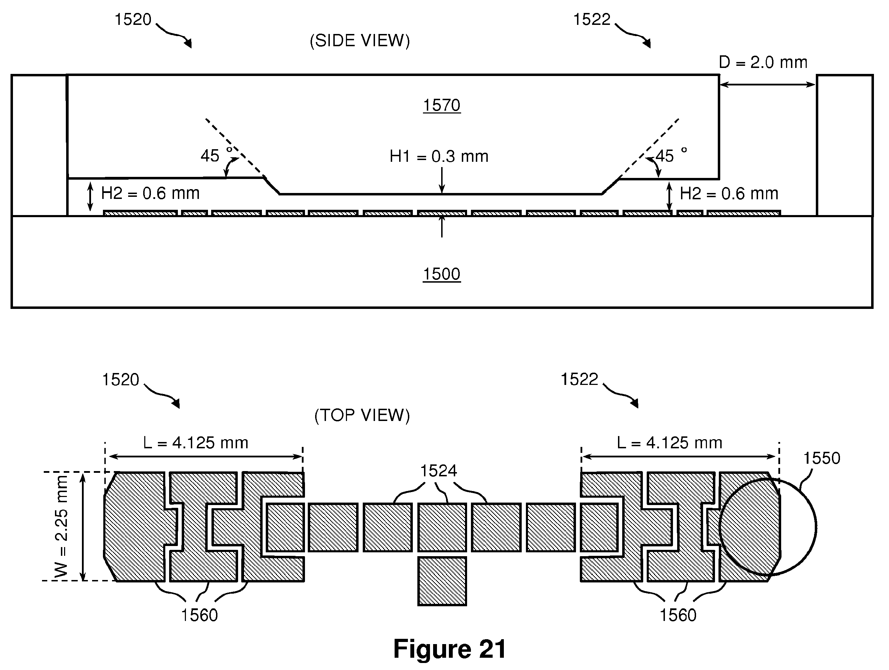

**Figure 21** illustrates a side view and top view of a portion of bottom substrate 1500 that includes one temporary storage reservoir 1520 and one adapter reservoir 1522. Temporary storage reservoir 1520 and adapter reservoir 1522 are also formed of an arrangement of multiple individually controlled electrodes, which collectively form temporary storage reservoir 1520 and adapter reservoir 1522 within electrode arrangement 1510 of Figure 15.

Although sized and/or shaped uniquely from those of large-capacity fluid reservoir 1512 of Figure 17, temporary storage reservoir 1520 and adapter reservoir 1522 also include the segmented reservoir electrodes 1560. In this example, the segmented reservoir electrodes 1560 may be, for example, T-shaped, Y-shaped, H-shaped, and/or any shape that ensures or, at least, helps the liquid contact the next electrodes for improved electrowetting. The benefits (e.g., mixing capability and capability to handle different volumes of fluid) of the segmented electrode design of temporary storage reservoir 1520 and adapter reservoir 1522 are substantially the same as described with respect to large-capacity fluid reservoir 1512 of Figure 17.

In one example, temporary storage reservoir 1520 and adapter reservoir 1522 may handle a fluid volume from about 1 µL to about 5.5 µL. Figure 17 also shows opening 1550 in relation to adapter reservoir 1522. In one example, the diameter of opening 1550 for adapter reservoir 1522 is about 2 mm. There is no opening 1550 associated with temporary storage reservoir 1520.

The side view in Figure 21 shows bottom substrate 1500 in relation to top substrate 1570. There is a gap height H1 along droplet operations electrodes 1524. In this example, there is a step in the profile of top substrate 1570 so that a gap height H2 at the area of the segmented reservoir electrodes 1560 is greater than the gap height H1. There may be a taper in the profile of top substrate 1570 to facilitate the transition from gap height H2 to gap height H1. The change in gap height and the taper may be useful for pulling fluid back into the reservoir without activating any reservoir electrodes. In one example, H1 may be from about 50 to about 600 µm, or from about 200 to about 400 µm, or about 300 µm. In one example, H2 at temporary storage reservoir 1520 is from about 500 to about 1000 µm, or from about 600 to about 700 µm, or about 625 µm; and H2 at adapter reservoir 1522 is from about 250 to about 750 µm, or from about 400 to about 500 µm, or about 425 µm.

Referring to Figures 15 through 21, off-actuator fluid reservoirs (not shown) that feed openings 1550 may be incorporated into the top substrate associated with bottom substrate 1500. Accordingly, the off-actuator fluid reservoirs are used on combination with the on-actuator fluid reservoirs, such as, but not limited to, large-capacity fluid reservoirs 1512, medium-capacity fluid reservoirs 1514, small-capacity fluid reservoirs 1516, collection reservoirs 1518, temporary storage reservoirs 1520, and adapter reservoirs 1522.

In another embodiment a method of conducting droplet operations in a droplet operations gap of an electrowetting device is provided. The method including providing a device with a dispensing region coated by an amorphous fluoropolymer, such as CYTOP® (available from Asahi Glass Co., Tokyo), dispensing an organic solvent in the dispensing region, and transporting the organic solvent into a region not coated with CYTOP®. The region not coated with CYTOP® may, for example, be coated with a fluorocarbon, such as polytetrafluoroethylene (e.g., TEFLON® coatings available from DuPont). The organic solvent may be any organic solvent, but is preferably a polar protic solvent. In one embodiment, the solvent is an alcohol and/or carboxylic acid. In another embodiment, the solvent is an alcohol and/or carboxylic acid having from 1 to 8 carbons, or from 1-6 carbons. The alcohol and/or carboxylic acid may be cyclic, linear, or branched. Examples include hexanol, pentanol, butanol, propanol, isopropanol, ethanol, methanol, formic acid, acetic acid, propionic acid, butyric acid, valeric acid.

The two substrates may be coupled together in any manner which leaves open a droplet operations gap between them. Typically, the droplet operations gap will be sealed around the perimeter, but this is not always necessary. Openings in the gap may be useful for introducing or removing liquids. For example substrates may be mechanically coupled together, and the droplet operations gap may be sealed around the perimeter with a gasket. Coupling and sealing of the droplet operations gap may, for example, be accomplished using an adhesive material. A PCB substrate may be bonded to a plastic top substrate using an adhesive, which also seals the perimeter of the droplet operations gap. A PCB substrate may be bonded to an acrylic top substrate using an adhesive which also seals the perimeter of the droplet operations gap. A PCB substrate may be bonded to a plastic top substrate using a urethane methacrylate polymer, which also seals the perimeter of the droplet operations gap. A PCB substrate may be bonded to an acrylic top substrate using a polymeric adhesive, which also seals the perimeter of the droplet operations gap. A PCB substrate may be bonded to an acrylic top substrate using a urethane methacrylate polymer, which also seals the perimeter of the droplet operations gap. The urethane methacrylate polymer may, for example, include PERMABOND® UV648 or UV632 UV-curable adhesive. A PCB substrate may be bonded to a glass top substrate using a polymeric adhesive, which also seals the perimeter of the droplet operations gap. A PCB substrate may be bonded to a glass top substrate using a UV curable epoxy resin based polymer, which also seals the perimeter of the droplet operations gap. Examples of suitable UV curable epoxy resin based polymers include those available from Master Bond, Inc., Hackensack, NJ (e.g., MASTER BOND® UV15x-5). Where a polymeric adhesive is used, the polymer may be deposited in a bead line around the perimeter of the bottom or top substrate, followed by positioning of the other surface (PCB or plastic). UV-curable polymers may then be exposed to UV light for UV curing. In certain embodiments, the adhesive seals the perimeter of the droplet operations gap between the bottom and top substrate. The gap may be substantially filled with a filler fluid, such as silicone oil.

In another embodiment, the top and bottom substrates are bonded by an adhesive tape, such as a tape coated with an adhesive, such as an acrylic adhesive. The tape can be cut to a shape suitable for sealing some portion or all of the perimeter of the droplet operations gap. Examples of suitable adhesive tapes include cloth tapes, polyethylene foam tapes, urethane tapes, paper tapes, polyester tapes, tissue tapes, and vinyl tapes. Preferred examples include 3M™ Adhesive Transfer Tape with 300LSE Adhesive: 9453FL, 9471FL, 9472FL; and 3M™ VHB™ Tapes. These and other suitable tapes are available from Can-Do National Tape Co., Nashville, TN, and other suppliers. In certain embodiments, the tape seals the perimeter of the droplet operations gap between the bottom and top substrate. The gap may be substantially filled with a filler fluid, such as silicone oil.

In one embodiment a bottom substrate, made of PCB material for example may be bonded to a plastic top substrate using a urethane methacrylate polymer, such as PERMABOND® UV648 UV-curable adhesive to form a structure. The polymer may be deposited in a bead line around the perimeter of the bottom or top substrate, followed by positioning of the other surface (PCB or plastic) and UV curing. In another embodiment, the urethane methacrylate polymer forms an enclosure between the bottom and top substrate and the enclosure may include an oil, such as a silicone oil. In another embodiment, the structure further includes electrodes on one or more surfaces of the substrates and are arranged to conduct droplet operations, such as electrowetting and/or dielectrophoresis mediated droplet operations. In still another embodiment the structure includes a microfluidics device.

The inventors have discovered that organic solvent wash droplets are difficult to reliably dispense on the droplet actuator, e.g., using electrowetting dispensing techniques. Among the solutions for improving such dispensing are the filler fluid formulations and wash droplet formulations described herein. Another solution, which may be used separately or together with the filler fluid and droplet formulation solutions, arises out of the discovery that organic solvents dispense more reliably on surfaces coated by amorphous fluoropolymers, such as CYTOP® coatings (available from (available from Asahi Glass Co., Tokyo). In one embodiment, droplet actuator surfaces contacted by an organic droplet during dispensing (e.g., surfaces of top and/or bottom substrates facing a droplet operations gap in a droplet dispensing region) are coated with an amorphous fluoropolymer, while other regions are coated with a non-fluoropolymer coating. In another embodiment, droplet actuator surfaces contacted by an organic droplet during dispensing are coated with a CYTOP® fluoropolymer coating, while other regions are coated with a separate fluoropolymer coating. Similarly, the invention comprises dispensing an organic solvent droplet using an electrowetting dispensing technique in a dispensing region coated with an amorphous fluoropolymer, and transporting the dispensed organic solvent droplet into a region not coated with the amorphous fluoropolymer. Similarly, the invention comprises dispensing an organic solvent droplet using an electrowetting dispensing technique in a dispensing region coated with CYTOP® amorphous fluoropolymer, and transporting the dispensed organic solvent droplet into a region not coated with the CYTOP® amorphous fluoropolymer. Similarly, the invention comprises dispensing an organic solvent droplet using an electrowetting dispensing technique in a dispensing region coated with CYTOP® amorphous fluoropolymer, and transporting the dispensed organic solvent droplet into a region coated with a TEFLON® amorphous fluoropolymer. TEFLON® polytetrafluoroethylene coatings are available from E.I. DuPont de Nemours & Co., Inc., Wilmington, DE.

### 7.4 Magnet Arrays

The droplet actuator may include or be associated with an array of magnets. For example, the droplet actuator may be mounted on an instrument deck is designed to fit onto an instrument deck that houses additional features, such as magnets and temperature control devices, such as heaters of heat sinks, for controlling the temperature within certain processing zones on the droplet actuator. Magnets may be used for immobilization of magnetically responsive beads in the droplet actuator. Magnets may be used for immobilization of magnetically responsive beads in droplets subject to droplet operations in the droplet actuator. Magnets may be used for immobilization of magnetically responsive beads in reservoirs on the droplet actuator, such as reservoirs formed in a top substrate of the droplet actuator assembly. Magnets may be used to manipulate magnetically responsive beads for droplet operations required for various processing steps in a nucleic acid library construction protocol, such as immobilization of beads during a bead washing step. Magnets may be used to manipulate magnetically responsive beads for droplet operations required for various processing steps in a nucleic acid library construction protocol, such as immobilization of beads during a droplet splitting operation in order to retain all or substantially all of the beads in one of the daughter droplets.

In some embodiments, the invention provides for movable magnets. In one example, a movable magnet may be positioned in a manner which ensures spatial immobilization of nucleic acid-attached beads during sample concentration. Sample concentration may, for example, be performed using a single step bead concentration protocol. Sample concentration may also be performed using a serial dispensing-bead concentration protocol. In another example, a movable magnet may be positioned in a manner which ensures spatial immobilization of nucleic acid-attached beads during bead washing between enzymatic reactions. In another example, a movable magnet may be positioned in a manner which ensures spatial immobilization of beads during bead removal following elution of processed nucleic acid. The sequence of droplet manipulations (electrowetting program), temperature control, and magnet position may, for example, be programmed using adjustable software and a programmable software interface.

In one example, an instrument deck may include one or more movable magnet arrays (e.g., Halbach arrays) and one or more heater assemblies (e.g., heater bars) positioned to align with certain processing zones on a droplet actuator, as well as electrical connections for controlling electrode activation and deactivation, and other electrical functions of the droplet actuator (e.g., sensors). This example is described with reference to Figures 22A, 22B, and 23.

**Figures 22A and 22B** illustrate perspective views of a magnet actuator 2200. Magnet actuator 2200 may include a frame structure 2210 that has a slidable plate 2212 installed therein for holding a set of cube magnets 2214. A solenoid 2216 is fixed to frame structure 2210. The position of slidable plate 2212 relative to frame structure 2210 is controlled by solenoid 2216. That is, the actuator of solenoid 2216 presses against a portion of slidable plate 2212 to move cube magnets 2214 relative to frame structure 2210. Certain mechanisms, such as adjustable set screws 2218, may be provided for controlling the linear travel of slidable plate 2212 and cube magnets 2214. Slidable plate 2122 may be mated with frame 2210 components, e.g., via groove and slot configuration, so that the motion of plate 2122 is constrained within certain bounds to achieve the functions described herein.

In operation, cube magnets 2214 that are on slidable plate 2212 are positioned in proximity to a droplet actuator, e.g., a droplet actuator 2220. Solenoid 2216 is used to move cube magnets 2214 close to or away from the surface of the droplet actuator. Figure 22A shows solenoid 2216 in a de-energized state and slidable plate 2212 with cube magnets 2214 in a disengaged state with respect to droplet actuator 2220. Figure 22B shows solenoid 2216 in an energized state and slidable plate 2212 with cube magnets 2214 in an engaged state with respect to droplet actuator 2220.

**Figure 23** illustrates a top view of an example of a mechanical fixture 2300 for holding one or more magnet actuators and one or more heater mechanisms. Mechanical fixture 2300 is suitable for use in processing of nucleic acid on a droplet actuator for construction of a nucleic acid library. In one example, mechanical fixture 2300 includes two magnet actuators 2200, such as a magnet actuator 2200a that includes a line of cube magnets 2214a and a magnet actuator 2200b that includes a line of cube magnets 2214b. Each set or line of cube magnets 2214 may, for example, be a Halbach array, as described with reference to Figures 24A, 24B, 25A, and 25B. Cube magnets 2214 may be moved into and out of proximity to a droplet actuator (not shown) by the action of magnet actuators 2200. In one example, cube magnets 2214 may be 2.25 mm cube magnets. In another example, cube magnets 2214 may be 4.5 mm cube magnets.

Mechanical fixture 2300 may also include one or more heaters 2310, such as heaters 2310a through 2310d. Heaters 2310 may, for example, be heater bars. Heaters 2310 may be used to control the temperature within designated temperature control zones of a droplet actuator. For example, one temperature control zone may provide 37 °C for A-tailing reactions or appropriate temperatures for performing 3-temperature PCR. A controller (not shown) may be used to control the output temperatures of heaters 2310.

In another embodiment, mechanical fixture 2300 may also include a cooling device, such as a heat sink or thermoelectric cooling device, such as a Peltier device. The cooling device may be used for maintaining a temperature in a region of the droplet actuator that is lower than ambient temperature. The cooling device may be used for preventing excessive heating in a region of the droplet actuator during heating of other regions of the droplet actuator. Using a combination of heaters and cooling devices, a desired thermal profile on the droplet actuator can be achieved, e.g., for performing 3-temperature PCR reactions. A controller (not shown) may be used to control heaters 2310 and the Peltier device.

Figure 23 also shows a clip 2320 for holding a droplet actuator in place on the assembly. Any type of restraining element would be suitable, so long as it is arranged to hold the droplet actuator assembly firmly in place during operation without damaging the droplet actuator assembly or otherwise interfering with its operation.

Magnets may be arranged to reinforce the magnetic field in regions of the droplet actuator in which bead immobilization is desired. Magnets may be arranged to cancel out or diminish the magnetic field in regions of the droplet actuator in which the magnetic field would otherwise interfere with desired operations. Magnets may be arranged to create a flux distribution in which the magnetic field is reinforced in regions of the droplet actuator in which bead immobilization is desired and diminished in regions of the droplet actuator in which the magnetic field would otherwise interfere with desired operations.

**Figures 24A and 24B** illustrate a perspective view of examples of a Halbach magnet array 2400. In one example and referring to Figure 24A, Halbach magnet array 2400 may include multiple cube magnets 2410, such as cube magnets 2410a through 2410e. The orientation of the magnetic field of each cube magnet 2410 is indicated by an arrow. The arrangement of cube magnets 2410 is such that the magnetic field on one side of the array is enhanced while the magnetic field on the other side or the array is cancelled to near zero. The array may be repeated any number of times to provide a magnet array of any length.

Figure 24B illustrates a Halbach magnet array 2400 with one or more posts which serve as focusing magnets 2412. Focusing magnets 2412 are used to further focus the magnetic field at a certain location of Halbach magnet array 2400. For example, Halbach magnet array 2400 includes focusing magnets 2412a and 2412b. Again, the orientation of the magnetic field of each focusing magnet 2412 is indicated by an arrow.

**Figures 25A** **and** **25B** illustrate the relationship of the magnetic fields of, for example, the Halbach magnet array 2400 of Figures 24A and 24B to the electrodes of a droplet actuator 2500. Droplet actuator 2500 may include a bottom substrate 2510 and a top substrate 2512 that are separated by a gap 2514. Bottom substrate 2510 may include an electrode arrangement, such as a path or array of droplet operations electrodes 2516 (e.g., electrowetting electrodes). Droplet operations are conducted atop droplet operations electrodes 2516 on a droplet operations surface.

In this example, Halbach magnet array 2400 is positioned below droplet actuator 2500. Halbach magnet array 2400 may include multiple cube magnets 2410a through 2410n. In Figures 25A and 25B, a representation of the magnetic field lines created by cube magnets 2410 is overlaid atop Halbach magnet array 2400 and droplet actuator 2500. Cube magnets 2410 may, for example, be 4.5 mm cube magnets.

The magnetic field lines in Figures 25A and 25B show that the magnetic field is concentrated at every other cube magnet 2410 (e.g., at cube magnets 2410a, 2410c, 2410e, 2410g, 2410i, 2410k, 2410m). In a preferred embodiment, these concentrated-field regions are substantially aligned with periodic electrode lanes that are arranged, for example, on about a 4.5 mm pitch for 4.5 mm cube magnets 2410. In this example, the concentrated-field regions are at about every 9 mm. In other example, when 2.25 mm cube magnets 2410 are used the concentrated-field regions are at about every 4.5 mm.

**Figures 26A and 26B** illustrate another view (i.e., a top view) of droplet actuator 2500 of Figures 25A and 25B in relation to Halbach magnet array 2400 of Figures 24A and 24B. For example, Figures 26A and 26B show cube magnets 2410 of Halbach magnet array 2400 in relation to certain lines of droplet operations electrodes 2516. Figure 26A shows Halbach magnet array 2400 without focusing magnets 2412, while Figure 26B shows Halbach magnet array 2400 with focusing magnets 2412. The alignment of Halbach magnet array 2400 is such that the centers of cube magnets 2410 are positioned at about the lines of droplet operations electrodes 2516. In one application, Halbach magnet array 2400 is positioned in a manner which ensures spatial immobilization of magnetically responsive beads during certain process steps, such as during sample concentration. Sample concentration may, for example, be performed using a single step bead concentration protocol. Sample concentration may also be performed using a serial dispensing-bead concentration protocol. An example of a sample concentration process is described with reference to Figure 27.

**Figure 27** illustrates a flow diagram of a method 2700 of sample concentration in a droplet actuator. For example, method 2700 may utilize one or more magnet actuators 2200 of Figures 22A, 22B, and 23 to provide moveable magnets in relation to a droplet actuator. Method 2700 begins with a sample volume of up to 50 µL, which is off-actuator, the sample being about 50-100 nanograms of nucleic acid. Then, while still off-actuator, the sample is mixed with beads that are in a binding buffer solution (50 µL). The result is a 100 µL of fluid that is then incubated off-actuator for some period of time (e.g., about 10 minutes). Following the incubation period, the 100 µL of fluid is loaded into the droplet actuator. Then, the magnets (e.g., cube magnets 2214 of one or more magnet actuators 2200) are moved in close proximity to the sample fluid. As a result, substantially all beads are pulled out of the 100 µL sample fluid and concentrated in the magnetic fields of cube magnets 2214. The beads are then washed and eluted. Nucleic acid is eluted the droplet may be snapped off the beads. A buffer droplet may be used to pick up the beads and carry them away, e.g., to a waste reservoir or an unused region of the electrode array.

### 7.5 Roller Assembly for Use With Droplet Actuators

The roller assembly of the invention is designed to fit onto an instrument deck that houses certain components that may be useful with respect to droplet actuators, such as magnets and heaters. Because the droplet actuator of the invention may be adapted for use with a number of different next-generation sequencing platforms, different arrangements of instrument components may be required for different library construction protocols.

**Figures 28A through 28D** illustrate a side view and cross-sectional views of a roller assembly 2800 that includes an arrangement of other components that may be useful with respect to droplet actuators. Referring to Figure 28A, roller assembly 2800 may include a roller body 2810. Roller body 2810 may, for example, be cylindrical in shape. Roller body 2810 may be connected to an instrument (not shown) by a mounting bar (or axle) 2812. Rotation (e.g., clockwise or counterclockwise) of roller assembly 2800 may be controlled by a motor (not shown), such as a stepper motor. Referring to Figures 28A and 28B, roller body 2810 may include one or more slots 2814, such as slots 2814a through 2814d, that may contain one or more components, such as, but not limited to, magnets, temperature control devices, and sonication devices. In one embodiment, magnets 2816a through 2816d may be positioned in slots 2814a through 2814d, respectively. Each magnet 2816 may be a permanent magnet or an electromagnet. In one example, each magnet 2816 may be a bar magnet. In another example, each magnet 2816 may be multiple smaller magnets (not shown). In another example, magnets 2816 may have different magnetic strengths. A single roller assembly 2800 may include multiple sets of magnets having different magnets, magnet strengths, arrangements of magnets, and the like.

Referring to Figures 28C and 28D, roller assembly 2800 may be positioned in proximity to a droplet actuator 2818. Droplet actuator 2818 may include a bottom substrate 2820. Bottom substrate 2820 may include an arrangement of droplet operations electrodes 2822 (e.g., electrowetting electrodes). Droplet operations are conducted atop droplet operations electrodes 2822 on a droplet operations surface. In one example, roller assembly 2800 may be positioned such that slot 2814a and magnet 2816a are aligned with certain droplet operations electrodes 2822.

In operation, a droplet 2824 that includes magnetically responsive beads 2826 may be positioned on a certain droplet operations electrode 2822 and aligned with magnet 2816a of roller assembly 2800. Because of the magnetic force of magnet 2816a, magnetically responsive beads 2826 are held at the surface of droplet operations electrode 2822. Roller assembly 2800 may be rotated, e.g., counterclockwise, such that magnet 2816a is moved away from droplet 2824. As roller assembly 2800 and magnet 2816a are rotated away from droplet 2824, magnetically responsive beads 2826 are resuspended within droplet 2824 because of the reduction of the magnetic force of magnet 2816a. In one example, roller assembly 2800 may rotated and stopped such that droplet 2824 is positioned between magnet 2816a and 2816b. In this position, droplet 2824 may be outside the magnetic field of magnets 2816a and 2816b (i.e., the magnetic fields of magnets 2816a and 2816b are essentially "turned off").

In another embodiment, one or more magnets 2816 in slots 2814 may be replaced by heater assemblies or cooling assemblies (e.g., thermoelectric cooler or Peltier chip) for controlling the temperature within certain reaction and/or washing zones. In another embodiment, one or more magnets 2816 in slots 2814 may be replaced by a sonication device, such as a sonicator used for cell disruption and nucleic acid shearing. In another embodiment, any combination of magnets, heaters, coolers, sonicators, and other components may be used in roller assembly 2800. Because the various components may be moved into and out of position relative to certain droplet operations regions by rotating roller assembly 2800, the same area on the droplet actuator may be used for multiple different processes in a digital microfluidic assay. In another embodiment, different roller assemblies 2800 may be provided with specialized component arrangements that are matched to specific assay requirements and/or actuator sizes and architectures. The design of roller assembly 2800 is such that one roller assembly 2800 may be readily removed from the instrument and a different roller assembly 2800 readily inserted into the instrument.

### 7.6 Disposal of Waste Droplets

**Figures 29A and 29B** illustrate a top view and a cross-sectional view, respectively, of an example of a portion of a droplet actuator 2900 and show a process of dumping droplets to waste. Droplet actuator 2900 may include a bottom substrate 2910 and a top substrate 2912 that are separated by a gap 2914. Gap 2914 has a height h1. Bottom substrate 2910 may, for example, be a PCB. Top substrate 2912 may, for example, be formed of glass, injection-molded plastic, silicon, and/or ITO. Droplet actuator 2900 may include an arrangement of droplet operations electrodes 2916 (e.g., electrowetting electrodes). Droplet operations are conducted atop droplet operations electrodes 2916 on a droplet operations surface.

One aspect of the invention includes a recessed area in the top and/or bottom substrate and adjacent to the droplet operations electrodes for dumping droplets. In one example, Figures 29A and 29B show that top substrate 2912 further includes a recessed area 2918. The cavity between bottom substrate 2910 and top substrate 2912 that is created by recessed area 2918 has a height h2 that is greater than the height h1 of gap 2914. The arrangement may be configured such that the droplet enters the recessed area as a result of displacement caused by deformation of the droplet to a more energetically stable conformation in the region of greater gap height.

In operation, as long as certain droplet operations electrodes 2916 are turned ON, droplets stay on the electrode path. However, when no droplet operations electrodes 2916 are turned ON, the energetically stable position for the droplet is in the adjacent recessed area. That is, the droplet wicks into this space because of capillary forces. Thus droplets can be dumped off the electrode path when they are no longer needed.

By way of example, Figures 29A and 29B show certain droplets 2920 sitting atop certain droplet operations electrodes 2916 when the electrodes are turned ON. Figures 29A and 29B also show a droplet 2922 that has been dumped off the path and into recessed area 2918.

The shape of recessed area 2918 may, for example, be a stair step or a slope, which helps keep the dumped droplets at a position that is sufficiently distant from the electrode path so that the dumped droplets do not interfere with the electrode path. Similarly, if the droplet too readily moves into the recessed region, a ridge or other obstacle may be included on the top and/or bottom substrate to slow droplet movement. Any transition region which permits the droplet to enter the recessed region as a result of displacement, while retaining the droplet on the electrode path in the presence of an activated electrode, will be suitable.

**Figure 30** illustrates a cross-sectional view of another embodiment of droplet actuator 2900 of Figures 29A and 29B. In this embodiment, recessed area 2918 is open (e.g., an opening 2930 in top substrate 2912). Figure 30 also shows filler fluid 2932 in droplet actuator 2900. Because of the presence of opening 2930 in top substrate 2912, droplets can be recovered, e.g., with a pipette or into another device or part of this device for capillary electrophoresis or other processing.

### 7.7 PCR Amplification and High-resolution Melting (HRM) analysis

HRM analysis may be used in combination with PCR amplification for detection of sequence variations (e.g., single-nucleotide polymorphisms, nucleotide-repeat polymorphisms, mutation scanning and assessment of nucleic acid methylation) within one or more genes of interest. The PCR amplicons may be fluorescently labeled during amplification using a saturating nucleic acid intercalating fluorescent dye, a 5'-labeled primer, or labeled probes. In various embodiments, the invention also provides for droplet actuator-based sample preparation and detection of sequence variations on the same droplet actuator.

In one embodiment, the droplet actuator device and methods of the invention may be used for preparation of nucleic acid, target PCR amplification and HRM analysis for genotyping Fragile X syndrome.

The digital microfluidic protocol for detection of sequence variations (e.g., polymorphisms, mutations, and methylation) within a gene of interest combines PCR amplification of target sequences and high-resolution melting (HRM) analysis of the target amplicons on a single droplet actuator. HRM analysis is based on the physical property of nucleic acid melting temperature for a double-stranded target sequence (i.e., amplicon) of a gene of interest. Each gene in an organism (individual) is typically present in two (or more) copies, i.e., two alleles. The alleles may be the same, i.e., homozygous, or different, i.e., heterozygous. During amplification of a nucleic acid sample, both alleles are amplified. As the amplified nucleic acid is denatured and cooled post-PCR for HRM analysis, different combinations of annealed double-stranded amplicons may be formed. Homozygous samples result in the formation of homoduplexes. Due to differences in sequence composition, different homozygous samples have different denaturation temperatures that result in different melt curves. Heterozygous samples contain two different alleles, which result in the formation of both homoduplexes (i.e., two homoduplex products) and heteroduplexes (i.e., two heteroduplex products). Heteroduplexes arise from the annealing of non-complementary strands of nucleic acid, which form, for example, during fast cooling of the sample. Because of the mis-paired regions in the heteroduplexes, the double-stranded amplicon is less stable and therefore dissociates at a lower temperature. The lower melting temperature produces a different melt curve profile. Because a different melt curve profile is produced, heterozygous samples may be differentiated from homozygous samples.

In the digital microfluidic protocol, rapid PCR thermocycling may be performed in a flow-through format where for each cycle the reaction droplets are cyclically transported between different temperature zones within the oil filled droplet actuator. Incorporation of a fluorescent label in the target amplicons may be used to monitor the PCR reaction and for subsequent HRM analysis. In one embodiment, target amplicons may be fluorescently labeled during PCR amplification using a saturating double-stranded nucleic acid intercalating dye such as LCGreen (available from Idaho Technology Inc, Salt Lake City, UT), EvaGreen (available from Biotium Inc, Hayward, CA), or SYTO 9 (available from Invitrogen™ by Life Technologies Corp, Carlsbad, CA). In another embodiment, a 5 -fluorescently labeled primer may be used to label the target amplicons. In another embodiment, fluorescently labeled probes may be used to label the target amplicons. Established PCR protocols that include optimum cycling parameters and concentration of reagents including Taq polymerase, buffers and primers (forward and reverse primers) may be selected for each gene of interest. For example, the sequence and length of the forward and reverse primers may be selected to produce amplicons of sufficient length for precise discrimination of alleles. The concentration of each primer, primer annealing temperature and magnesium concentration may be selected to provide specific amplification of the gene of interest with high yield. Annealing/extension time and number of thermocycles may be selected to provide high quality amplicons and rapid throughput in a PCR-HRM integrated protocol.

Established HRM protocols for allele discrimination may be adapted for use on a droplet actuator^{1,2} For example, prior to HRM analysis, the amplified nucleic acid is typically subjected to a final round of denaturation and annealing selected to enhance heteroduplex formation. The rate of denaturation and cooling may be selected for substantial formation of heteroduplexes. In one example, a higher heating rate (e.g., 0.4 °C/second) and a rapid cooling rate (e.g., about> 0.1 °C/second to about < 5 °C/seconds) may be selected to produce a higher number of heteroduplexes for more accurate discrimination of alleles. In another example, the ionic strength (e.g., a lower ionic strength) of the annealing buffer may be selected for substantial formation of heteroduplexes. Final HRM analysis may be performed on the duplexed nucleic acid amplicons using direct melting, i.e., precise warming of the nucleic acid amplicons from about 50 °C to about 95 °C at a selected temperature transition rate (e.g., 0.05 °C/second).

### 7.8 Droplet Dispensing Electrode Configurations

**Figure 31** illustrates top views of a portion of an example of an electrode arrangement 3100 and a reservoir dispensing sequence for dispensing 2X droplets. Electrode arrangement 3100 may include a dispensing electrode 3110 (of a fluid reservoir) that is segmented into multiple individually controlled electrodes. For example, along the center of dispensing electrode 3110 may be segmented reservoir electrodes 3112A, 3112B, 3112C, and 3112D. Smaller reservoir flanking electrodes 3114A, 3114B, 3114C, and 3114D may be arranged on one side of segmented reservoir electrodes 3112A, 3112B, 3112C, and 3112D. Smaller reservoir flanking electrodes 3114AA, 3114BB, 3114CC, and 3114DD may be arranged on the other side of segmented reservoir electrodes 3112A, 3112B, 3112C, and 3112D. Segmented reservoir electrode 3112D of dispensing electrode 3110 is arranged in relation to a path, line, and/or array of droplet operations electrodes (e.g., electrowetting electrodes); hereafter called path electrodes 3116. Additionally, a path flanking electrode 3118A may be arranged on one side of path electrodes 3116, while a path flanking electrode 3118B may be arranged on the other side of path electrodes 3116. Droplet operations are conducted atop these various electrodes on a droplet operations surface. Dispensing electrode 3110 that includes the multiple individually controlled electrodes supports a fluid reservoir that is designed to perform complex droplet mixing and/or droplet dispensing operations.

An aspect of the segmented dispensing electrode 3110 of the invention is that, when the reservoir is not fully filled, smaller volumes of fluid may be moved to the dispensing end of dispensing electrode 3110 for dispensing various sized droplets. Additionally, path flanking electrodes 3118, which are lateral to path electrodes 3116, may be activated to help pull the liquid out of dispensing electrode 3110 and onto the electrode path. Then, the path flanking electrodes 3118 are deactivated and, then, an intermediate electrode on the path is deactivated to yield the dispensed droplet. Examples of reservoir dispensing sequences are shown with reference to Figures 31, 32, and 33.

Referring again to Figure 31, an example of a reservoir dispensing sequence for dispensing 2X droplets may include, but is not limited to, the following steps.

At step 1, segmented reservoir electrodes 3112A, 3112B, and 3112C are deactivated; reservoir flanking electrodes 3114A, 3114B, 3114C, 3114AA, 3114BB, and 3114CC are deactivated; segmented reservoir electrode 3112D is activated; and reservoir flanking electrodes 3114D and 3114DD of are activated. In this way, a certain amount of fluid (not shown) may be pulled to the dispensing end of dispensing electrode 3110, which is the end closest to the line of path electrodes 3116.

At step 2, segmented reservoir electrodes 3112A, 3112B, and 3112C remain deactivated; reservoir flanking electrodes 3114A, 3114B, 3114C, 3114AA, 3114BB, and 3114CC remain deactivated; segmented reservoir electrode 3112D remains activated; and reservoir flanking electrodes 3114D and 3114DD remain activated. Additionally, the first four path electrodes 3116 in the line are activated and both path flanking electrodes 3118A and 3118B are activated. As a result, the volume of fluid (not shown) may be pulled yet further onto the line of path electrodes 3116.

At step 3, segmented reservoir electrodes 3112A, 3112B, and 3112C remain deactivated; reservoir flanking electrodes 3114A, 3114B, 3114C, 3114AA, 3114BB, and 3114CC remain deactivated; segmented reservoir electrode 3112D remains activated; and reservoir flanking electrodes 3114D and 3114DD remain activated. The first four path electrodes 3116 in the line remain activated. Both path flanking electrodes 3118A and 3118B remain activated. Additionally, the next (fifth) path electrode 3116 in the line is activated, pulling the volume of fluid (not shown) yet further onto the line of path electrodes 3116.

At step 4, all reservoir flanking electrodes 3114 are deactivated; all segmented reservoir electrodes 3112 are deactivated except segmented reservoir electrode 3112D; and path flanking electrodes 3118A and 3118B are also deactivated. Leaving only segmented reservoir electrode 3112D and the five path electrodes 3116 activated. This causes the volume of fluid to be concentrated at segmented reservoir electrode 3112D of dispensing electrode 3110 and along the line of five path electrodes 3116.

At step 5, segmented reservoir electrodes 3112A and 3112B are deactivated; reservoir flanking electrodes 3114A, 3114B, 3114AA, and 3114BB are deactivated; segmented reservoir electrodes 3112C and 3112D are activated; and flanking electrodes 3114C, 3114D, 3114CC, and 3114DD are activated. Additionally, path flanking electrodes 3118A and 3118B remain deactivated. Further, the third path electrode 3116 in the line is deactivated, while the first, second, fourth, and fifth path electrodes 3116 remain activated. As a result, a droplet splitting operation occurs because one of the intermediate path electrodes 3116 is turned off, leaving a 2X droplet (not shown) atop, for example, the fourth and fifth path electrodes 3116.

**Figure 32** illustrates top views of electrode arrangement 3100 of Figure 31 and a reservoir dispensing sequence for dispensing 1X droplets. An example of a reservoir dispensing sequence for dispensing 1X droplets may include, but is not limited to, the following steps.

At step 1, segmented reservoir electrodes 3112A, 3112B, and 3112C are deactivated; reservoir flanking electrodes 3114A, 3114B, 3114C, 3114AA, 3114BB, and 3114CC are deactivated; segmented reservoir electrode 3112D is activated; and reservoir flanking electrodes 3114D and 3114DD of are activated. In this way, a certain amount of fluid (not shown) may be pulled to the dispensing end of dispensing electrode 3110, which is the end closest to the line of path electrodes 3116.

At step 2, segmented reservoir electrodes 3112A, 3112B, and 3112C remain deactivated; reservoir flanking electrodes 3114A, 3114B, 3114C, 3114AA, 3114BB, and 3114CC remain deactivated; segmented reservoir electrode 3112D remains activated; and reservoir flanking electrodes 3114D and 3114DD remain activated. Additionally, the first four path electrodes 3116 in the line are activated and both path flanking electrodes 3118A and 3118B are activated. Further, the fifth path electrode 3116 in the line is deactivated. As a result, the volume of fluid (not shown) may be pulled yet further onto the line of path electrodes 3116.

At step 3, all reservoir flanking electrodes 3114 are deactivated; all segmented reservoir electrodes 3112 are deactivated except segmented reservoir electrode 3112D; and path flanking electrodes 3118A and 3118B are also deactivated. Leaving only segmented reservoir electrode 3112D and the first four of five path electrodes 3116 activated. This causes the volume of fluid to be concentrated at segmented reservoir electrode 3112D of dispensing electrode 3110 and along the line of four path electrodes 3116.

At step 4, segmented reservoir electrodes 3112A and 3112B are deactivated; reservoir flanking electrodes 3114A, 3114B, 3114AA, and 3114BB are deactivated; segmented reservoir electrodes 3112C and 3112D are activated; and flanking electrodes 3114C, 3114D, 3114CC, and 3114DD are activated. Additionally, path flanking electrodes 3118A and 3118B are deactivated. The first, second, and fourth path electrode 3116 in the line remain activated. Further, the third path electrode 3116 in the line is deactivated, while the first, second, and fourth path electrodes 3116 in the line remain activated. As a result, a droplet splitting operation occurs because two of the intermediate path electrodes 3116 are turned off, leaving a 1X droplet (not shown) atop, for example, the fourth path electrode 3116.

**Figure 33** illustrates top views of another embodiment of electrode arrangement 3100 of Figure 31 and another reservoir dispensing sequence for dispensing 1X droplets. In this embodiment of electrode arrangement 3100, the size of path flanking electrodes 3118A and 3118B and the position of path electrodes 3116 with respect to segmented reservoir electrode 3112D of dispensing electrode 3110 are slightly modified, as shown. More particularly, the misalignment of path flanking electrodes 3118A and 3118B in relation to path electrodes 3116 helps liquid to advance to the next electrodes. Accordingly, another example of a reservoir dispensing sequence for dispensing 1X droplets may include, but is not limited to, the following steps.

At step 1, segmented reservoir electrodes 3112A and 3112B are deactivated; reservoir flanking electrodes 3114A, 3114B, 3114AA, and 3114BB are deactivated; segmented reservoir electrodes 3112C, 3112D are activated; and flanking electrodes 3114C, 3114D, 3114CC, and 3114DD are activated. Additionally, path flanking electrodes 3118A and 3118B are deactivated. Further, the path electrodes 3116 are deactivated. In this way, a certain amount of fluid (not shown) may be pulled to the dispensing end of dispensing electrode 3110, which is the end closest to the line of path electrodes 3116.

At step 2, segmented reservoir electrodes 3112A, 3112B, and 3112C are deactivated; reservoir flanking electrodes 3114A, 3114B, 3114C, 3114AA, 3114BB, and 3114CC are deactivated; segmented reservoir electrode 3112D remains activated; and reservoir flanking electrodes 3114D and 3114DD remain activated. Additionally, the first two path electrodes 3116 in the line are activated and both path flanking electrodes 3118A and 3118B are activated. As a result, the volume of fluid (not shown) may be pulled yet further onto the line of path electrodes 3116.

At step 3, segmented reservoir electrodes 3112A, 3112B, and 3112C remain deactivated; reservoir flanking electrodes 3114A, 3114B, 3114C, 3114AA, 3114BB, and 3114CC remain deactivated; and segmented reservoir electrode 3112D remains activated. Reservoir flanking electrodes 3114D and 3114DD are now deactivated. Additionally, now the first three path electrodes 3116 in the line are activated. This causes fluid (not shown) to concentrate on the line of path electrodes 3116 as well as on segmented reservoir electrode 3112D and on reservoir flanking electrodes 3114D and 3114DD of dispensing electrode 3110.

At step 4, all reservoir flanking electrodes 3114 are deactivated; all segmented reservoir electrodes 3112 are deactivated except segmented reservoir electrode 3112D; and path flanking electrodes 3118A and 3118B are also deactivated. Leaving only segmented reservoir electrode 3112D and the first three path electrodes 3116 activated. This causes the volume of fluid to be concentrated at segmented reservoir electrode 3112D of dispensing electrode 3110 and along the first three path electrodes 3116.

At step 5, segmented reservoir electrodes 3112A and 3112B are deactivated; reservoir flanking electrodes 3114A, 3114B, 3114AA, and 3114BB are deactivated; segmented reservoir electrodes 3112C and 3112D are activated; and flanking electrodes 3114C, 3114D, 3114CC, and 3114DD are activated. Additionally, path flanking electrodes 3118A and 3118B remain deactivated. Further, the second path electrode 3116 in the line of path electrodes 3116 is deactivated, while the first and third path electrodes 3116 remain activated. As a result, a droplet splitting operation occurs because one of the intermediate path electrodes 3116 is turned off, leaving a 1X droplet (not shown) atop, for example, the third path electrode 3116.

**Figures 34A through 34E** illustrate top views of an example of a portion of an electrode arrangement 3400 of a droplet actuator and show a process of integrating PCR amplification and HRM analysis for allele discrimination on a droplet actuator. The method of the invention of Figures 34A through 34E is an example of an amplification and HRM analysis protocol wherein target amplicons may be fluorescently labeled during PCR amplification using a saturating double-stranded nucleic acid intercalating dye such as LCGreen. Intercalating dyes bind specifically to double-stranded nucleic acid. When the intercalating dye is bound to double-stranded nucleic acid, a fluorescent signal is produced. During HRM analysis, as the double-stranded nucleic acid is heated and the two strands of the nucleic acid melt apart, the presence of double stranded nucleic acid decreases and consequently the fluorescence signal is reduced. The rate of fluorescence decrease is generally greatest near the melting temperature (Tₘ) of the PCR product. The melting temperature is a function of PCR product characteristics, including GC-content (Tₘ is higher in GC-rich PCR products), length, and sequence content. The data may be acquired and plotted as a melt curve showing relative fluorescence versus temperature and/or derived melting peaks.

Electrode arrangement 3400 may include an arrangement of droplet operations electrodes 3410 that is configured for PCR amplification and HRM analysis. Droplet operations are conducted atop droplet operations electrodes 3410 on a droplet operations surface. Two temperature control zones 3412, such as temperature control zone 3412a and 3412b, may be associated with electrode arrange 3400. Thermal control elements (not shown) control the temperature of filler fluid (not shown) in the vicinity of temperature control zones 3412a and 3412b. For example, temperature control zone 3412a may be heated to about 95 °C, which is a temperature sufficient for denaturation of double-stranded nucleic acid. Temperature control zone 3412b may, for example, be heated to about 55 °C, which is a temperature sufficient for primer annealing and extension.

In one example, temperature control zones 3412a and 3412b may be used for PCR thermocycling. In another example, thermal conditions in temperature control zone 3412b may be adjusted for acquisition of a melting curve for HRM analysis. While two temperature control zones 3412 are shown, any number of temperature control zones 3412 may be associated with electrode arrangement 3410. A detection spot 3414 may be arranged in close proximity to droplet operations electrode 3410D within temperature control zone 3412b.

An example of a general process of PCR amplification and HRM analysis may include, but is not limited to, the following steps.

In one step, Figure 34A shows a sample droplet 3416 that is positioned at a certain droplet operations electrode 3410 within temperature control zone 3412a. Sample droplet 3416 may, for example, include nucleic acid template (nucleic acid target) for amplification. In one example, the nucleic acid template may include a variant region of interest for a particular gene. Because sample droplet 3416 is within temperature control zone 3412a, the nucleic acid template is denatured (single-stranded).

In other steps, Figure 34B and 34C show an incubation process in which a reagent droplet 3418 is merged using droplet operations with sample droplet 3416 within temperature control zone 3412a to yield a reaction droplet 3420. Reagent droplet 3418 may include primers and PCR reagents (e.g., dNTPs, buffers, DNA polymerase) for target amplification. Reagent droplet 3418 may also include a fluorescent saturating DNA intercalating dye such as LCGreen. Reaction droplet 3420 is transported using droplet operations to a certain droplet operations electrode 3410 within temperature control zone 3412b. Reaction droplet 3420 is incubated in temperature control zone 3412b for a period of time that is sufficient for primer annealing/extension and incorporation of the fluorescent intercalating dye. Reaction droplet 3420 may be repeatedly transported back and forth for any number of cycles using droplet operations between thermal reaction zones 3412b and 3412a for PCR amplification of target nucleic acid.

Referring to Figure 34C, reaction droplet 3420 may be transported using droplet operations to droplet operations electrode 3410D, which is within the range of detection spot 3414. An imaging device (e.g., fluorimeter, not shown), arranged in proximity with detection spot 3414, is used to capture and quantitate the amount of fluorescence in reaction droplet 3420. Amplified nucleic acid may be detected after any number of amplification cycles (i.e., real-time or end-point).

Figure 34D shows reaction droplet 3420 transported, after completion of PCR amplification, using droplet operations to a certain droplet operations electrode 3410 within temperature control zone 3412a. In this step, a final denaturation and cooling of the amplified nucleic acid within reaction droplet 3420 is performed to produce a high number of heteroduplexes for more accurate discrimination of alleles. In one example, the temperature within temperature control zone 3412a may be adjusted to provide a higher heating rate (e.g., 0.4 °C/second) and a rapid cooling rate (e.g., about>0.1 °C/second to about < 5 °C/seconds) that enhances heteroduplex formation.

Figure 34E shows reaction droplet 3420 transported using droplet operations to a droplet operations electrode 3410D within temperature control zone 3412b, which is within the range of detection spot 3414. In this step, HRM analysis is performed. In one example, the temperature within temperature control zone 3412b may be adjusted at a ramping rate of 0.2 °C/second from about 50 °C to about 95 °C. An imaging device (e.g., fluorimeter, not shown), arranged in proximity with detection spot 3414, is used to continuously capture and quantitate the amount of fluorescence in reaction droplet 3420 as the temperature is increased.

The invention provides integrated PCR amplification and HRM analysis methods for detection of Fragile X syndrome on a droplet actuator. Fragile X syndrome is associated with the expansion of a single CGG trinucleotide repeat in the 5'-untranslated region of the fragile X-mental retardation 1 (FMR1) gene on the X chromosome. The FMR1 protein encoded by this gene is required for normal neural development. Among people without the fragile X mutation, the number of CGG repeats varies from 6 to about 40. The fragile X mutation involves an expanded number of the CGG repeats. Expansions with from about 55 to about 200 CGG repeats, called permutations, are seen in unaffected carriers. About 40 to about 55 repeats is considered a "grey zone" where normal and permutation size ranges overlap. Expansions with more than 200 repeats, called full mutations, are associated with increased methylation of that region of the nucleic acid which effectively silences the expression of the FMR1 protein.

In one embodiment, the invention provides methods for a droplet-based integrated PCR amplification and HRM assay that correlates FRMlamplicon melting point with the length of the CGG repeat domain. The melting temperature of a nucleic acid molecule is dependent on both the length of the molecule and the specific nucleotide sequence composition of that molecule (e.g., a higher Tₘ is associated with a higher GC content). In one example, the PCR primers may be selected to amplify a region of the CGG repeat domain of the FRM1 alleles which have been shown to be associated with Fragile X syndrome. Primer pairs (forward and reverse primers) may be selected to produce amplicons of sufficient length for precise discrimination of alleles within the polymorphic CGG region. PCR amplification and HRM analysis may be performed as described in reference to Figure 1.

In another embodiment, the invention provides methods for a droplet-based integrated PCR amplification and HRM assay that correlates FRMlamplicon melting point with methylation of the FRM1 allele. Existing assays for fragile X syndrome based on detection of hypermethylated FMR1 alleles by methylation-specific melting curve analysis may be adapted for use on a droplet actuator³. In general, methylation-specific melting curve analysis uses sodium bisulfite treatment of isolated nucleic acid prior to PCR amplification. Bisulfite treatment is used to convert unmethylated cytosines to uracil, while methylated cytosines remain unchanged⁴. The uracil is then converted to thymine during subsequent PCR amplification, while the methylcytosine will be amplified as cytosine. PCR products generated from bisulfate-treated nucleic acid templates with different contents of methylcytosine show differences in melting temperature, which may be resolved by melting analysis. The melting profiles may be used to differentiate among four different methylation states: unmethylated alleles generate a single low melting peak, fully methylated alleles generate a single high melting peak, a mixture of unmethylated and fully methylated alleles generate both the low and high melting peaks, and heterogeneously methylated alleles generate a broadened melting top located between the low and high melting peaks³.

In one example, single-tube analysis of nucleic acid methylation using silica superparamagnetically responsive beads (SSBs)⁵ may be adapted for use on a droplet actuator. An example of a digital microfluidic protocol for methylation-specific melting curve analysis may include, but is not limited to, the following: Nucleic acid may be prepared on a droplet actuator from a buccal swab using superparamagnetically responsive beads such as CHARGESWITCH® beads. A sample droplet that includes magnetically responsive beads with purified nucleic acid thereon is dispensed and transported using droplet operations to a temperature control zone and the purified nucleic acid is denatured using, for example, alkali treatment (NaOH) at 42 °C. The sample droplet with denatured nucleic acid therein is combined using droplet operations with a bisulfite reagent droplet to yield a reaction droplet. The reaction droplet is incubated, for example, at 55 °C for a period of time sufficient for conversion of unmethylated cytosines to uracil. Following bisulfite conversion, the reaction droplet is transported using droplet operations into the presence of a magnet and washed using a merge-and-split wash protocol to purify the converted nucleic acid. The purified nucleic acid is then eluted from the CHARGESWITCH® beads with 10 mM Tris HCl, 1 mM EDTA, pH 7.4. The eluted nucleic acid contained in the droplet surrounding the CHARGESWITCH® beads may then be transported away from the beads for execution of a droplet-based integrated PCR amplification and HRM analysis. PCR amplification and HRM analysis may be performed on the converted sample droplet as described in reference to Figure 1. For PCR amplification, primers may be selected for methylation-insensitive amplification or methylation-sensitive amplification.

### 7.9 Systems

With respect to the library construction cartridge users may attempt to re-use the cartridge, or in the case where 24 samples are available, users may want to run some samples now and run the remaining samples later. This may be undesirable for a number of reasons, for example quality assurance. In one embodiment a fuse could be blown on the PCB or within the cartridge. The fuse could be an electronic component soldered on the PCB, or could perhaps be a trace on the PCB (e.g., formed with a material other than copper). The fuse could for example be put in the path to the top-plate which would physically disable the entire device. As an alternative to physically disabling the device, the fuse could be used to store a bit of data. In this case the controller would interrogate the status of the fuse before proceeding with a run. In this case the "fuse" could be anything that could have its electrical state written to and read by the controller, for example, a change in capacitance could be used instead of an open/short to represent the status. In another embodiment devices may have metalized blister packs or other single-use features that are capable of producing an electrical signature that may be used to detect the use status or serviceability of the cartridge. In this case the "writing" is produced through normal use of the cartridge and the instrument only performs the detection function. In another embodiment, multiple bits of data may be encoded within the cartridge. For example, the cartridge could include an EEPROM which is used to encode the use status of the cartridge as well as other data. In an embodiment the EEPROM authentication system would be designed to avoid re-use of the cartridge and would also be resistant to cloning and replacement of EEPROMS as a user work-around. Many other types of data besides use status could be included in the EEPROM. One feature may include the barcode number. A barcode sticker on the cartridge could be used to identify the cartridge to a laboratory robotic system with barcode readers. In another embodiment, for nucleic acid applications an additional alternative is to intentionally contaminate the interior cartridge surfaces to prevent re-use. For example, as a final step, a droplet containing a high concentration of nucleic acid could be routed all around the cartridge including the previously un-used areas. Thus, intentionally contaminate the interior cartridge surfaces and preventing re-use. U.S. Patent No. 6,495,104 Entitled "Indicator Components For Microfluidic Sytems," filed on August 19, 1999 the entire disclosures of which is incorporated herein by reference for its teaching concerning suitable indicator elements useful for identifying whether a cartridge has been used.

It will be appreciated that various aspects of the invention may be embodied as a method, system, computer readable medium, and/or computer program product. Aspects of the invention may take the form of hardware embodiments, software embodiments (including firmware, resident software, micro-code, etc.), or embodiments combining software and hardware aspects that may all generally be referred to herein as a "circuit," "module" or "system." Furthermore, the methods of the invention may take the form of a computer program product on a computer-usable storage medium having computer-usable program code embodied in the medium.

Any suitable computer useable medium may be utilized for software aspects of the invention. The computer-usable or computer-readable medium may be, for example but not limited to, an electronic, magnetic, optical, electromagnetic, infrared, or semiconductor system, apparatus, device, or propagation medium. The computer readable medium may include transitory and/or non-transitory embodiments. More specific examples (a non-exhaustive list) of the computer-readable medium would include some or all of the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a transmission medium such as those supporting the Internet or an intranet, or a magnetic storage device. Note that the computer-usable or computer-readable medium could even be paper or another suitable medium upon which the program is printed, as the program can be electronically captured, via, for instance, optical scanning of the paper or other medium, then compiled, interpreted, or otherwise processed in a suitable manner, if necessary, and then stored in a computer memory. In the context of this document, a computer-usable or computer-readable medium may be any medium that can contain, store, communicate, propagate, or transport the program for use by or in connection with the instruction execution system, apparatus, or device.

Program code for carrying out operations of the invention may be written in an object oriented programming language such as Java, Smalltalk, C++ or the like. However, the program code for carrying out operations of the invention may also be written in conventional procedural programming languages, such as the "C" programming language or similar programming languages. The program code may be executed by a processor, application specific integrated circuit (ASIC), or other component that executes the program code. The program code may be simply referred to as a software application that is stored in memory (such as the computer readable medium discussed above). The program code may cause the processor (or any processor-controlled device) to produce a graphical user interface ("GUI"). The graphical user interface may be visually produced on a display device, yet the graphical user interface may also have audible features. The program code, however, may operate in any processor-controlled device, such as a computer, server, personal digital assistant, phone, television, or any processor-controlled device utilizing the processor and/or a digital signal processor.

The program code may locally and/or remotely execute. The program code, for example, may be entirely or partially stored in local memory of the processor-controlled device. The program code, however, may also be at least partially remotely stored, accessed, and downloaded to the processor-controlled device. A user's computer, for example, may entirely execute the program code or only partly execute the program code. The program code may be a stand-alone software package that is at least partly on the user's computer and/or partly executed on a remote computer or entirely on a remote computer or server. In the latter scenario, the remote computer may be connected to the user's computer through a communications network.

The invention may be applied regardless of networking environment. The communications network may be a cable network operating in the radio-frequency domain and/or the Internet Protocol (IP) domain. The communications network, however, may also include a distributed computing network, such as the Internet (sometimes alternatively known as the "World Wide Web"), an intranet, a local-area network (LAN), and/or a wide-area network (WAN). The communications network may include coaxial cables, copper wires, fiber optic lines, and/or hybrid-coaxial lines. The communications network may even include wireless portions utilizing any portion of the electromagnetic spectrum and any signaling standard (such as the IEEE 802 family of standards, GSM/CDMA/TDMA or any cellular standard, and/or the ISM band). The communications network may even include powerline portions, in which signals are communicated via electrical wiring. The invention may be applied to any wireless/wireline communications network, regardless of physical componentry, physical configuration, or communications standard(s).

Certain aspects of invention are described with reference to various methods and method steps. It will be understood that each method step can be implemented by the program code and/or by machine instructions. The program code and/or the machine instructions may create means for implementing the functions/acts specified in the methods.

The program code may also be stored in a computer-readable memory that can direct the processor, computer, or other programmable data processing apparatus to function in a particular manner, such that the program code stored in the computer-readable memory produce or transform an article of manufacture including instruction means which implement various aspects of the method steps.

The program code may also be loaded onto a computer or other programmable data processing apparatus to cause a series of operational steps to be performed to produce a processor/computer implemented process such that the program code provides steps for implementing various functions/acts specified in the methods of the invention.

### 8 Concluding Remarks

The foregoing detailed description of embodiments refers to the accompanying drawings, which illustrate specific embodiments of the invention and is for the purpose of illustration only. Other embodiments having different structures and operations that do not depart from the scope of the present invention will be readily apparent to the skilled artisan in view of the instant description. The term "the invention" or the like is used with reference to certain specific examples of the many alternative aspects or embodiments of the applicants' invention set forth in this specification, and neither its use nor its absence is intended to limit the scope of the applicants' invention or the scope of the claims. This specification is divided into sections for the convenience of the reader only. Headings should not be construed as limiting of the scope of the invention. The definitions are intended as a part of the description of the invention. It will be understood that various details of the present invention may be changed without departing from the scope of the present invention.

The present application is a divisional application based on an earlier European Application No. 11747926.1, which was in turn derived from PCT Application No PCT/US2011/025711. The following numbered clauses, which correspond to the clauses of that earlier PCT Application as filed, form part of the present disclosure and in particular form further aspects of the invention, whether or not they appear in the present clauses.
1. A method of preparing a nucleic acid library in droplets in contact with oil, comprising:
   (a) blunt-ending nucleic acid fragments in a droplet in the oil to yield blunt- ended nucleic acid fragments;
   (b) phosphorylating the blunt-ended nucleic acid fragments in a droplet in the oil to yield phosphorylated nucleic acid fragments;
   (c) coupling A-tails to the phosphorylated nucleic acid fragments in a droplet in the oil to yield A-tailed nucleic acid fragments; and
   (d) coupling nucleic acid adapters to the A-tailed nucleic acid fragments in a droplet in the oil to yield the nucleic acid library comprising adapter- ligated nucleic acid fragments.
2. A method of preparing a nucleic acid library in droplets in contact with oil, comprising:
   (a) blunt-ending nucleic acid fragments in a droplet in the oil to yield blunt- ended nucleic acid fragments;
   (b) phosphorylating the blunt-ended nucleic acid fragments in a droplet in the oil to yield phosphorylated nucleic acid fragments; and
   (c) coupling nucleic acid adapters to the blunt ended nucleic acid fragments in a droplet in the oil to yield the nucleic acid library comprising adapter- ligated nucleic acid fragments.
3. The method of any of clauses 1 and following, wherein recovery of adapter- ligated nucleic acid fragments from step 1(d) is at least 5% on a molar basis of nucleic acid fragments input into step 1(a).
4. The method of any of clauses 1 and following, wherein recovery of adapter- ligated nucleic acid fragments from step 1(d) is at least 10% on a molar basis of nucleic acid fragments input into step 1(a).
5. The method of any of clauses 1 and following, wherein recovery of adapter- ligated nucleic acid fragments from step 1(d) is at least 15% on a molar basis of nucleic acid fragments input into step 1(a).
6. The method of any of clauses 1 and following, wherein recovery of adapter- ligated nucleic acid fragments from step 1(d) is at least 20% on a molar basis of nucleic acid fragments input into step 1(a).
7. The method of any of clauses 1 and following, wherein recovery of adapter- ligated nucleic acid fragments from step 1(d) is at least 50% on a molar basis of nucleic acid fragments input into step 1(a).
8. The method of any of clauses 1 and following, wherein recovery of adapter- ligated nucleic acid fragments from step 1(d) is at least 75% on a molar basis of nucleic acid fragments input into step 1(a).
9. The method of any of clauses 1 and following, wherein recovery of adapter- ligated nucleic acid fragments from step 1(d) is at least 90% on a molar basis of nucleic acid fragments input into step 1(a).
10. The method of any of clauses 1 and following, wherein recovery of adapter- ligated nucleic acid fragments from step 1(d) is at least 95% on a molar basis of nucleic acid fragments input into step 1(a).
11. The method of any of clauses 1 and following, wherein recovery of adapter- ligated nucleic acid fragments from step 1(d) is at least 99% on a molar basis of nucleic acid fragments input into step 1(a).
12. The method of clause 1, wherein steps 1(a) and 1(b) are performed together in a single droplet.
13. The method of clause 1, wherein steps 1(b) and 1(c) are performed together in a single droplet.
14. The method of clause 1, wherein steps 1(a), 1(b) and 1(c) are performed together in a single droplet.
15. The method of any of clauses 1 and following, comprising purifying the blunt- ended nucleic acid fragments prior to initiating step 1(b).
16. The method of any of clauses 1 and following, comprising purifying the phosphorylated nucleic acid fragments prior to initiating step 1(c).
17. The method of any of clauses 1 and following, comprising purifying the A-tailed nucleic acid fragments prior to initiating step 1(d).
18. The method of any of clauses 15 and following, wherein the purifying comprises capturing the nucleic acid fragments on beads in a droplet in the oil and wash1ng the beads in the oil.
19. The method of clause 18, wherein the beads comprise charge switch beads or solid phase reversible immobilization beads.
20. The method of any of clauses 18 and following, wherein the purifying comprises:
   (a) merging wash droplets with a droplet comprising the beads to yield a merged droplet; and (b) splitting the merged droplet wh1le restraining the beads yielding a daughter droplet with the beads and a daughter droplet which is substantially lacking in the beads.
21. The method of clause 20, wherein any bead loss in 20(b) is not sufficient to render the nucleic acid library unsuitable for its intended purpose.
22. The method of any of clauses 1 and following, wherein the nucleic acid fragments comprise nucleic acid fragments with 5'- and/or 3'- overhangs.
23. The method of any of clauses 1 and following, wherein blunt-ending comprises combining in the oil a droplet comprising the nucleic acid fragments with a droplet comprising blunt-ending reagents.
24. The method of any of clauses 1 and following, wherein phosphorylating comprises combining in the oil a droplet comprising the nucleic acid fragments with a droplet comprising phosphorylating reagents.
25. The method of any of clauses 1 and following, wherein coupling A-tails comprises combining in the oil a droplet comprising the nucleic acid fragments with a droplet comprising A-tailing reagents.
26. The method of any of clauses 1 and following, wherein coupling nucleic acid adapters comprises combining in the oil a droplet comprising the nucleic acid fragments with a droplet comprising adapter-ligation reagents.
27. A method of performing nucleic acid library construction in droplets in contact with oil, comprising:
   a) blunt-ending and phosphorylating nucleic acid fragments in a droplet in the oil to yield blunt-ended/phosphorylated nucleic acid fragments;
   b) capturing the blunt-ended/phosphorylated nucleic acid fragments in a droplet in the oil using solid phase reversible immobilization beads in a binding buffer;
   c) washing the solid phase reversible immobilization beads in a droplet in the oil using an aqueous buffer;
   d) eluting the blunt-ended/phosphorylated nucleic acid fragments from the solid phase reversible immobilization beads in a droplet in the oil;
   e) coupling A-tails on both ends of the phosphorylated nucleic acid fragments in a droplet in the oil to yield A-tailed nucleic acid fragments;
   f) capturing the A-tailed nucleic acid fragments in a droplet in the oil using solid phase reversible immobilization beads in a binding buffer;
   g) washing the solid phase reversible immobilization beads in a droplet in the oil using an aqueous buffer;
   h) eluting the A-tailed nucleic acid fragments from the solid phase reversible immobilization beads in a droplet in the oil;
   i) coupling nucleic acid adapters to the A-tailed nucleic acid fragments in a droplet in the oil to yield adapter-ligated nucleic acid fragments;
   j) capturing the adapter-ligated nucleic acid fragments in a droplet in the oil using solid phase reversible immobilization beads in a binding buffer;
   k) washing the solid phase reversible immobilization beads in a droplet in the oil using an aqueous buffer;
   l) eluting the adapter-ligated nucleic acid fragments from the solid phase reversible immobilization beads in a droplet in the oil; and
   m) separating the adapter-ligated nucleic acid fragments from the solid phase reversible immobilization beads in a droplet in the oil.
28. The method of any of clauses 27 and following, wherein recovery of the adapter- ligated nucleic acid fragments from step 27(m) is at least 5% of nucleic acid fragments input into step 27(a).
29. The method of any of clauses 27 and following, wherein recovery of the adapter- ligated nucleic acid fragments from step 27 (m) is at least 10% of nucleic acid fragments input into step 27(a).
30. The method of any of clauses 27 and following, wherein recovery of the adapter- ligated nucleic acid fragments from step 27 (m) is at least 15% of nucleic acid fragments input into step 27(a).
31. The method any of clauses 27 and following, wherein recovery of the adapter- ligated nucleic acid fragments from step 27(m) is at least 20% of nucleic acid fragments input into step 27(a).
32. The method of any of clauses 27 and following, wherein recovery of the adapter- ligated nucleic acid fragments from step 27(m) is at least 50% of nucleic acid fragments input into step 27(a).
33. The method of any of clauses 27 and following, wherein recovery of the adapter- ligated nucleic acid fragments from step 27 (m) is at least 75% of nucleic acid fragments input into step 27(a).
34. The method of any of clauses 27 and following, wherein recovery of the adapter- ligated nucleic acid fragments from step 27(m) is at least 90% of nucleic acid fragments input into step 27(a).
35. The method of any of clauses 27 and following, wherein recovery of the adapter- ligated nucleic acid fragments from step 27 (m) is at least 95% of nucleic acid fragments input into step 27(a).
36. The method of any of clauses 27 and following, wherein recovery of the adapter- ligated nucleic acid fragments from step 27(m) is at least 99% of nucleic acid fragments input into step 27(a).
37. The method of any of clauses 1 and following, further comprising amplifying the nucleic acid library.
38. The method of any of clauses 27 and following, further comprising amplifying the nucleic acid library on a droplet actuator.
39. The method of any of clauses 27 and following, further comprising sequencing the nucleic acid library on an automated sequencer.
40. The method of any of clauses 27 and following, further comprising sequencing the nucleic acid library on an automated sequencer without an intervening nucleic acid amplification step.
41. The method of any of clauses 27 and following, further comprising sequencing the nucleic acid library on an automated sequencer without conducting a nucleic acid amplification step.
42. A method of making blunt-ended/phosphorylated nucleic acid fragments in a droplet in contact with oil, comprising merging a sample droplet comprising nucleic acid fragments with one or more reagent droplets comprising blunt- ending and phosphorylating reagents to yield a product droplet comprising blunt- ended/phosphorylated nucleic acid fragments.
43. The method of clause 42, further comprising merging the product droplet with a bead droplet comprising solid phase reversible immobilization beads to capture the blunt-ended/phosphorylated nucleic acid fragments in a capture droplet.
44. The method of clause 43 further comprising washing the solid phase reversible immobilization beads using a droplet-based merge-and-split wash protocol using wash buffer droplets to yield a droplet comprising washed beads comprising the blunt-ended/phosphorylated nucleic acid fragments, wherein the wash buffer droplets consist essentially of an aqueous buffer.
45. The method of clause 44, further comprising merging a droplet comprising washed beads with an elution buffer droplet to yield an elution droplet comprising eluted blunt-ended/phosphorylated nucleic acid fragments.
46. The method of clause 45, further comprising separating the blunt- ended/phosphorylated nucleic acid fragments from the solid phase reversible immobilization beads to yield a droplet comprising the blunt- ended/phosphorylated nucleic acid fragments in the oil.
47. A method of ligating a nucleic acid to a blunt-ended nucleic acid fragment in a droplet in contact with oil, comprising merging a sample droplet comprising bunt-ended nucleic acid fragments with one or more reagent droplets comprising the nucleic acid and ligation reagents to yield a product droplet comprising ligated nucleic acid fragments.
48. The method of clause 47, further comprising merging the product droplet with a bead droplet comprising solid phase reversible immobilization beads to capture the ligated nucleic acid fragments.
49. The method of clause 48, further comprising washing the solid phase reversible immobilization beads using a droplet-based merge-and-split wash protocol using wash buffer droplets to yield a droplet comprising washed beads comprising the ligated nucleic acid fragments, wherein the wash buffer droplets consist essentially of an aqueous buffer.
50. The method of clause 49, further comprising merging a droplet comprising washed beads with an elution buffer droplet to yield an elution droplet comprising eluted ligated nucleic acid fragments.
51. The method of clause 50, further comprising separating the A-tailed nucleic acid fragments from the solid phase reversible immobilization beads to yield a droplet comprising the ligated nucleic acid fragments in the oil.
52. A method of making A-tailed nucleic acid fragments in a droplet in contact with oil, comprising merging in the oil a sample droplet comprising nucleic acid fragments with one or more reagent droplets comprising A-tailing reagents to yield a product droplet comprising A-tailed nucleic acid fragments.
53. The method of clause 52, further comprising merging the product droplet with a bead droplet comprising solid phase reversible immobilization beads to capture the A-tailed nucleic acid fragments.
54. The method of clause 53, further comprising washing the solid phase reversible immobilization beads using a droplet-based merge-and-split wash protocol using wash buffer droplets to yield a droplet comprising washed beads comprising the A-tailed nucleic acid fragments, wherein the wash buffer droplets consist essentially of an aqueous buffer.
55. The method of clause 54, further comprising merging a droplet comprising washed beads with an elution buffer droplet to yield an elution droplet comprising eluted A-tailed nucleic acid fragments.
56. The method of clause 55, further comprising separating the A-tailed nucleic acid fragments from the solid phase reversible immobilization beads to yield a droplet comprising the A-tailed nucleic acid fragments in the oil.
57. A method of making adapter ligated nucleic acid fragments in a droplet in contact with oil, comprising merging in contact with oil a sample droplet comprising nucleic acid fragments with one or more reagent droplets comprising A-tailing reagents to yield a product droplet comprising adapter ligated nucleic acid fragments.
58. The method of clause 57, further comprising merging the product droplet with a bead droplet comprising solid phase reversible immobilization beads to capture the adapter ligated nucleic acid fragments.
59. The method of clause 57, further comprising washing the solid phase reversible immobilization beads using a droplet-based merge-and-split wash protocol using wash buffer droplets to yield a droplet comprising washed beads comprising the adapter ligated nucleic acid fragments, wherein the wash buffer droplets consist essentially of an aqueous buffer.
60. The method of clause 58, further comprising merging a droplet comprising washed beads with an elution buffer droplet to yield an elution droplet comprising eluted adapter ligated nucleic acid fragments.
61. The method of clause 59, further comprising separating the adapter ligated nucleic acid fragments from the solid phase reversible immobilization beads to yield a droplet comprising the adapter ligated nucleic acid fragments in the oil.
62. A method of purifying nucleic acid fragments in a droplet in contact with oil, comprising conducting the following, steps in contact with oil:
   (a) merging a droplet comprising the nucleic acid fragments with a bead droplet comprising solid phase reversible immobilization beads to capture the nucleic acid fragments;
   (b) washing the solid phase reversible immobilization beads using a droplet- based merge-and-split wash protocol using wash buffer droplets to yield a droplet comprising washed beads comprising the nucleic acid fragments;
   (c) merging a droplet comprising washed beads with an elution buffer droplet to yield an elution droplet comprising eluted blunt- ended/phosphorylated nucleic acid fragments; and (d) separating the nucleic acid fragments from the solid phase reversible immobilization beads to yield a droplet comprising the purified nucleic acid fragments in the oil.
63. The method of any of clauses 62 and following, wherein the wash buffer droplets comprise droplets that consist essentially of an aqueous buffer.
64. The method of clause 63, wherein the aqueous buffer consists essentially of a binding buffer.
65. The method of clause 63, wherein the aqueous buffer is substantially lacking in organic solvents.
66. The method of clause 63, wherein the aqueous buffer comprises no more than about 10% organic solvent.
67. The method of clause 63, wherein the aqueous buffer is substantially lacking in ethanol.
68. The method of clause 63, wherein the aqueous buffer comprises no more than about 10% ethanol.
69. The method of any of clauses 62 and following, wherein the wash buffer droplets comprise droplets comprising at least about 25% organic solvent.
70. The method of any of clauses 62 and following, wherein the wash buffer droplets comprise droplets comprising at least about 50% organic solvent.
71. The method of any of clauses 62 and following, wherein the wash buffer droplets comprise droplets comprising at least about 75% organic solvent.
72. The method of any of clauses 69 and following, wherein the organic solvent comprises an alcohol.
73. The method of any of clauses 69 and following, wherein the organic solvent comprises ethanol.
74. The method of any of clauses 69 and following, wherein the organic solvent consists essentially of ethanol.
75. The method of any of clauses 69 and following, wherein the wash buffer droplets are spiked with a salt.
76. The method of any of clauses 69 and following, wherein the wash buffer droplets are spiked with NaCl.
77. The method of any of clauses 69 and following, wherein the wash buffer droplets comprise a salt in an amount ranging from about 0.01 to about 100 mM.
78. The method of any of clauses 69 and following, wherein the wash buffer droplets comprise a salt in an amount ranging from about 0.1 to about 10 mM.
79. The method of any of clauses 69 and following, wherein the wash buffer droplets are spiked in an amount ranging from about 0.01 to about 100 mM with a normal salt that is soluble in the wash buffer.
80. The method of any of clauses 69 and following, wherein the wash buffer droplets are spiked in an amount ranging from about 0.1 to about 10 mM with a normal salt that is soluble in the wash buffer.
81. The method of any of clauses 69 and following, wherein the wash buffer droplets are spiked in an amount ranging from about 0.01 to about 100 mM with a simple salt that is soluble in the wash buffer.
82. The method of any of clauses 69 and following, wherein the wash buffer droplets are spiked in an amount ranging from about 0.1 to about 10 mM with a simple salt that is soluble in the wash buffer.
83. The method of any of clauses 69 and following, wherein the wash buffer droplets are spiked with NaCl in an amount ranging from about 0.01 to about 100 niM.
84. The method of any of clauses 69 and following, wherein the wash buffer droplets are spiked with NaCl in an amount ranging from about 0.1 to about 10 niM.
85. The method of any of clauses 69 and following, wherein the salt improves the repeatability of one or more electrowetting droplet operations relative to the wash buffer droplet comprising the organic solvent in the absence of the salt.
86. The method of clause 85, wherein the one or more electrowetting droplet operations are selected from the group consisting of: droplet transport, droplet splitting, and droplet dispensing.
87. The method of any of clauses 1 and following, wherein the oil comprises a silicone oil.
88. The method of any of clauses 1 and following, wherein the oil comprises a fluorosilicone oil.
89. The method of any of clauses 1 and following, further comprising sequencing the nucleic acid library on an automated sequencer.
90. The method of any of clauses 1 and following, further comprising sequencing the nucleic acid library on an automated sequencer without an intervening nucleic acid amplification step.
91. The method of any of clauses 1 and following, further comprising sequencing the nucleic acid library on an automated sequencer without conducting a nucleic acid amplification step.
92. The method of any of clauses 1 and following, wherein the droplets are controlled by a droplet actuator.
93. The method of clause 92, wherein the droplet actuator controls the steps using droplet operations comprising electrode-mediated droplet operations.
94. The method of clause 93, wherein electrode-mediated droplet operations comprise electro wetting-mediated droplet operations.
95. The method of clause 93, wherein electrode-mediated droplet operations comprise optoelectro wetting-mediated droplet operations.
96. The method of clause 93, wherein electrode-mediated droplet operations comprise dielectrophoresis-mediated droplet operations.
97. The method of any of clauses 1 and following, wherein the steps are performed in an integrated manner on a single droplet actuator without operator interference.
98. The method of any of clauses 1 and following, wherein the droplet in contact with oil is substantially surrounded by the oil.
99. The method of any of clauses 1 and following, wherein the droplet in contact with oil is floating in the oil.
100. The method of any of clauses 1 and following, wherein the droplet in contact with oil is submersed in the oil.
101. The method of any of clauses 1 and following, wherein the droplet in contact with oil is sandwiched between two substrates separated to form a droplet operations gap wh1ch contains the oil.
102. The method of any of clauses 1 and following, wherein the droplet in contact with oil is sandwiched between two substrates separated to form a droplet operations gap wh1ch is substantially filled with the oil.
103. A method of blunt-ending nucleic acid fragments comprising merging in contact with oil a droplet comprising nucleic acid fragments with a droplet comprising blunt-ending reagents to conduct a blunt-ending reaction yielding blunt-ended nucleic acid fragments.
104. A method of phosphorylating nucleic acid fragments comprising merging in contact with oil a droplet comprising blunt-ended nucleic acid fragments with a droplet comprising phosphorylation reagents to conduct a phosphorylation reaction yielding phosphorylated nucleic acid fragments.
105. A method of modifying nucleic acid fragments comprising merging in contact with oil a droplet comprising blunt-ended nucleic acid fragments with a droplet comprising blunt-ending and phosphorylation reagents to conduct blunt-ending and phosphorylation reactions yielding blunt-ended, phosphorylated nucleic acid fragments.
106. A method of A-tailing nucleic acid fragments comprising merging in contact with oil a droplet comprising phosphorylated nucleic acid fragments with a droplet comprising A-tailing reagents to conduct an A-tailing reaction yielding A-tailed nucleic acid fragments.
107. A method of ligating nucleic acid fragments comprising merging in contact with oil a droplet comprising a first nucleic acid with one or more droplets comprising a second nucleic acid and ligation reagents.
108. The method of clause 107, wherein the first nucleic acid comprises an A-tailed nucleic acid, and the second nucleic acid comprises an adapter.
109. The method of any of clauses 1 and following, further comprising ligating the adapters to form a cyclic nucleic acid in a droplet in the oil.
110. The method of clause 109, further comprising fragmenting the cyclic nucleic acid in a droplet in the oil.
111. A system comprising a droplet actuator and a controller programmed to conduct any of the methods of clauses 1 and following.
112. A storage medium comprising encoded software programmed to conduct any of the methods of clauses 1 and following.
113. A droplet actuator comprising affixed thereto the storage medium of clause 112.
114. A droplet actuator, comprising:
   (a) a top substrate and a bottom substrate, the two substrates configured to form a droplet operations gap having a height h1;
   (b) an electrode path comprising electrodes associated with one or both of the bottom substrate and the top substrate, and configured for conducting droplet operations in the gap having a height h1; and
   (c) a recessed area formed in one of the top or bottom substrates configured to form a cavity between the two substrates having a height h2, wherein h2 is greater than h1.
115. The droplet actuator of any of clauses 114 and following, wherein the cavity is adjacent to the electrodes configured for conducting droplet operations such that deactivation of electrodes in the presence of a droplet permits a droplet to flow from the electrodes into the recessed area.
116. The droplet actuator of any of clauses 114 and following, wherein the droplet enters the cavity as a result of displacement caused by deformation of the droplet to a more energetically stable conformation.
117. The droplet actuator of any of clauses 114 and following, wherein the cavity has dimensions selected to prevent the droplet from re-entering the region of the gap having a height h1 upon reactivation of electrodes of the electrode path.
118. The droplet actuator of any of clauses 114 and following, wherein the shape of the recessed area comprises a stair step shape from h1 to h2.
119. The droplet actuator of any of clauses 114 and following, wherein the shape of the recessed area comprises a slope from h1 to h2.
120. The droplet actuator of any of clauses 114 and following, wherein the recessed area is formed in the top substrate, the bottom substrate, or both substrates.
121. The droplet actuator of any of clauses 120 and following, wherein the recessed area is open at its top.
122. A method of displacing a droplet from atop an electrode in a droplet actuator, the method comprising deactivating an electrode to permit the droplet to be displaced into an adjacent region of the droplet actuator in wh1ch the droplet takes on a more energetically stable conformation relative to its conformation atop the electrode.
123. A method of displacing a droplet in an initial position in a droplet actuator, the method comprising deactivating an electrode to permit the droplet to be displaced into an adjacent region of the droplet actuator in wh1ch the droplet takes on a more energetically stable conformation relative to its conformation in its initial position.
124. The method of clause 123, wherein:
   (a) the droplet actuator comprises: a top substrate and a bottom substrate, the two substrates configured to form a droplet operations gap having a height h1 ; an electrode path comprising the electrode, the gap having a height h1 in the region of the electrode; and a recessed area adjacent to the electrode formed in one of the top or bottom substrates configured to form a cavity between the two substrates having a height h2, wherein h2 is greater than h1 ; and (b) the displacement is from the region of the gap having a height h1 to the region of the gap having a height h2.
125. The method of any of clauses 123 and following, wherein the displacement is permanent such that reactivation of the electrode cannot return the droplet to its former position atop the electrode.
126. The method of any of clauses 123 and following, wherein the displacement is temporary such that reactivation of the electrode returns the droplet to its former position atop the electrode.
127. The method of any of clauses 123 and following, wherein the displaced droplet is positioned adjacent to a th1rd electrode, such that activation of the th1rd electrode displaces the droplet to a position atop the th1rd electrode.
128. The method of any of clauses 124 and following, wherein the shape of the recessed area comprises a stair step shape from h1 to h2.
129. The method of any of clauses 124 and following, wherein the shape of the recessed area comprises a slope from h1 to h2.
130. The method of any of clauses 124 and following, wherein the recessed area is formed in the top substrate, the bottom substrate, or both substrates.
131. The method of any of clauses 124 and following, wherein the recessed area is open at its top.
132. A method of dispensing a droplet comprising:
   (a) collecting a source droplet at an end of a segmented path of reservoir electrodes;
   (b) elongating the source droplet along a set of path electrodes and path flanking electrodes;
   (c) deactivating the path flanking electrodes; and
   (d) deactivating one or more of the path electrodes to yield a dispensed droplet and a remaining portion of the source droplet.
133. The method of clause 132 wherein:
   (a) the source droplet includes magnetically responsive beads;
   (b) a magnetic field is supplied at a position which attracts the magnetically responsive beads into a region of the droplet atop the path electrodes; and
   (c) the deactivating step yields the dispensed droplet with a quantity of magnetically responsive beads from the source droplet.
134. The method of clause 133, wherein the deactivating step yields the dispensed droplet with at least 25% of magnetically responsive beads from the source droplet.
135. The method of clause 133, wherein the deactivating step yields the dispensed droplet with at least 50% of magnetically responsive beads from the source droplet.
136. The method of clause 133, wherein the deactivating step yields the dispensed droplet with at least 75% of magnetically responsive beads from the source droplet.
137. The method of clause 133, wherein the deactivating step yields the dispensed droplet with at least 90% of magnetically responsive beads from the source droplet.
138. The method of clause 133, wherein the deactivating step yields the dispensed droplet with at least 95% of magnetically responsive beads from the source droplet.
139. The method of clause 133, wherein the deactivating step yields the dispensed droplet with at least 99% of magnetically responsive beads from the source droplet.
140. The method of clause 133, wherein the deactivating step yields the dispensed droplet with substantially all magnetically responsive beads from the source droplet.
141. The method of any of clauses 133 and following, wherein the source droplet comprises thousands of magnetically responsive beads.
142. The method of any of clauses 133 and following, wherein the source droplet comprises millions of magnetically responsive beads.
143. A droplet actuator assembly comprising:
   (a) one or more substrates;
   (b) a series of reaction lanes on the one or more substrates, each reaction lane comprising a path of electrodes;
   (c) a first set of droplet dispensing electrode assemblies on the one or more substrates, each assembly of the first set arranged to dispense sample droplets onto one of the reaction lanes without traversing any other of the reaction lanes; and
   (d) a second set of droplet dispensing electrode assemblies on the one or more substrates, each assembly of the second set arranged to dispense reagent droplets onto one of the reaction lanes.
144. The droplet actuator assembly of clause 143 wherein:
   (a) the one or more substrates are arranged to form a droplet operations gap; and (b) the reaction lanes are situated in the droplet operations gap.
145. The droplet actuator assembly of any of clauses 144 and following, comprising a fluid path extending from an exterior of the droplet operations gap into the droplet operations gap and arranged to deliver liquid into proximity one or more of the first set of droplet dispensing electrodes.
146. The droplet actuator assembly of any of clauses 144 and following, comprising a fluid path extending from an exterior of the droplet operations gap into the droplet operations gap and arranged to deliver liquid into proximity one or more of the second set of droplet dispensing electrodes.
147. The droplet actuator assembly of any of clauses 143 and following, wherein each of the first set of droplet dispensing electrode assemblies is associated with a reservoir comprising a sample fluid.
148. The droplet actuator assembly of any of clauses 143 and following, comprising at least 2 reaction lanes.
149. The droplet actuator assembly of any of clauses 143 and following, comprising at least 8 reaction lanes.
150. The droplet actuator assembly of any of clauses 143 and following, comprising at least 16 reaction lanes.
151. The droplet actuator assembly of any of clauses 143 and following, comprising at least 24 reaction lanes.
152. The droplet actuator assembly of any of clauses 143 and following, comprising at least 48 reaction lanes.
153. The droplet actuator assembly of any of clauses 143 and following, comprising at least 96 reaction lanes.
154. The droplet actuator assembly of any of clauses 143 and following, wherein each of the second set of droplet dispensing electrode assemblies is situated in a reservoir comprising a library construction reagent.
155. The droplet actuator assembly of clause 154, wherein the second set of droplet dispensing electrode assemblies is divided into subsets, each subset comprising two or more droplet dispensing electrode assemblies arranged to dispense sample droplets onto the same one of the reaction lanes without traversing any other of the reaction lanes.
156. The droplet actuator assembly of clause 155, wherein with1n each subset of droplet dispensing electrode assemblies, each assembly with1n such subset is associated with a reservoir comprising a different library construction reagent.
157. The droplet actuator assembly of clause 156, wherein the reagents are selected from blunt-ending reagents, phosphorylation reagents, A-tailing reagents, and adapter ligation reagents.
158. The droplet actuator assembly of any of clauses 143 and following, further comprising a magnet array situated relative to the reaction lanes such that the magnetic fields in the vicinity of the reaction lanes have strength sufficient to immobilize magnetically responsive beads in droplets in one or more regions of the reaction lanes.
159. The droplet actuator assembly of any of clauses 143 and following, further comprising a magnet array situated relative to the reaction lanes such that the magnetic fields in the vicinity of the reaction lanes have strength sufficient to restrain magnetically responsive beads in droplets during a droplet splitting reaction controlled by the electrodes of the reaction lane.
160. The droplet actuator assembly of clause 158, wherein the magnet array comprises magnets arranged to produce reinforced regions of the magnetic field and the reinforced regions are aligned with the reaction lanes to immobilize magnetically responsive beads in the reaction lanes.
161. A method of conducting a droplet based assay using electrode-mediated droplet operations, the method comprising:
   (a) dispensing two or more sample droplets and transporting each sample droplet onto an independent reaction lane without causing any sample droplet to traverse a reaction lane of another droplet; and
   (b) dispensing a first set of reagent droplets and transporting each droplet of the first set of reagent droplets onto a reaction lane without causing any droplet of the first set of reagent droplets to traverse any region of any other reaction lane that has been previously traversed by a sample droplet.
162. The method of any of clauses 161 and following, further comprising merging each sample droplet with one of the first set of reagent droplets.
163. The method of any of clauses 161 and following, further comprising advancing each sample droplet along its independent reaction lane.
164. The method of any of clauses 161 and following, further comprising dispensing a second set of reagent droplets and transporting each droplet of the second set of reagent droplets onto a reaction lane without causing any droplet of the second set of reagent droplets to traverse any region of any other reaction lane that has been previously traversed by a sample droplet.
165. The method of any of clauses 161 and following, further comprising merging each sample droplet with one of the second set of reagent droplets.
166. The method of any of clauses 161 and following, further comprising advancing each sample droplet along its independent reaction lane.

## Claims

1. A method of performing nucleic acid library construction in droplets in contact with oil, comprising:
a) blunt-ending and phosphorylating nucleic acid fragments in a droplet in the oil to yield blunt-ended/phosphorylated nucleic acid fragments;
b) capturing the blunt-ended/phosphorylated nucleic acid fragments in a droplet in the oil using solid phase reversible immobilization beads in a binding buffer;
c) washing the solid phase reversible immobilization beads in a droplet in the oil using an aqueous buffer;
d) eluting the blunt-ended/phosphorylated nucleic acid fragments from the solid phase reversible immobilization beads in a droplet in the oil;
e) coupling A-tails on both ends of the phosphorylated nucleic acid fragments in a droplet in the oil to yield A-tailed nucleic acid fragments;
f) capturing the A-tailed nucleic acid fragments in a droplet in the oil using solid phase reversible immobilization beads in a binding buffer;
g) washing the solid phase reversible immobilization beads in a droplet in the oil using an aqueous buffer;
h) eluting the A-tailed nucleic acid fragments from the solid phase reversible immobilization beads in a droplet in the oil;
i) coupling nucleic acid adapters to the A-tailed nucleic acid fragments in a droplet in the oil to yield adapter-ligated nucleic acid fragments;
j) capturing the adapter-ligated nucleic acid fragments in a droplet in the oil using solid phase reversible immobilization beads in a binding buffer;
k) washing the solid phase reversible immobilization beads in a droplet in the oil using an aqueous buffer;
l) eluting the adapter-ligated nucleic acid fragments from the solid phase reversible immobilization beads in a droplet in the oil; and
m) separating the adapter-ligated nucleic acid fragments from the solid phase reversible immobilization beads in a droplet in the oil.

2. The method of claim 1 wherein recovery of the adapter-ligated nucleic acid fragments from step 1(m) is at least 5% of nucleic acid fragments input into step 1(a).

3. The method of claim 1 or claim 2 wherein recovery of the adapter-ligated nucleic acid fragments from step 1(m) is at least 10% of nucleic acid fragments input into step 1(a).

4. The method of claims 1 to 3 wherein recovery of the adapter-ligated nucleic acid fragments from step 1(m) is at least 15% of nucleic acid fragments input into step 1(a).

5. The method of claims 1 to 4 wherein recovery of the adapter-ligated nucleic acid fragments from step 1(m) is at least 20% of nucleic acid fragments input into step 1(a).

6. The method of claims 1 to 5 wherein recovery of the adapter-ligated nucleic acid fragments from step 1(m) is at least 50% of nucleic acid fragments input into step 1(a).

7. The method of claims 1 to 6 wherein recovery of the adapter-ligated nucleic acid fragments from step 1(m) is at least 75% of nucleic acid fragments input into step 1(a).

8. The method of claims 1 to 7 wherein recovery of the adapter-ligated nucleic acid fragments from step 1(m) is at least 90% of nucleic acid fragments input into step 1(a).

9. The method of claims 1 to 8 wherein recovery of the adapter-ligated nucleic acid fragments from step 1(m) is at least 95% of nucleic acid fragments input into step 1(a).

10. The method of claims 1 to 9 wherein recovery of the adapter-ligated nucleic acid fragments from step 1(m) is at least 99% of nucleic acid fragments input into step 1(a).

11. The method of any of claims 1 to 10 further comprising amplifying the nucleic acid library.

12. The method of any of claims 1 to 11 further comprising amplifying the nucleic acid library on a droplet actuator.

13. The method of any of claims 1 to 12 further comprising sequencing the nucleic acid library on an automated sequencer.

14. The method of any of claims 1 to 13 further comprising sequencing the nucleic acid library on an automated sequencer without an intervening nucleic acid amplification step.

15. The method of any of claims 1 to 14 further comprising sequencing the nucleic acid library on an automated sequencer without an conducting a nucleic acid amplification step.
